# EUROPEAN PATENT APPLICATION

(11) **EP 1 182 259 A1**
(43) Date of publication of application: **27.02.2002**
(21) Application number: 00117730.2
(22) Date of filing: 17.08.2000
(51) Int. Cl.: C12N 15/82, C12N 9/02, C12N 15/53, C12N 5/10, A01H 5/00, G01N 33/53

(54) **Pyruvate:NADP+ oxidoreductase and uses thereof**

(71) Applicant: BASF Plant Science GmbH, 67056 Ludwigshafen (DE)
(72) Inventor: Cirpus, Petra, Dr., 68163 Mannheim (DE); Lerchl, Jens, Dr., 68526 Ladenburg (DE); Martin, William, Prof. Dr., 41469 Neuss (DE); Rotte, Carmen, 40221 Düsseldorf (DE)

(57) **Abstract**

Provided are polynucleotides encoding Pyruvate:NADP+ oxidoreductases (PNO) as well as methods for obtaining the same. Furthermore, vectors comprising said polynucleotides are described, wherein the polynucleotides are operatively linked to expression control sequences allowing the expression in prokaryotic and/or eukaryotic host cells. In addition, polypeptides encoded by said polynucleotides, antibodies to said polypeptides and methods for their production are provided. Further described are methods for increasing the acetyl CoA synthesis as well as methods for the production of fatty acids, carotenoids, isoprenoids, vitamins, lipids, wax esters, (poly)saccharides and/or polyhydroxyalkanoates, or its metabolism products, in particular, steroid hormones, prostaglandin, cholesterol, triacylglycerols, bile acids or ketone bodies, comprising the expression of the polynucleotide or polypeptide described herein in a host cell or plant cell, plant tissue or plant. Methods for the identification of compounds being capable of activating or inhibiting PNO are described as well. Further, a pharmaceutical composition comprising the aforementioned inhibiting compounds and antibodies is described. Furthermore, transgenic plants, plant tissues, and plant cells containing the above described polynucleotides and vectors are described as well as the use of the mentioned polynucleotides, vectors, polypeptides, antibodies, and/or compounds identified by the method of the invention in the production of acetyl CoA metabolism products, e.g., fatty acids, carotenoids, isoprenoids, vitamins, lipids, (poly)saccharides, wax esters, and/or polyhydroxyalkanoates, and/or its metabolism products, in particular, steroid hormones, prostaglandin, cholesterol, triacylglycerols, bile acids and/or ketone bodies and pharmaceutical compositions.

## Description

Provided are polynucleotides encoding Pyruvate:NADP+ oxidoreductases (PNO) as well as methods for obtaining the same. Furthermore, vectors comprising said polynucleotides are described, wherein the polynucleotides are operatively linked to expression control sequences allowing the expression in prokaryotic and/or eukaryotic host cells. In addition, polypeptides encoded by said polynucleotides, antibodies to said polypeptides and methods for their production are provided. Further described are methods for increasing the acetyl CoA synthesis as well as methods for the production of fatty acids, carotenoids, isoprenoids, vitamins, lipids, wax esters, (poly)saccharides and/or polyhydroxyalkanoates, or its metabolism products, in particular, steroid hormones, prostaglandin, cholesterol, triacylglycerols, bile acids or ketone bodies, comprising the expression of the polynucleotide or polypeptide described herein in a host cell or plant cell, plant tissue or plant. Methods for the identification of compounds being capable of activating or inhibiting PNO are described as well. Further, a pharmaceutical composition comprising the aforementioned inhibiting compounds and antibodies is described. Furthermore, transgenic plants, plant tissues, and plant cells containing the above described polynucleotides and vectors are described as well as the use of the mentioned polynucleotides, vectors, polypeptides, antibodies, and/or compounds identified by the method of the invention in the production of acetyl CoA metabolism products, e.g., fatty acids, carotenoids, isoprenoids, vitamins, lipids, (poly)saccharides, wax esters, and/or polyhydroxyalkanoates, and/or its metabolism products, in particular, steroid hormones, prostaglandin, cholesterol, triacylglycerols, bile acids and/or ketone bodies and pharmaceutical compositions.

Several documents are cited throughout the text of this specification either by name or full reference. Full bibliographic citations may be found at the end of the specification immediately preceding the claims. Each of the documents cited herein (including any manufacture's specifications, instructions, etc.) are hereby incorportated by reference; however, there is no admisssion that any document is indeed prior art as to the present invention.

### Background of the invention

Certain products and by-products of naturally-occurring metabolic processes in cells have utility in a wide array of industries, including the food, feed, cosmetics, and pharmaceutical industries. These molecules, collectively termed 'fine chemicals', comprise, e.g., fatty acids, carotenoids, isoprenoids, vitamins, lipids, wax esters, (poly)saccharides, polyhydroxyalkanoates, steroid hormones, prostaglandin, cholesterol, triacylglycerols, bile acids and/or ketone bodies and/or cofactors. Fine chemicals can be produced in microorganisms through the large-scale culture of microorganisms developed to produce and secrete large quantities of one or more desired molecules.

Their production is most conveniently performed through the large-scale culture of microorganisms developed to produce and/or secrete large quantities of one or more desired molecules. Through strain selection, a number of mutant strains of the respective microorganisms have been developed which produce an array of desirable compounds. However, selection of strains improved for the production of a particular molecule is a time-consuming and difficult process.

Alternatively the production of fine chemicals can be most conveniently performed via the large scale production of plants developed to produce one of aforementioned fine chemicals. Particularly well suited plants for this purpose are oilseed plants containing high amounts of lipid compounds like rapeseed, canola, linseed, soybean and sunflower. But also other crop plants containing fatty acids, carotenoids, isoprenoids, vitamins, lipids, (poly)saccharides, wax esters and/or polyhydroxyalkanoates, and/ or its metabolism products, in particular, steroid hormones, prostaglandin, cholesterol, triacylglycerols, bile acids and/or ketone bodies are well suited as mentioned in the detailed description of this invention. Through conventional breeding, a number of mutant plants have been developed which produce an array of desirable lipids and fatty acids, carotenoids, cofactors and enzymes.

The production of fine chemicals by biological processes as, e.g. via the cultivation of microorganisms, cells or plants producing said fine chemicals, is limited by the often small concentrations of educts, e.g., acetyl CoA, for the production of said compounds.

Recently, several molecular biological approaches to increase the efficiency of fine chemical production, in particular, of fatty acids and lipids, have been developed. Some reports describe an increase of acetyl CoA production for higher fatty acid quantities.

WO 00/00614 reports the overexpression of several enzymes in a cell, i.e., acetyl CoA synthetase, plastidic pyruvate dehydrogenase, ATP citrate lysase, pyruvate decarboxylase and aldehyde dehydrogenase to alter the acetyl CoA content in plants. WO 00/11199 describe compositions comprising nucleotide sequences encoding acetyl CoA synthetases for the increased biosynthesis of fatty acids and carotenoids in plants.

Therefore, the technical problem underlying the present invention is to provide alternative, preferably advantageous means and methods for the efficient biological production of fine chemicals, e.g., fatty acids, carotenoids, isoprenoids, vitamins, lipids, (poly)saccharides, wax esters and/or polyhydroxyalkanoates, and/or its metabolism products, in particular, e.g. steroid hormones, prostaglandin, cholesterol, triacylglycerols, bile acids and/or ketone bodies which can minimize the expenses of such a production and to provide microorganisms, cells or plants which synthesize fine chemicals, in particular, fatty acids, carotenoids, isoprenoids, vitamins, lipids, (poly)saccharides, wax esters and/or polyhydroxyalkanoates, and/or its metabolism products, in particular, steroid hormones, prostaglandin, cholesterol, triacylglycerols, bile acids and/or ketone bodies in high amounts.

The solution of the technical problem is achieved by providing the embodiments characterized in the claims.

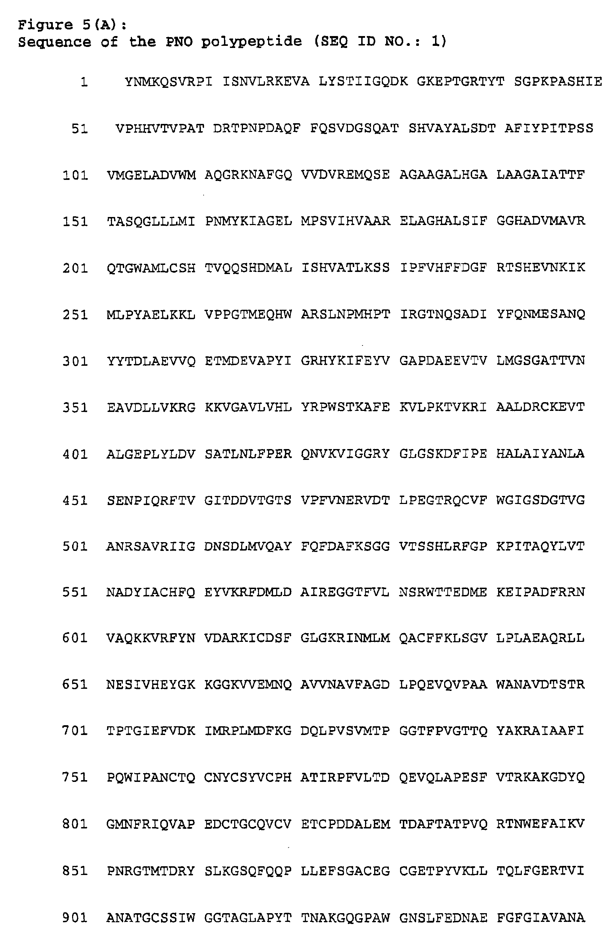

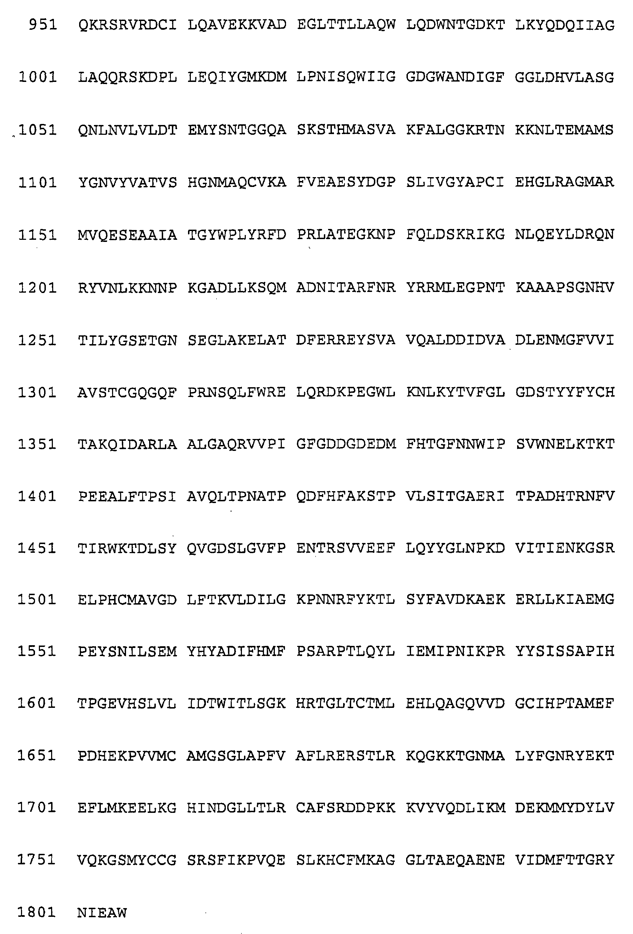

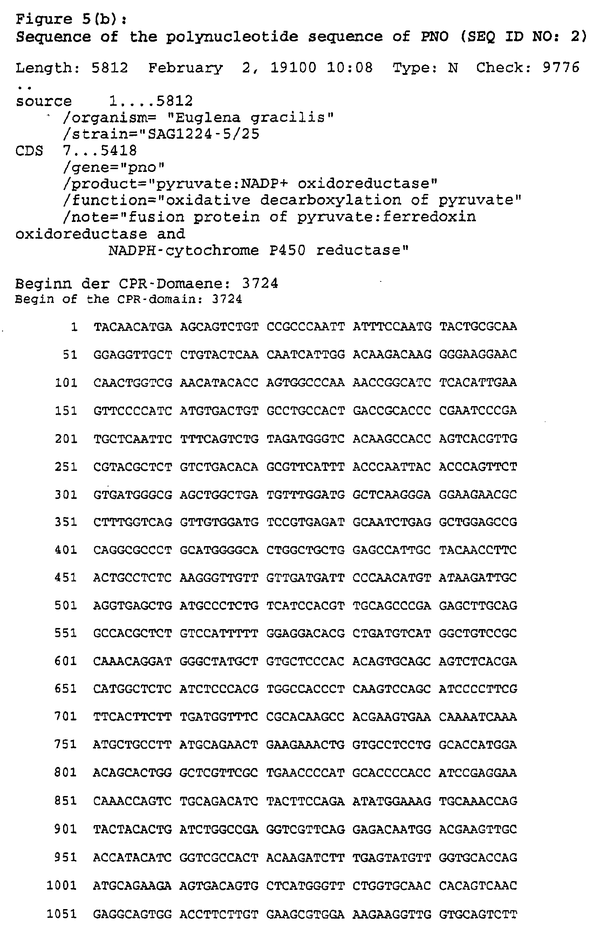

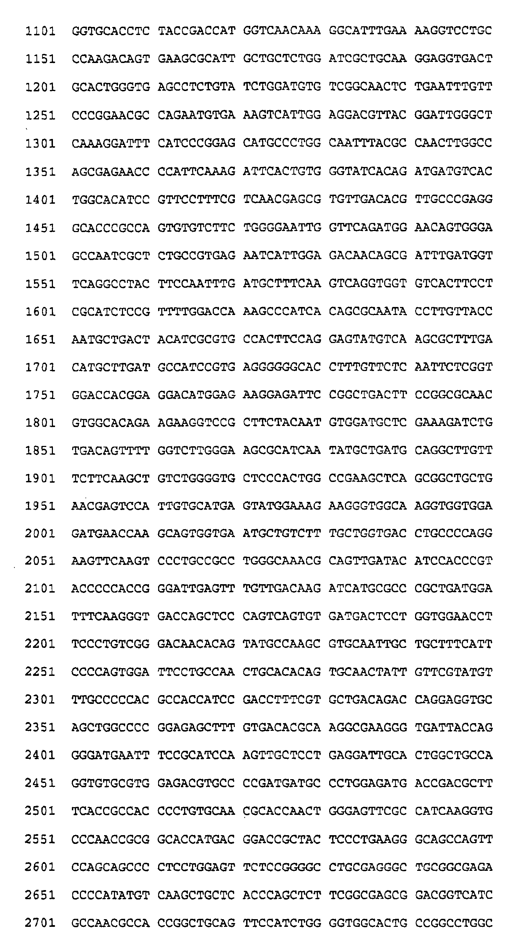

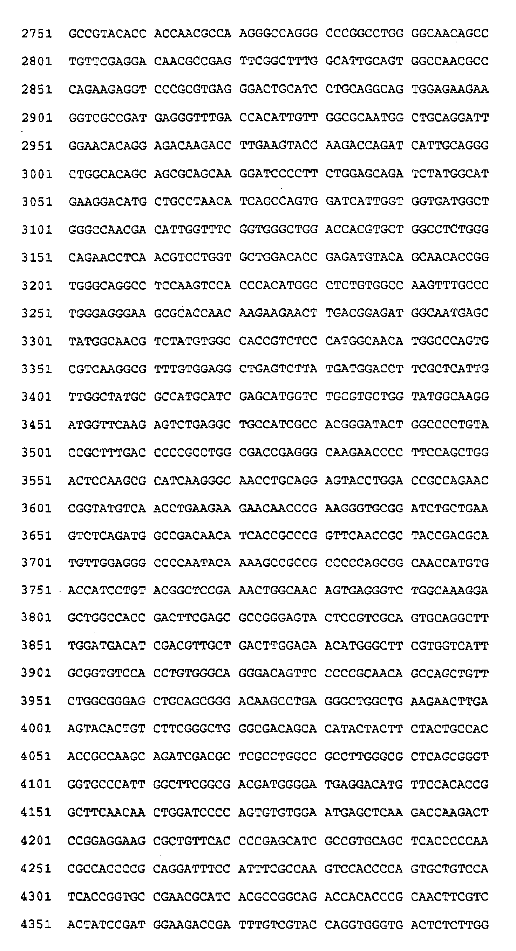

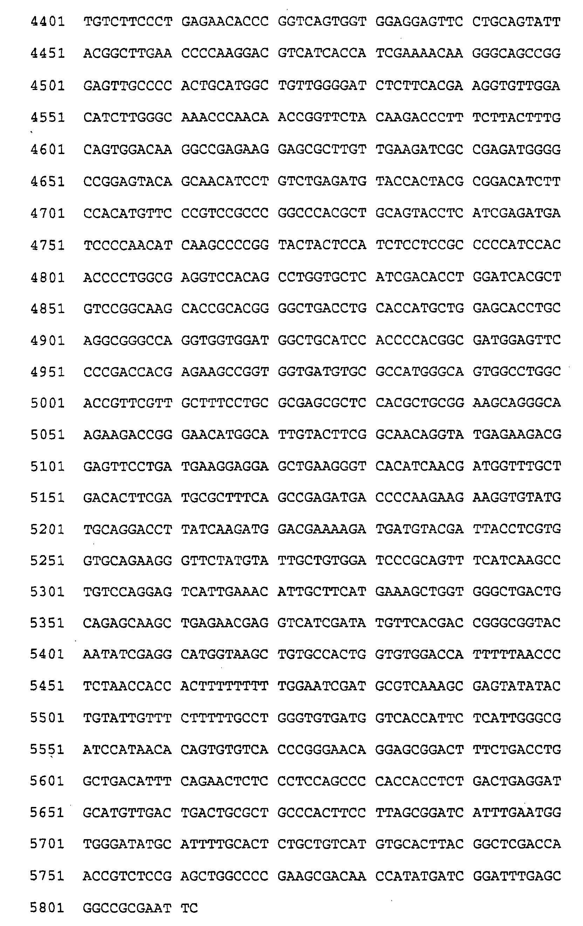

Accordingly, the present invention relates to a polynucleotide comprising a nucleic acid molecule selected from the group consisting of:
(a) nucleic acid molecules encoding at least the mature form of the polypeptide depicted in SEQ ID NO: 1 (Figure 5);
(b) nucleic acid molecules comprising the coding sequence as depicted in SEQ ID NO: 2 (Figure 5) encoding at least the mature form of the polypeptide;
(c) nucleic acid molecules the nucleotide sequence of which is degenerate as a result of the genetic code to a nucleotide
(d) nucleic acid molecules encoding a polypeptide derived from the polypeptide encoded by a polynucleotide of (a) to (c) by way of substitution, deletion and/or addition of one or several amino acids of the amino acid sequence of the polypeptide encoded by a polynucleotide of (a) to (c);
(e) nucleic acid molecules encoding a polypeptide the sequence of which has an identity of 60% or more to the amino acid sequence of the polypeptide encoded by a nucleic acid molecule of (a) or (b);
(f) nucleic acid molecules comprising a fragment or a epitope-bearing portion of a polypeptide encoded by a nucleic acid molecule of any one of (a) to (e) and having acetyl-CoA synthesis regulating activity;
(g) nucleic acid molecules comprising a polynucleotide having a sequence of a nucleic acid molecule amplified from an Euglena nucleic acid library using the primers depicted in SEQ ID NO: 3 and 4;
(h) nucleic acid molecules encoding a pyruvate dehydrogenase active fragment, a pyruvate:ferredoxin oxidoreductase active fragment, and/or a NADPH-cytochrome P450 reductase active fragment of a polypeptide encoded by any one of (a) to (g);
(i) nucleic acid molecules comprising at least 15 nucleotides of a polynucleotide of any one of (a) or (d);
(j) nucleic acid molecules encoding a polypeptide having pyruvate:NADP+ oxidoreductase (PNO) activity being recognized by antibodies that have been raised against a polypeptide encoded by a nucleic acid molecule of any one of (a) to (h);
(k) nucleic acid molecules obtainable by screening an appropriate library under stringent conditions with a probe having the sequence of the nucleic acid molecule of any one of (a) to (j) and having a pyruvate:NADP+ oxidoreductase (PNO) activity;
(1) nucleic acid molecules the complementary strand of which hybridizes under stringent conditions with a nucleic acid molecule of any one of (a) or (k) and having pyruvate:NADP+ oxidoreductase (PNO) activity;
   or the complementary strand of any one of (a) to (1);
   wherein the polynucleotide is not a polynucleotide encoding a polypeptide having the sequence TSGPKPASXI (SEQ ID No.: 5), TSGPKPASXIEVSXAK (SEQ ID No.: 6) or AAAPSGNXVTILYGSEEGNS (SEQ ID No.: 7).

The terms "gene(s)", "polynucleotide", "nucleic acid sequence", "nucleotide sequence", "DNA sequence" or "nucleic acid molecule(s)" as used herein refers to a polymeric form of nucleotides of any length, either ribonucleotides or deoxyribonucleotides. This term refers only to the primary structure of the molecule.

Thus, this term includes double- and single-stranded DNA, and RNA. It also includes known types of modifications, for example, methylation, "caps" substitution of one or more of the naturally occurring nucleotides with an analog. Preferably, the DNA sequence of the invention comprises a coding sequence encoding the above defined polypeptide.

A "coding sequence" is a nucleotide sequence which is transcribed into mRNA and/or translated into a polypeptide when placed under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, recombinant nucleotide sequences or genomic DNA, while introns may be present as well under certain circumstances.

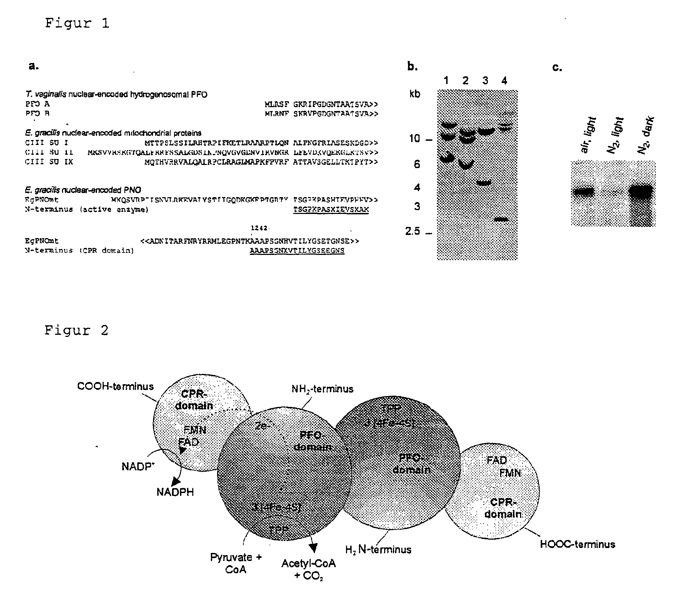

By "hybridizing" it is meant that such nucleic acid molecules hybridize under conventional hybridization conditions, preferably under stringent conditions such as described by, e.g., Sambrook (Molecular Cloning; A Laboratory Manual, 2nd Edition, Cold Spring Harbor Laboratory Press, Cold Spring Harbor, NY (1989)). An example of one such stringent hybridization condition is hybridization at 4XSSC at 65°C, followed by a washing in 0.1XSSC at 65°C for one hour. Alternatively, an exemplary stringent hybridization condition is in 50 % formamide, 4XSSC at 42°C. PNO derived from other organisms, may be encoded by other DNA sequences which hybridize to the sequences for Euglena gracilis under relaxed hybridization conditions and which code on expression for peptides having the ability to interact with PNOs. Further, some applications have to be performed at low stringency hybridisation conditions, without any consequences for the specificity of the hybridisation. For example, as described in the Example 2, a Southern blot analysis of total Euglena DNA was probed with a polynucleotide of the present invention further defined below (pFgPNO3) and washed at low stringency (55°C in 2xSSPE, 0,1% SDS). The hybridisation analysis revealed a simple pattern of only genes encoding Eugelna PNO (Figure 1b). A further example of such non-stringent hybridization conditions are 4XSSC at 50°C or hybridization with 30-40 % formamide at 42°C. Such molecules comprise those which are fragments, analogues or derivatives of the pyruvate:NADP+ oxidoreductase (PNO) of the invention and differ, for example, by way of amino acid and/or nucleotide deletion(s), insertion(s), substitution (s), addition(s) and/or recombination (s) or any other modification(s) known in the art either alone or in combination from the above-described amino acid sequences or their underlying nucleotide sequence(s).

The term "homology" means that the respective nucleic acid molecules or encoded proteins are functionally and/or structurally equivalent. The nucleic acid molecules that are homologous to the nucleic acid molecules described above and that are derivatives of said nucleic acid molecules are, for example, variations of said nucleic acid molecules which represent modifications having the same biological function, in particular encoding proteins with the same or substantially the same biological function. They may be naturally occurring variations, such as sequences from other plant varieties or species, or mutations. These mutations may occur naturally or may be obtained by mutagenesis techniques. The allelic variations may be naturally occurring allelic variants as well as synthetically produced or genetically engineered variants. Structurally equivalents can, for example, identified by testing the binding of said polypeptide to antibodies. Structurally equivalent have the similar immunological characteristic, e.g. comprise similar epitopes.

The terms "fragment", "fragment of a sequence" or "part of a sequence" mean a truncated sequence of the original sequence referred to. The truncated sequence (nucleic acid or protein sequence) can vary widely in length; the minimum size being a sequence of sufficient size to provide a sequence with at least a comparable function and/or activity of the original sequence referred to, while the maximum size is not critical. In some applications, the maximum size usually is not substantially greater than that required to provide the desired activity and/or function(s) of the original sequence.

Typically, the truncated amino acid sequence will range from about 5 to about 60 amino acids in length. More typically, however, the sequence will be a maximum of about 50 amino acids in length, preferably a maximum of about 30 amino acids. It is usually desirable to select sequences of at least about 10, 12 or 15 amino acids, up to a maximum of about 20 or 25 amino acids.

The term "epitope" relates to specific immunoreactive sites within an antigen, also known as antigenic determinates. These epitopes can be a linear array of monomers in a polymeric composition - such as amino acids in a protein - or consist of or comprise a more complex secondary or tertiary structure. Those of skill will recognize that all immunogens (i.e., substances capable of eliciting an immune response) are antigens; however, some antigen, such as haptens, are not immunogens but may be made immunogenic by coupling to a carrier molecule. The term "antigen" includes references to a substance to which an antibody can be generated and/or to which the antibody is specifically immunoreactive.

The term "one or several amino acids" relates to at least one amino acid but not more than that number of amino acids which would result in a homology of below 60% identity. Preferably, the identity is more than 70% or 80%, more preferred are 85%, 90% or 95%, even more preferred are 96%, 97%, 98%, or 99% identity.

The term "PNO" or "PNO activity" relates to enzymatic activities of a polypeptide as described below or which can be determined as described in Example 5. Furthermore, polypeptides that are inactive in an assay as described in Example 5 but are recognized by an antibody specifically binding to PNOs, i.e., having one or more PNO epitopes, are also comprised under the term "PNO". In these cases activity refers to their immunological activity.

The terms "polynucleotide" and "nucleic acid molecule" also relate to "isolated" polynucleotides or nucleic acids molecules. An "isolated" nucleic acid molecule is one which is separated from other nucleic acid molecules which are present in the natural source of the nucleic acid. Preferably, an "isolated" nucleic acid is free of sequences which naturally flank the nucleic acid (i.e., sequences located at the 5' and 3' ends of the nucleic acid) in the genomic DNA of the organism from which the nucleic acid is derived. For example, in various embodiments, the PNO polynucleotide can contain less than about 5 kb, 4kb, 3kb, 2kb, 1 kb, 0.5 kb or 0.1 kb of nucleotide sequences which naturally flank the nucleic acid molecule in genomic DNA of the cell from which the nucleic acid is derived (e.g., a Euglena cell). Moreover, the polynucleotides of the present invention, in particular an "isolated" nucleic acid molecule, such as a cDNA molecule, can be substantially free of other cellular material, or culture medium when produced by recombinant techniques, or chemical precursors or other chemicals when chemically synthesized.

According to the invention, said technical problem can be solved by providing the polynucleotide of the present invention. The polynucleotide of the present invention encoding PNO can, e.g., be expressed in a host cell, a plant cell, a plant tissue and/or a plant modulating the biosynthesis of acetyl CoA and, thus, of its metabolism products.

Inui et al. reported already 1991 two short PNO polypeptides of 16 and 20 amino acid length encoding the processed N-terminus of the native E. gracilis PNO enzyme as well as a short internal polypeptide sequence, N-terminus of a NADPH diaporase trypsin fragment of E. gracilis PNO. Surprisingly, more than nine years after the publication of said peptides the PNO primary structure has still not been solved.

On basis of said sequences degenerated primers were constructed which could hybridize with nucleic acid molecules encoding said peptides. The primer were then used to amplify E. gracilis cDNA to reveal a polynucleotide fragment encoding E. gracilis cDNA. However, all approaches failed. Although varying PCR conditions were used it was not possible to isolate a PNO encoding cDNA fragment on basis of the information published in Inui et al, 1991.

Accordingly, a new isolation approach had to developed to solve the problem of the present invention and to provide a PNO encoding polynucleotide.

PNO is so far an unique enzyme and, until now, it is only known to occur in E. gracilis. Thus, in detailed evolutionary studies the evolutionary closest microorganisms to E. gracilis were determined and their relation to E. gracilis were mapped. The protein domains of the PFOs of said related organisms were analyzed, in particular, the reaction centers of different PFOs were compared to identify common structure characteristics. Finally, two evolutionary conservative domains of PFOs could be revealed. Primers hybridizing with polynucleotides encoding said conservative PFO domains were synthesized and put in an amplification reaction (PCR) with E. gracilis cDNA as template.

Surprisingly, said amplification reaction with E. gracilis cDNA as template but with primers against PFO polynucleotides revealed an around 700bp PNO DNA fragment which could be isolated, sequenced and further used as hybridizytion probe for the cDNA library screening resulting in the first identification of the polypeptide and polynucleotide sequence of an PNO. Sequencing of the complete gene revealed that around 30% of the in Inui disclosed N-terminal PNO sequence was incorrect explaining the negative results of the first PNO identification attemps.

Because PNO is not present in higher plant cells, the heterologous expression of the Euglena PNO gene is an alternative pathway for the production of acetyl-CoA in plant cells. Advantageously, this exogenous pathway is not controlled by endogenous regulation mechanisms present in plants.

The oxidative decarboxylation of pyruvate to acetyl-CoA is a key reaction in intermediary metabolism. In most aerobically growing eubacteria and in mitochondriate organisms, this reaction is catalyzed by a well-studied pyruvate dehydrogenase multi-enzyme-complex (PDH). In most anaerobic eubacteria and archaebacteria, and in many anaerobic protists studied to date, the oxidative decarboxylation of pyruvate to acetyl-CoA is performed by pyruvate:ferredoxin oxidoreductase (PFO), functioning with ferredoxin as electron acceptor. PFO contains thiamine pyrophosphate as a cofactor and 1-3 [4Fe-4S] clusters are involved as redox centers.

The facultatively anaerobic mitochondria of the photosynthetic protist Euglena gracilis represent a peculiar exception among mitochondria-bearing eukaryotes. Activtity of PDH has so far not convincingly been demonstrated. Instead, E. gracilis contains an oxygen-sensitive pyruvate:NADP⁺ oxidoreductase (PNO), the key enzyme of wax ester fermentation (Inui et al. 1984b). Transfer of aerobically grown E. gracilis to anaerobic conditions causes a prompt synthesis of wax esters with a concomitant fall of the reserve polysaccharide paramylon (Inui et al. 1982). This anaerobic wax ester formation is accompanied by a net synthesis of ATP by substrate level phosphorylation in glycolysis, thus allowing the organism to survive anaerobiosis up to 30 days (Buetow, 1989). When the cells are brought back to aerobiosis the reverse change takes place; wax esters are rapidly decomposed while paramylon is synthesized (Inui et al. 1982). Under aerobic conditions, acetyl-CoA produced by PNO feeds oxidative phosphorylation via a modified Krebs cycle (Buetow, 1989).

The polynucleotide provided in the present invention encoding the PNO of E. gracilis provides the unique possibility to synthesize acetyl-CoA from pyruvate in various specifically targeted organelles, e.g., of plant cells, in addition to acetyl-CoA formed by endogenous PDH during intermediary metabolism.

Acetyl-CoA synthesis in higher plant plastids proceeds via a multi-subunit enzyme complex (PDH). Accordingly the clone for the unique single subunit PNO enzyme from E. gracilis possesses great potential for modifying metabolism of a host cell, e.g. a microorganism or a plant cell, by expressing PNO, for example, fused to an appropriate plastid-signal peptide that directs the PNO protein into the plastids.

Acetyl CoA itself is the starting material for several biochemical pathways which all result in the biosynthesis of fine chemicals of high economical interest. For example, acetyl CoA is the central educt for the biosynthesis of fatty acids, which then are further transformed to triacylglycerols, membrane lipids and prostaglandin. Fatty acids and fatty alkohols are, further, also metabolized to become moieties of wax esters. Isoprenoids are components synthesized by polymerization of series of isoprenes via prenyl transfer reactions. A few example of biological important isoprenoids are cholesterol, Ganglioside, mevalonate, farnesyl pyrophosphate, squalene, protosterol, etc. Further example are well know to skilled person. The carbon atoms of the isopentenyl pyrophosphate and dimethylallyl pyrophosphate for the synthesis of isoprenes originate in acetyl-CoA and acetoacetyl-CoA. Carotenoids can not be synthesized by humans, like most other animals. Main suppliers are fruits, vegetables and also rather indirect fungi, algae and bacteria. Carotenoids like all terpenoids are synthesized from hydroxymethylglutaryl-coenzyme A, which is first converted to mevalonic acid. One carotenoid produced in high quantities is beta-carotene. Prostaglandins comprise a family of compounds that be regarded as derivates of the hypothetical compound prostanic acid. They comprise PGEs, PGFs and several further compounds well known to a person skilled in the art. Further, via hydroxymethylgluteryl CoA acetyl CoA is a precursor for the biosynthesis of ketone bodies as well as of cholesterol. Cholesterol is further metabolized to bile acids and steroid hormones. In addition in some microorganisms or transgenic plants acetyl CoA is the educt for biosynthesis of polymers such as polyhydroxyalkanoates. For example, US 5,650,555 and US5,610,041 and references therein disclose genetically modified plants producing poly-beta-D-hydroxybutric acid (PHB) and related polyhydroxyalkonoates. PHB, for example, is a very useful polymer which is biodegradable. Furthermore, it is known, that acetyl CoA is a precursor in the metabolism of vitamins, e.g. vitamin E. Furthermore, under anaerobic conditions some microorganisms, in particular E. gracilis produce wax esters, mainly C28 esters with considerable amounts of saturated C26 and C27 esters but no unsaturated ones are formed. The biochemistry of all said compounds is well know to a person skilled in the art and is easily found in any biochemistry text book, e.g. Rawn, J.D., Biochemistry, Cambridge, 1983, Streyer, Biochemistry, New York, 1988, etc.

The term 'fine chemical' includes molecules produced by an organism which have applications in various industries, such as, but not limited to, the pharmaceutical, agriculture, and cosmetics industries. Such compounds include isoprenoids, prostaglandin, tracylglycerols, cholesterol, polyhydroxyalkanoates, cofactors and enzymes, both proteinogenic and non-proteinogenic amino acids, purine and pyrimidine bases, nucleosides, and nucleotides (as described e.g. in Kuninaka, A. (1996) Nucleotides and related compounds, p. 561-612, in Biotechnology vol. 6, Rehm et al., eds. VCH: Weinheim, and references contained therein), lipids, wax esters, both saturated and polyunsaturated fatty acids (e.g., arachidonic acid), diols (e.g., propane diol, and butane diol), carbohydrates, (e.g. (poly)saccharides or hyaluronic acid and trehalose), aromatic compounds (e.g., aromatic amines, vanillin, and indigo), vitamins, in particular vitamin E, and cofactors (as described in Ullmann's Encyclopedia of Industrial Chemistry, vol. A27, Vitamins, p. 443-613 (1996) VCH: Weinheim and references therein; and Ong, A.S., Niki, E. & Packer, L. (1995) Nutrition, Lipids, Health, and Disease Proceedings of the UNESCO/Confederation of Scientific and Technological Associations in Malaysia, and the Society for Free Radical Research, Asia, held Sept. 1-3, 1994 at Penang, Malaysia, AOCS Press, (1995)), enzymes, and all other chemicals described in Gutcho (1983) Chemicals by Fermentation, Noyes Data Corporation, ISBN: 0818805086 and references therein.

For example, seed storage lipids of higher plants are made of fatty acids, primarily of 16 to 18 carbon atoms. These fatty acids are located in the seed oils of various plant genera. Few plants, such as Cruciferae accumulate oils of C20 and C22. The production of said oils can be increased due to the expression of the polynucleotide of the present invention. In particular for industrial uses, vegetable oils, e.g. with a high erucic acid level, are useful. These oils can be used as diesel fuel and as a material for an array of products, such as plastics, pharmaceuticals and lubricants. Accordingly, the term "lipids" as used in the present invention also relates to seed storage lipids and seed oil.

For example, the synthesis of membranes is a well-characterized process involving a number of components, the most important of which are lipid molecules. Lipid synthesis may be divided into two parts: the synthesis of fatty acids and their attachment to sn-glycerol-3-phosphate, and the addition or modification of a polar head group. Typical lipids utilized in bacterial membranes include phospholipids, glycolipids, sphingolipids, and phosphoglycerides. Fatty acid synthesis begins with the conversion of acetyl CoA either to malonyl CoA by acetyl CoA carboxylase, or to acetyl-ACP by acetyltransacylase. Following a condensation reaction, these two product molecules together form acetoacetyl-ACP, which is converted by a series of condensation, reduction and dehydration reactions to yield a saturated fatty acid molecule having a desired chain length. The production of unsaturated fatty acids from such molecules is catalyzed by specific desaturases either aerobically, with the help of molecular oxygen, or anaerobically (for reference on fatty acid synthesis in microorganisms, see F.C. Neidhardt et al. (1996) E. coli and Salmonella. ASM Press: Washington, D.C., p. 612-636 and references contained therein; Lengeler et al. (eds) (1999) Biology of Prokaryotes. Thieme: Stuttgart, New York, and references contained therein; and Magnuson, K. et al., (1993) *Microbiological Reviews* 57: 522-542, and references contained therein). Furthermore fatty acid have to be transported and incorporated into the triacylglycerol storage lipid subsequent to various modifications. For publications on plant fatty acid biosynthesis, desaturation, lipid metabolism and membrane transport of lipoic compounds, beta-oxidation, fatty acid modification and cofactors, triacylglycerol storage and assembly including references therein see following articles: Kinney, 1997, Genetic Engeneering, ed.: JK Setlow, 19:149-166; Ohlrogge and Browse, 1995, Plant Cell 7:957-970; Shanklin and Cahoon, 1998, Annu. Rev. Plant Physiol. Plant Mol. Biol.,49:611-641; Voelker, 1996, Genetic Engeneering, ed.: JK Setlow, 18:111-13; Gerhardt, 1992, Prog. Lipid R. 31:397-417; Gühnemann-Schäfer &Kindl, 1995, Biochim. Biophys Acta 1256:181-186; Kunau et al., 1995, Prog. Lipid Res. 34:267-342; Stymne et al 1993, in: Biochemistry and Molecular Biology of Membrane and Storrage Lipids of Plants, Eds: Murata and Somerville, Rockville, American Society of Plant Physiologists, 150-158, Murphy & Ross 1998, Plant Journal. 13(1):1-16. Another essential step in lipid synthesis is the transfer of fatty acids onto the polar head groups by, for example, glycerol-phosphate-acyltransferases (see Frentzen, 1998, Lipid, 100(4-5):161-166).

The combination of various precursor molecules and biosynthetic enzymes results in the production of different fatty acid molecules, which has a profound effect on the composition of the membrane.

Vitamins, cofactors, and nutraceuticals comprise a group of molecules which ability to synthesize higher animals have lost. These molecules are either bioactive substances themselves, or are precursors of biologically active substances which may serve as electron carriers or intermediates in a variety of metabolic pathways. Aside from their nutritive value, these compounds also have significant industrial value as coloring agents, antioxidants, and catalysts or other processing aids. (For an overview of the structure, activity, and industrial applications of these compounds, see, for example, Ullman's Encyclopedia of Industrial Chemistry, Vitamins vol. A27, p. 443-613, VCH: Weinheim, 1996.).

In case of polyunsaturated fatty acids see and also references cited therein: Simopoulos 1999, Am. J. Clin. Nutr., 70 (3 Suppl):560-569, Takahata et al., Biosc. Biotechnol. Biochem, 1998, 62 (11):2079-2085, Willich und Winther, 1995, Deutsche Medizinische Wochenschrift, 120 (7):229 ff.

The language "cofactor" includes nonproteinaceous compounds required for a normal enzymatic activity to occur. Such compounds may be organic or inorganic; the cofactor molecules of the invention are preferably organic. The term nutraceutical includes dietary supplements having health benefits in plants and animals, particularly humans. Examples of such molecules are vitamins, antioxidants, and also certain lipids (e.g., polyunsaturated fatty acids). The biosynthesis of these molecules in organisms capable of producing them, such as bacteria, has been largely characterized (Ullman's Encyclopedia of Industrial Chemistry, Vitamins vol. A27, p. 443-613, VCH: Weinheim, 1996; Michal, G. (1999) Biochemical Pathways: An Atlas of Biochemistry and Molecular Biology, John Wiley & Sons; Ong, A.S., Niki, E. & Packer, L. (1995) Nutrition, Lipids, Health, and Disease" Proceedings of the UNESCO/Confederation of Scientific and Technological Associations in Malaysia, and the Society for Free Radical Research Asia, held Sept. 1-3, 1994 at Penang, Malaysia, AOCS Press: Champaign, IL X, 374 S).

Accordingly, the present invention provides polynucleotides and polypeptides which are involved in the biosynthesis of acetyl CoA and, further, products of the metabolism of acetyl CoA, e.g., fatty acids, carotenoids, isoprenoids, wax esters, vitamins, lipids, (poly)saccharides and/or polyhydroxyalkanoates, and/or its metabolism products, in particular, steroid hormones, prostaglandin, cholesterol, triacylglycerols, bile acids and/or ketone bodies, and/or further cofactors and molecules well known to the persons skilled in art. The molecules of the invention may be utilized in the modulation of production of fine chemicals, preferably said compounds, from microorganisms, such as *Corynebacterium,* ciliates, fungi, algae and plants like maize, wheat, rye, oat, triticale, rice, barley, soybean, peanut, cotton, Brassica species like rapeseed, canola and turnip rape, pepper, sunflower and tagetes, solanaceaous plants like potato, tobacco, eggplant, and tomato, Vicia species, pea, manihot, alfalfa, bushy plants (coffee, cacao, tea), Salix species, trees (oil palm, coconut) and perennial grasses and forage crops either directly (e.g., where overexpression or optimization of a fatty acid biosynthesis protein has a direct impact on the yield, production, and/or efficiency of production of the fatty acid from modified organisms), or may have an indirect impact which nonetheless results in an increase of yield, production, and/or efficiency of production of the desired compound or decrease of undesired compounds (e.g., where modulation of the metabolism of acetyl CoA, lipids, fatty acids, carotenoids, etc. results in alterations in the yield, production, and/or efficiency of production or the composition of desired compounds within the cells, which in turn may impact the production of one or more acetyl CoA metabolism based compounds as mentioned herein).

Accordingly, due to the expression of PNO microorganisms, cells or plants metabolic pathways are modulated in yield production, and/or efficiency of production.

The terms "production" or "productivity" are art-recognized and include the concentration of the fermentation product (for example fatty acids, carotenoids, (poly)saccharides, vitamins, isoprenoids, lipids, wax esters, and/or polymers like polyhydroxyalkanoates and/or its metabolism products or further desired fine chemical as mentioned herein) formed within a given time and a given fermentation volume (e.g., kg product per hour per liter) .

The term "efficiency" of production includes the time required for a particular level of production to be achieved (for example, how long it takes for the cell to attain a particular rate of output of a said acetyl CoA metabolism products, in particular, fatty acids, carotenoids, (poly)saccharides, vitamins, isoprenoids, lipids, wax esters, polyhydroxyalkanoates etc.).

The term "yield" or "product/carbon yield" is art-recognized and includes the efficiency of the conversion of the carbon source into the product (i.e. acetyl CoA, fatty acids, carotenoids, (poly)saccharides, vitamins, isoprenoids, lipids, wax esters, polyhydroxyalkanoates etc. and/or further compounds as defined above and which biosynthesis is based on said products). This is generally written as, for example, kg product per kg carbon source. By increasing the yield or production of the compound, the quantity of recovered molecules, or of useful recovered molecules of that compound in a given amount of culture over a given amount of time is increased.

The terms "biosynthesis" (which is used synonymously for "synthesis" of "biological production" in cells, tissues plants, etc.) or a "biosynthetic pathway" are art-recognized and include the synthesis of a compound, preferably an organic compound, by a cell from intermediate compounds in what may be a multistep and highly regulated process.

The language "metabolism" is art-recognized and includes the totality of the biochemical reactions that take place in an organism. The metabolism of a particular compound, then, (e.g., the metabolism of acetyl CoA, an fatty acid, hexose, lipid, isoprenoid, wax esteres, vitamin, polyhydroxyalkanoate etc.) comprises the overall biosynthetic, modification, and degradation pathways in the cell related to this compound.

Preferably, the polypeptide of the invention comprises one of the nucleotide sequences shown in SEQ ID No:2. The sequence of SEQ ID No:2 corresponds to the Euglena gracilis PNO cDNAs of the invention.

Further, the polynucleotide of the invention comprises a nucleic acid molecule which is a complement of one of the nucleotide sequences of above mentioned polynucleotides or a portion thereof. A nucleic acid molecule which is complementary to one of the nucleotide sequences shown in SEQ ID No:2 is one which is sufficiently complementary to one of the nucleotide sequences shown in SEQ ID No:2 such that it can hybridize to one of the nucleotide sequences shown in SEQ ID No:2, thereby forming a stable duplex.

The polynucleotide of the invention comprises a nucleotide sequence which is at least about 60%, preferably at least about 65-70%, more preferably at least about 70-80%, 80-90%, or 90-95%, and even more preferably at least about 95%, 96%, 97%, 98%, 99% or more homologous to a nucleotide sequence shown in SEQ ID No:2 A, or a portion thereof. The polynucleotide of the invention comprises a nucleotide sequence which hybridizes, e.g., hybridizes under stringent conditions as defined herein, to one of the nucleotide sequences shown in SEQ ID No:2, or a portion thereof.

Moreover, the polynucleotide of the invention can comprise only a portion of the coding region of one of the sequences in SEQ ID No:2, for example a fragment which can be used as a probe or primer or a fragment encoding a biologically active portion of an PNO. The nucleotide sequences determined from the cloning of the PNO gene from E. gracilis allows for the generation of probes and primers designed for use in identifying and/or cloning PNO homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., in SEQ ID No. No:2, an anti-sense sequence of one of the sequences, e.g., set forth in SEQ ID No.: 2, or naturally occurring mutants thereof. Primers based on a nucleotide of invention can be used in PCR reactions to clone PNO homologues. Probes based on the PNO nucleotide sequences can be used to detect transcripts or genomic sequences encoding the same or homologous proteins. The probe can further comprise a label group attached thereto, e.g. the label group can be a radioisotope, a fluorescent compound, an enzyme, or an enzyme co-factor. Such probes can be used as a part of a genomic marker test kit for identifying cells which express an PNO, such as by measuring a level of an PNO-encoding nucleic acid molecule in a sample of cells, e.g., detecting PNO mRNA levels or determining whether a genomic PNO gene has been mutated or deleted.

The polynucleotide of the invention encodes a polypeptide or portion thereof which includes an amino acid sequence which is sufficiently homologous to an amino acid sequence of SEQ ID No:1 such that the protein or portion thereof maintains the ability to participate in the synthesis of acetyl CoA, in particular a PNO activity as described in the examples in microorganisms or plants. As used herein, the language "sufficiently homologous" refers to proteins or portions thereof which have amino acid sequences which include a minimum number of identical or equivalent (e.g., an amino acid residue which has a similar side chain as an amino acid residue in one of the sequences of the polypeptide of the present invention amino acid residues to an amino acid sequence of Seq. ID No.: 1 such that the protein or portion thereof is able to participate in the synthesis of acetyl-CoA in microorganisms or plants. Examples of a PNO activity are also described herein. Thus, the function of an PNO contributes either directly or indirectly to the yield, production, and/or efficiency of production of acetyl CoA or products of pathways, wherein acetyl CoA is an educt, e.g., fatty acids, carotenoids, (poly)saccharides, vitamins, isoprenoids, lipids, wax esters, polyhydroxyalkanoate and/or one or more of said further products of their metabolism.

The protein is at least about 60-65%, preferably at least about 66-70%, and more preferably at least about 70-80%, 80-90%, 90-95%, and most preferably at least about 96%, 97%, 98%, 99% or more homologous to an entire amino acid sequence of SEQ ID No:2.

Portions of proteins encoded by the PNO polynucleotide of the invention are preferably biologically active portions of one of the PNO.

As mentioned herein, the term "biologically active portion of PNO" is intended to include a portion, e.g., a domain/motif, that participates in the metabolism of acetyl-CoA or has an immunological activity such that it is binds to an antibody binding specifially to PNO, e.g., it has an activity as set forth in teh Examples. To determine whether an PNO or a biologically active portion thereof can participate in the metabolism an assay of enzymatic activity may be performed. Such assay methods are well known to those skilled in the art, as detailed in the Examples. Additional nucleic acid fragments encoding biologically active portions of an PNO can be prepared by isolating a portion of one of the sequences in SEQ ID No:2, expressing the encoded portion of the PNO or peptide (e.g., by recombinant expression *in vitro)* and assessing the activity of the encoded portion of the PNO or peptide.

The invention further encompasses polynucleotides that differ from one of the nucleotide sequences shown in SEQ ID No:2 (and portions thereof) due to degeneracy of the genetic code and thus encode a PNO as that encoded by the nucleotide sequences shown in SEQ ID No:2. Further the polynucleotide of the invention has a nucleotide sequence encoding a protein having an amino acid sequence shown in SEQ ID No:1. In a still further embodiment, the polynucleotide of the invention encodes a full length E. gracilis protein which is substantially homologous to an amino acid sequence of SEQ ID No:1.

In addition, it will be appreciated by those skilled in the art that DNA sequence polymorphisms that lead to changes in the amino acid sequences may exist within a population (e.g., the E. gracilis population). Such genetic polymorphism in the PNO gene may exist among individuals within a population due to natural variation.

As used herein, the terms "gene" and "recombinant gene" refer to nucleic acid molecules comprising an open reading frame encoding an PNO, preferably a E. gracilis PNO. Such natural variations can typically result in 1-5% variance in the nucleotide sequence of the PNO gene. Any and all such nucleotide variations and resulting amino acid polymorphisms in PNO that are the result of natural variation and that do not alter the functional activity of PNO are intended to be within the scope of the invention.

Polynucleotides corresponding to natural variants and non-E. gracilis homologues of the PNO cDNA of the invention can be isolated based on their homology to E. gracilis PNO polynucleotides disclosed herein using the polynucleotide of the invention, or a portion thereof, as a hybridization probe according to standard hybridization techniques under stringent hybridization conditions. Accordingly, in another embodiment, an polynucleotide of the invention is at least 15 nucleotides in length. Preferably it hybridizes under stringent conditions to the nucleic acid molecule comprising a nucleotide sequence of the polynucleotide of the present invention, e.g. SEQ ID No:2. In other embodiments, the nucleic acid is at least 20, 30, 50, 100, 250 or more nucleotides in length. The term "hybridizes under stringent conditions" is defined above and is intended to describe conditions for hybridization and washing under which nucleotide sequences at least 60% identical to each other typically remain hybridized to each other. Preferably, the conditions are such that sequences at least about 65% or 70%, more preferably at least about 75% or 80%, and even more preferably at least about 85%, 90% or 95% or more identical to each other typically remain hybridized to each other. Preferably, polynucleotide of the invention that hybridizes under stringent conditions to a sequence of SEQ ID No:2 corresponds to a naturally-occurring nucleic acid molecule.

As used herein, a "naturally-occurring" nucleic acid molecule refers to an RNA or DNA molecule having a nucleotide sequence that occurs in nature (e.g., encodes a natural protein). Preferably, the polynucleotide encodes a natural E. gracilis PNO.

In addition to naturally-occurring variants of the PNO sequence that may exist in the population, the skilled artisan will further appreciate that changes can be introduced by mutation into a nucleotide sequence of the polynucleotide encoding PNO, thereby leading to changes in the amino acid sequence of the encoded PNO, without altering the functional ability of the PNO. For example, nucleotide substitutions leading to amino acid substitutions at "non-essential" amino acid residues can be made in a sequence of the polynucleotide encoding PNO, e.g. SEQ ID No:2. A "non-essential" amino acid residue is a residue that can be altered from the wild-type sequence of one of the PNO without altering the activity of said PNO, whereas an "essential" amino acid residue is required for PNO activity. Other amino acid residues, however, (e.g., those that are not conserved or only semi-conserved in the domain having PNO activity) may not be essential for activity and thus are likely to be amenable to alteration without altering PNO activity.

Accordingly, the invention relates to polynucleotides encoding PNO that contain changes in amino acid residues that are not essential for PNO activity. Such PNOs differ in amino acid sequence from a sequence contained in SEQ ID No:1 yet retain the PNO activity described herein. The polynucleotide can comprise a nucleotide sequence encoding a polypeptide, wherein the polypeptide comprises an amino acid sequence at least about 60% identical to an amino acid sequence of SEQ ID No:1 and is capable of participation in the synthesis of acetyl-CoA. Preferably, the protein encoded by the nucleic acid molecule is at least about 60-65% identical to the sequence in SEQ ID No:1, more preferably at least about 60-70% identical to one of the sequences in SEQ ID No:1, even more preferably at least about 70-80%, 80-90%, 90-95% homologous to the sequence in SEQ ID No:1, and most preferably at least about 96%, 97%, 98%, or 99% identical to the sequence in SEQ ID No:1.

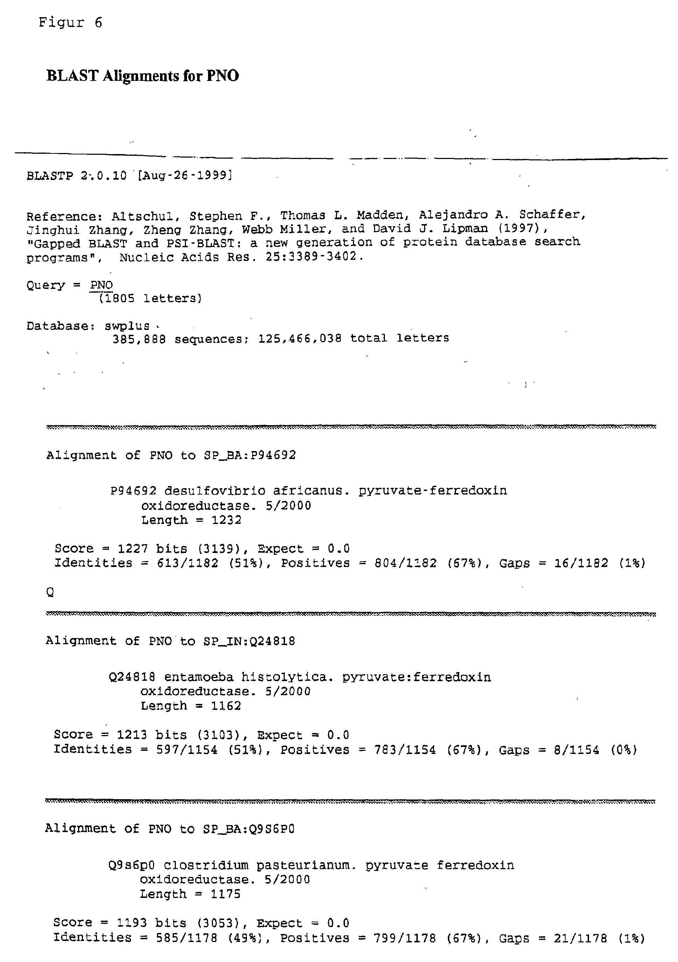

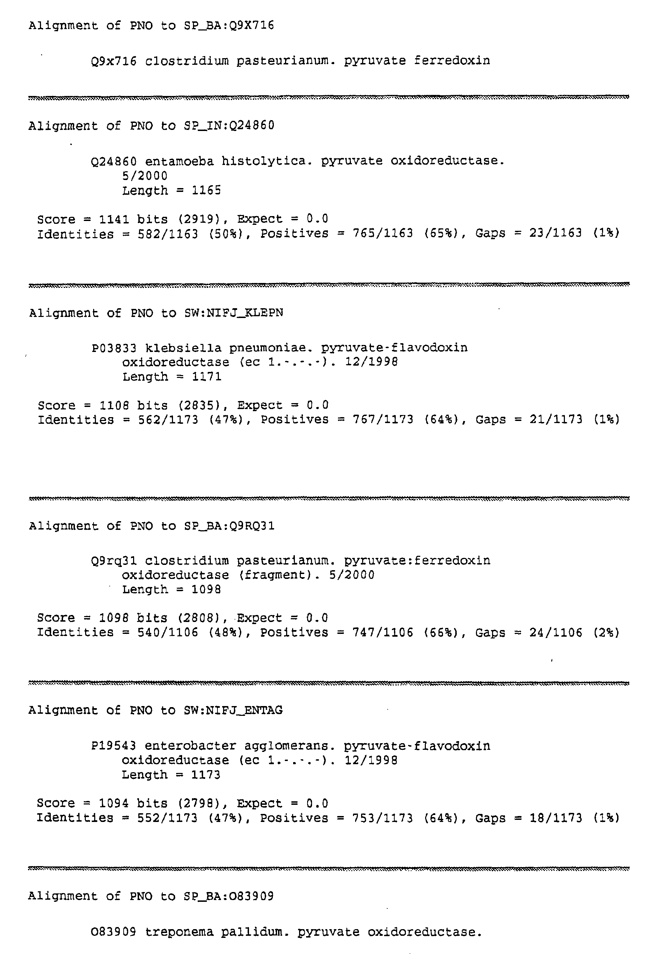

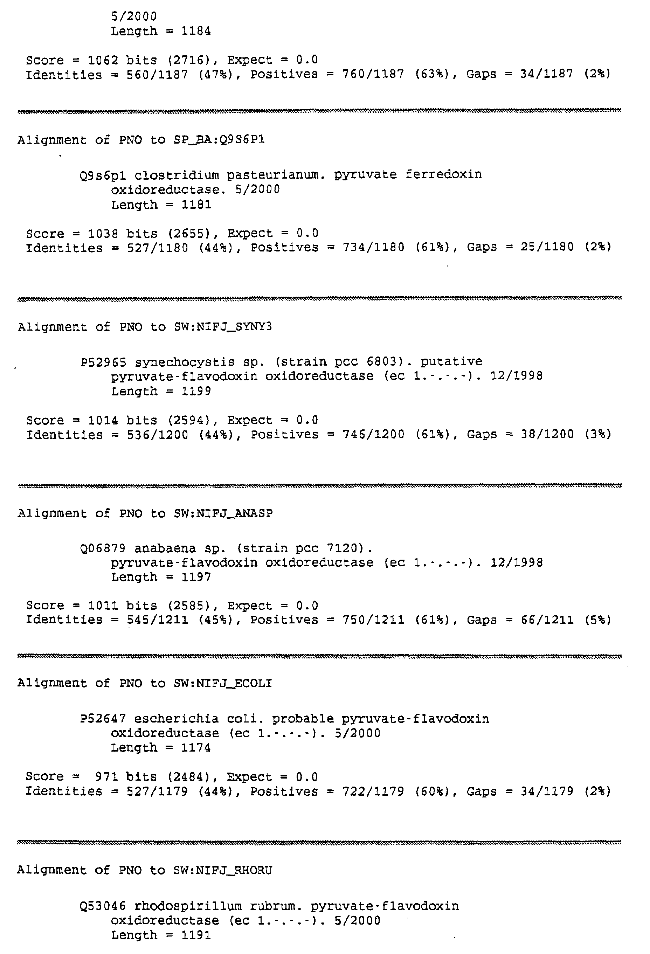

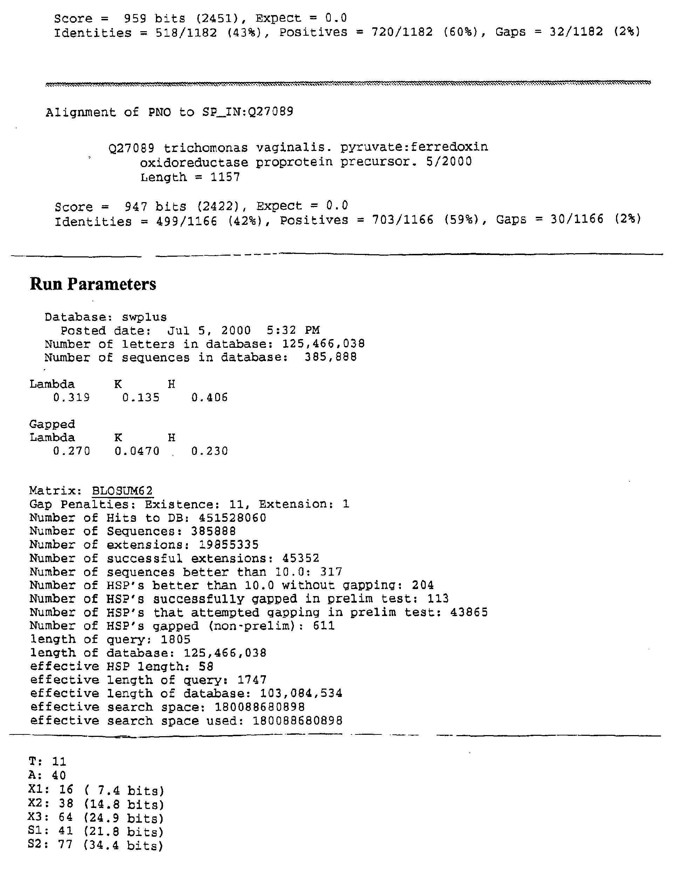

To determine the percent homology of two amino acid sequences (e.g., one of the sequences of Seq. ID No.: 1 and a mutant form thereof) or of two nucleic acids, the sequences are aligned for optimal comparison purposes (e.g., gaps can be introduced in the sequence of one protein or nucleic acid for optimal alignment with the other protein or nucleic acid). The amino acid residues or nucleotides at corresponding amino acid positions or nucleotide positions are then compared. When a position in one sequence (e.g., one of the sequences of SEQ ID No:1 or 2) is occupied by the same amino acid residue or nucleotide as the corresponding position in the other sequence (e.g., a mutant form of the sequence selected), then the molecules are homologous at that position (i.e., as used herein amino acid or nucleic acid "homology" is equivalent to amino acid or nucleic acid "identity"). The percent homology between the two sequences is a function of the number of identical positions shared by the sequences (i.e., % homology = numbers of identical positions/total numbers of positions x 100). The homology can be e.g. determined by computer programs as e.g. Blast 2.0. Fig. 6 shown the results of a blast search.

A nucleic acid molecule encoding an PNO homologous to a protein sequence of SEQ ID No:1 can be created by introducing one or more nucleotide substitutions, additions or deletions into a nucleotide sequence of the polynucleotide of the present invention, in particular of SEQ ID No: 2 such that one or more amino acid substitutions, additions or deletions are introduced into the encoded protein. Mutations can be introduced into the sequences of, e.g., SEQ ID No:2 by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in an PNO is preferably replaced with another amino acid residue from the same family. Alternatively, in another embodiment, mutations can be introduced randomly along all or part of an PNO coding sequence, such as by saturation mutagenesis, and the resultant mutants can be screened for an PNO activity described herein to identify mutants that retain PNO activity. Following mutagenesis of one of the sequences of SEQ ID No:2, the encoded protein can be expressed recombinantly and the activity of the protein can be determined using, for example, assays described herein (see Examples).

Accordingly, in one preferred embodiment the polynucleotide of the present invention is DNA or RNA.

A polynucleotide of the present invention, e.g., a nucleic acid molecule having a nucleotide sequence of Seq ID NO: 2, or a portion thereof, can be isolated using standard molecular biology techniques and the sequence information provided herein. For example, PNO cDNA can be isolated from a library using all or portion of one of the sequences of the polynucleotide of the present invention as a hybridization probe and standard hybridization techniques (e.g., as described in Sambrook et al., *Molecular Cloning: A Laboratory Manual. 2nd, ed., Cold Spring Harbor Laboratory,* Cold Spring Harbor Laboratory Press, Cold Spring Harbor, NY, 1989). Moreover, a polynucleotide encompassing all or a portion of one of the sequences of the polynucleotide of the present invention can be isolated by the polymerase chain reaction using oligonucleotide primers designed based upon this sequence (e.g., a nucleic acid molecule encompassing all or a portion of one of the sequences of polynucleotide of the present invention can be isolated by the polymerase chain reaction using oligonucleotide primers, e.g. of SEQ ID No:3 or 4, designed based upon this same sequence of polynucleotide of the present invention. For example, mRNA can be isolated from cells, e.g. Euglena (e.g., by the guanidinium-thiocyanate extraction procedure of Chirgwin et al. (1979) *Biochemistry* 18: 5294-5299) and cDNA can be prepared using reverse transcriptase (e.g., Moloney MLV reverse transcriptase, available from Gibco/BRL, Bethesda, MD; or AMV reverse transcriptase, available from Seikagaku America, Inc., St. Petersburg, FL). Synthetic oligonucleotide primers for polymerase chain reaction amplification can be designed based upon one of the nucleotide sequences shown in SEQ ID 100:2. A polynucleotide of the invention can be amplified using cDNA or, alternatively, genomic DNA, as a template and appropriate oligonucleotide primers according to standard PCR amplification techniques. The polynucleotide so amplified can be cloned into an appropriate vector and characterized by DNA sequence analysis. Furthermore, oligonucleotides corresponding to an PNO nucleotide sequence can be prepared by standard synthetic techniques, e.g., using an automated DNA synthesizer.

In another preferred embodiment of the invention the polynucleotide is operatively linked to a nucleic acid sequence encoding a signal sequence.

In the case that a nucleic acid molecule according to the invention is expressed in a cell it is in principle possible to modify the coding sequence in such a way that the protein is located in any desired compartment of the plant cell. These include the nucleus, endoplasmatic reticulum, the vacuole, the mitochondria, the plastids like amyloplasts, chloroplasts, chromoplasts, the apoplast, the cytoplasm, extracellular space, oil bodies, peroxisomes and other compartments of plant cells (for review see Kermode, Crit. Rev. Plant Sci. 15, 4 (1996), 285-423 and references cited therin). E. gracilis PNO bears a 37 amino acid long N-terminal transit peptide for the import into the mitochondria. The peptide sequence is indicated in Figure 1. In case the polypeptide of the present invention is to be imported into one of said further compartments, said PNO mitochondria transit signal can be mutated or deleted (which will be performed conveniently at the polynucleotide level). The polynucleotide can then operatively be fused to an appropriate polynucleotide, e.g., a vector, encoding a signal for the transport into the desirable compartment.

In general, the acetyl-CoA concentration can be altered in the cytoplasm of the cell due to the expression of PNO. However, since several pathways for the biosynthesis of important acetyl CoA based products, e.g., fatty acids, carotenoids, (poly)saccharides, vitamins, isoprenoids, lipids, wax esters, polyhydroxyalkanoates and/or their above defined metabolism compounds, take place in specialized cell organelles, i.e. plastids, corresponding signal sequences are introduced into the polynucleotide to direct the protein of the invention in the desirable compartment.

Methods how to carry out this modifications and signal sequences ensuring localization in a desired compartment are well known to the person skilled in the art.

The acetyl CoA concentration is advantageously increased in such a organelle or plastid due to the expression of the polynucleotide of the present invention. Consequently, the increased amounts of acetyl CoA are then mainly metabolized to fatty acids, carotenoids, (poly)saccharides, vitamins, isoprenoids, lipids, wax esters, polyhydroxyalkanoates and/or products, which are based on the metabolism of said compounds as defined above.

The increase of acetyl CoA in a cellular compartment might be achieved be coexpressing the polypeptide together with molecules involved in the transport of acetyl CoA into such a compartment, e.g. carnitine-acetyl CoA transferase. Preferably, the increase of acetyl CoA in plastids is achieved by expressing a PNO encoded by the polynucleotide of the present invention comprising further an appropriate signal sequence.

Accordingly, in one preferred embodiment the present invention relates to a polynucleotide wherein the signal sequence is a plastidal transit signal sequence.

Accordingly, preferably, the mitochondrial PNO transit signal is replaced by a plastidal transit signal sequence. For example, for the N-terminal basic amino acids of Arabidipsis PRPP-amidotransferase can be used as plastidal transit signal (Heijne, Eur. J. Biochem. 180, 1989, 535 - 545, Kermode, Crit. Rev. Plant. Sci. 15, 1996, 285 - 423). A sequence encoding such a signal sequence can be cloned in to a plant transformation vector as the vector of the present invention replacing, e.g. the existing signal sequence, i.e., the mitochondrial transit peptide.

In an other embodiment, the present invention relates to a method for making a recombinant vector comprising inserting a polynucleotide of the invention into a vector.

Further, the present invention relates to a recombinant vector containing the polynucleotide of the invention or produced by said method of the invention.

As used herein, the term "vector" refers to a nucleic acid molecule capable of transporting a polynucleotide to which it has been linked. One type of vector is a "plasmid", which refers to a circular double stranded DNA loop into which additional DNA segments can be ligated. Another type of vector is a viral vector, wherein additional DNA or PNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e.g., replication defective retroviruses, adenoviruses and adeno-associated viruses), which serve equivalent functions.

The present invention also relates to cosmids, viruses, bacteriophages and other vectors used conventionally in genetic engineering that contain a nucleic acid molecule according to the invention. Methods which are well known to those skilled in the art can be used to construct various plasmids and vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1989). Alternatively, the nucleic acid molecules and vectors of the invention can be reconstituted into liposomes for delivery to target cells.

In an other preferred embodiment to present invention relates to a vector in which the polynucleotide of the present invention is operatively linked to expression control sequences allowing expression in prokaryotic or eukaryotic host cells. The nature of such control sequences differs depending upon the host organism. In prokaryotes, control sequences generally include promoter, ribosomal binding site, and terminators. In eukaryotes, generally control sequences include promoters, terminators and, in some instances, enhancers, transactivators; or transcription factors.

The term "control sequence" is intended to include, at a minimum, components the presence of which are necessary for expression, and may also include additional advantageous components.

The term "operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences. In case the control sequence is a promoter, it is obvious for a skilled person that double-stranded nucleic acid is used.

Such regulatory sequences are described, for example, in Goeddel; Gene Expression Technology: Methods in Enzymology 185, Academic Press, San Diego, CA (1990) or see: Gruber and Crosby, in: Methods in Plant Molecular Biology and Biotechnolgy, CRC Press,Boca Raton, Florida, eds.:Glick and Thompson, Chapter 7, 89-108 including the references therein. Regulatory sequences include those which direct constitutive expression of a nucleotide sequence in many types of host cell and those which direct expression of the nucleotide sequence only in certain host cells or under certain conditions. It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by polynucleotides as described herein.

The recombinant expression vectors of the invention can be designed for expression of PNO in prokaryotic or eukaryotic cells. For example, genes encoding the polynucleotide of the invention can be expressed in bacterial cells such as E. coli, *C. glutamicum,* insect cells (using baculovirus expression vectors), yeast and other fungal cells (see Romanos, M.A. et al. (1992) Foreign gene expression in yeast: a review, *Yeast* 8: 423-488; van den Hondel, C.A.M.J.J. et al. (1991) Heterologous gene expression in filamentous fungi, in: More Gene Manipulations in Fungi, J.W. Bennet & L.L. Lasure, eds., p. 396-428: Academic Press: San Diego; and van den Hondel, C.A.M.J.J. & Punt, P.J. (1991) Gene transfer systems and vector development for filamentous fungi, in: Applied Molecular Genetics of Fungi, Peberdy, J.F. et al., eds., p. 1-28, Cambridge University Press: Cambridge), algae (Falciatore et al., 1999, Marine Biotechnology.1, 3:239-251), ciliates of the types: Holotrichia, Peritrichia, Spirotrichia, Suctoria, Tetrahymena, Paramecium, Colpidium, Glaucoma, Platyophrya, Potomacus, Pseudocohnilembus, Euplotes, Engelmaniella, and Stylonychia, especially of the genus Stylonychia lemnae with vectors following a transformation method as described in WO9801572 and multicellular plant cells (see Schmidt, R. and Willmitzer, L. (1988), High efficiency *Agrobacterium* tumefaciens-mediated transformation of *Arabidopsis thaliana* leaf and cotyledon explants, *Plant Cell Rep.:* 583-586); Plant Molecular Biology and Biotechnology, C Press, Boca Raton, Florida, chapter 6/7, S.71-119 (1993); F.F. White, B. Jenes et al., Techniques for Gene Transfer, in: Transgenic Plants, Vol. 1, Engineering and Utilization, eds.:Kung und R. Wu, Academic Press (1993), 128-43; Potrykus, Annu. Rev. Plant Physiol. Plant Molec. Biol. 42 (1991), 205-225 (and references cited therein) or mammalian cells. Suitable host cells are discussed further in Goeddel, *Gene Expression Technology: Methods in Enzymology* 185, Academic Press, San Diego, CA (1990). Alternatively, the recombinant expression vector can be transcribed and translated *in vitro,* for example using T7 promoter regulatory sequences and T7 polymerase.

Expression of proteins in prokaryotes is most often carried out with vectors containing constitutive or inducible promoters directing the expression of either fusion or non-fusion proteins. Fusion vectors add a number of amino acids to a protein encoded therein, usually to the amino terminus of the recombinant protein but also to the C-terminus or fused within suitable regions in the proteins. Such fusion vectors typically serve three purposes: 1) to increase expression of recombinant protein; 2) to increase the solubility of the recombinant protein; and 3) to aid in the purification of the recombinant protein by acting as a ligand in affinity purification. Further, the fusion vector can also encode for additional proteins, which expression supports an increase of metabolic products of acetyl CoA in a cell, for example transporters, which provide an increase of precursors in a cell or a compartment of a cell or which transport the product of a metabolic pathway based on acetyl CoA. Other enzymes are well know to a person skilled in the art and include enlongases, carboxylases, decarboxylases, synthases, synthetases, dehydrogenases etc., e.g. involved in plant fatty acid biosynthesis, desaturation, lipid metabolism and membrane transport of lipoic compounds, beta-oxidation, fatty acid modification, etc. of educts and products of acetyl CoA based metablosims. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase.

Typical fusion expression vectors include pGEX (Pharmacia Biotech Inc; Smith, D.B. and Johnson, K.S. (1988) Gene 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein. In one embodiment, the coding sequence of the polypeptide encoded by the polynucleotide of the present invention is cloned into a pGEX expression vector to create a vector encoding a fusion protein comprising, from the N-terminus to the C-terminus, GST-thrombin cleavage site-X protein. The fusion protein can be purified by affinity chromatography using glutathione-agarose resin. E.g. recombinant PNO unfused to GST can be recovered by cleavage of the fusion protein with thrombin.

Examples of suitable inducible non-fusion *E. coli* expression vectors include pTrc (Amann et al., (1988) *Gene* 69:301-315) and pET 11d (Studier et al., *Gene Expression Technology: Methods in Enzymology* 185, Academic Press, San Diego, California (1990) 60-89). Target gene expression from the pTrc vector relies on host RNA polymerase transcription from a hybrid trp-lac fusion promoter. Target gene expression from the pET 11d vector relies on transcription from a T7 gn10-lac fusion promoter mediated by a coexpressed viral RNA polymerase (T7 gnl). This viral polymerase is supplied by host strains BL21(DE3) or HMS174(DE3) from a resident λ prophage harboring a T7 gn1 gene under the transcriptional control of the lacUV 5 promoter.

One strategy to maximize recombinant protein expression is to express the protein in a host bacteria with an impaired capacity to proteolytically cleave the recombinant protein (Gottesman, S., *Gene Expression Technology: Methods in Enzymology* 185, Academic Press, San Diego, California (1990) 119-128). Another strategy is to alter the nucleic acid sequence of the nucleic acid to be inserted into an expression vector so that the individual codons for each amino acid are those preferentially utilized in the bacterium chosen for expression, such as E. coli or *C. glutamicum* (Wada et al. (1992) *Nucleic Acids Res.* 20:2111-2118). Such alteration of nucleic acid sequences of the invention can be carried out by standard DNA synthesis techniques.

Further, the PNO vector can be a yeast expression vector. Examples of vectors for expression in yeast *S. cerivisae* include pYepSec1 (Baldari, et al., (1987) *Embo J.* 6:229-234), pMFa (Kurjan and Herskowitz, (1982) *Cell* 30:933-943), pJRY88 (Schultz et al., (1987) *Gene* 54:113-123), and pYES2 (Invitrogen Corporation, San Diego, CA). Vectors and methods for the construction of vectors appropriate for use in other fungi, such as the filamentous fungi, include those detailed in: van den Hondel, C.A.M.J.J. & Punt, P.J. (1991) "Gene transfer systems and vector development for filamentous fungi, in: Applied Molecular Genetics of Fungi, J.F. Peberdy, et al., eds., p. 1-28, Cambridge University Press: Cambridge.

Alternatively, the polynucleotide of the invention can be introduced in insect cells using baculovirus expression vectors. Baculovirus vectors available for expression of proteins in cultured insect cells (e.g., Sf 9 cells) include the pAc series (Smith et al. (1983) *Mol. Cell Biol.* 3:2156-2165) and the pVL series (Lucklow and Summers (1989) *Virology* 170:31-39).

Alternatively, the polynucleotide of the invention is introduced in mammalian cells using a mammalian expression vector. Examples of mammalian expression vectors include pCDM8 (Seed, B. (1987) *Nature* 329:840) and pMT2PC (Kaufman et al. (1987) *EMBO J.* 6:187-195). When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook, J., Fritsh, E. F., and Maniatis, T. *Molecular Cloning: A Laboratory Manual. 2nd, ed., Cold Spring Harbor Laboratory,* Cold Spring Harbor Laboratory Press, Cold Spring Harbor, NY, 1989.

The recombinant mammalian expression vector can be capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; Pinkert et al. (1987) *Genes Dev.* 1:268-277), lymphoid-specific promoters (Calame and Eaton (1988) *Adv. Immunol.* 43:235-275), in particular promoters of T cell receptors (Winoto and Baltimore (1989) *EMBO J.* 8:729-733) and immunoglobulins (Banerji et al. (1983) *Cell* 33:729-740; Queen and Baltimore (1983) *Cell* 33:741-748), neuron-specific promoters (e.g., the neurofilament promoter; Byrne and Ruddle (1989) *PNAS* 86:5473-5477), pancreas-specific promoters (Edlund et al. (1985) *Science* 230:912-916), and mammary gland-specific promoters (e.g., milk whey promoter; U.S. Patent No. 4,873,316 and European Application Publication No. 264,166). Developmentally-regulated promoters are also encompassed, for example the murine hox promoters (Kessel and Gruss (1990) *Science* 249:374-379) and the fetoprotein promoter (Campes and Tilghman (1989) *Genes Dev.* 3:537-546).

Preferably, the polynucleotide of the present invention is operatively linked to a plant expression control sequences, e.g. expression cassettes a plant expression cassette preferably contains regulatory sequences capable to drive gene expression in plants cells and which are operably linked so that each sequence can fulfil its function such as termination of transcription such as polyadenylation signals. Preferred polyadenylation signals are those originating from Agrobacterium tumefaciens t-DNA such as the gene 3 known as octopine synthase of the Ti-plasmid pTiACH5 (Gielen et al., EMBO J. 3 (1984), 835 ff) or functional equivalents therof but also all other terminators functionally active in plants are suitable.

As plant gene expression is very often not limited on transcriptional levels as plant expression cassette preferably contains other operably linked sequences like translational enhancers such as the overdrive-sequence containing the 5'-untranlated leader sequence from tobacco mosaic virus enhancing the protein per RNA ratio (Gallie et al 1987, Nucl. Acids Research 15:8693-8711).

Accordingly, the polynucleotide can be operably linked to an appropriate promoter conferring gene expression in a timely, cell or tissue specific manner. Preferrred are promoters driving constitutitive expression (Benfey et al., EMBO J. 8 (1989) 2195-2202) like those derived from plant viruses like the 35S CAMV (Franck et al., Cell 21(1980) 285-294), the 19S CaMV (see also US5352605 and WO8402913) or plant promoters like those from Rubisco small subunit described in US4962028.

Plant gene expression can also be facilitated via a chemically inducible promoter (for rewiew see Gatz 1997, Annu. Rev. Plant Physiol. Plant Mol. Biol., 48:89-108). Chemically inducible promoters are especially suitable if gene expression is wanted to occur in a time specific manner. Examples for such promoters are a salicylic acid inducible promoter (WO 95/19443), a tetracycline inducible promoter (Gatz et al., (1992) Plant J. 2, 397-404) and an ethanol inducible promoter (WO 93/21334).

Also promoters responding to biotic or abiotic stress conditions are suitable promoters such as the pathogen inducible PRP1-gene promoter (Ward et al., Plant. Mol. Biol. 22 (1993), 361-366), the heat inducible hsp80-promoter from tomato (US5187267), cold inducible alpha-amylase promoter from potato (WO9612814) or the wound-inducible pinII-promoter (EP375091).

Especially those promoters are preferred which confer gene expression in tissues and organs where lipid and oil biosynthesis occurs in seed cells such as cells of the endosperm and the developing embryo. Suitable promoters are the napin-gene promoter from rapeseed (US5608152), the USP-promoter from Vicia faba (Baeumlein et al., Mol Gen Genet, 1991, 225 (3):459-67), the oleosin-promoter from Arabidopsis (WO9845461), the phaseolin-promoter from Phaseolus vulgaris (US5504200), the Bce4-promoter from Brassica (WO9113980) or the legumin B4 promoter (LeB4; Baeumlein et al., 1992, Plant Journal, 2 (2):233-9) as well as promoters conferring seed specific expression in monocot plants like maize, barley, wheat, rye, rice etc. Suitable promoters to note are the lpt2 or 1pt1-gene promoter from barley (WO9515389 and WO9523230) or those desribed in WO9916890 (promoters from the barley hordein-gene, the rice glutelin gene, the rice oryzin gene, the rice prolamin gene, the wheat gliadin gene, wheat glutelin gene, the maize zein gene, the oat glutelin gene, the Sorghum kasirin-gene, the rye secalin gene).

Also especially suited are promoters that confer plastid-specific gene expression as plastids are the compartment where precursors and some end products of lipid biosynthesis are synthesized. Suitable promoters such as the viral RNA-polymerase promoter are described in WO9516783 and WO9706250 and the clpP-promoter from Arabidopsis described in WO9946394.

Further, the polynucleotide of the invention can be cloned into the expression vector in an antisense orientation. That is, the DNA molecule is operatively linked to a regulatory sequence in a manner which allows for expression (by transcription of the DNA molecule) of an RNA molecule which is antisense to PNO mRNA. Regulatory sequences operatively linked to a nucleic acid cloned in the antisense orientation can be chosen which direct the continuous expression of the antisense RNA molecule in a variety of cell types, for instance viral promoters and/or enhancers, or regulatory sequences can be chosen which direct constitutive, tissue specific or cell type specific expression of antisense RNA. The antisense expression vector can be in the form of a recombinant plasmid, phagemid or attenuated virus in which antisense nucleic acid molecules are produced under the control of a high efficiency regulatory region, the activity of which can be determined by the cell type into which the vector is introduced. For a discussion of the regulation of gene expression using antisense genes see Weintraub, H. et al., Antisense RNA as a molecular tool for genetic analysis, *Reviews - Trends in Genetics,* Vol. 1(1) 1986 and Mol et al., 1990, FEES Letters 268:427-430.

In one embodiment the present invention relates to a method of making a recombinant host cell comprising introducing the vector or the polynucleotide of the present invention into a host cell.

Vector DNA can be introduced into prokaryotic or eukaryotic cells via conventional transformation or transfection techniques. As used herein, the terms "transformation" and "transfection", conjugation and transduction are intended to refer to a variety of art-recognized techniques for introducing foreign nucleic acid (e.g., DNA) into a host cell, including calcium phosphate or calcium chloride co-precipitation, DEAE-dextran-mediated transfection, lipofection, natural competence, chemical-mediated transfer, or electroporation. Suitable methods for transforming or transfecting host cells including plant cells can be found in Sambrook, et al. (*Molecular Cloning: A Laboratory Manual. 2nd, ed., Cold Spring Harbor Laboratory,* Cold Spring Harbor Laboratory Press, Cold Spring Harbor, NY, 1989) and other laboratory manuals such as Methods in Molecular Biology, 1995, Vol. 44, Agrobacterium protocols, ed: Gartland and Davey, Humana Press, Totowa, New Jersey.

For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., resistance to antibiotics) is generally introduced into the host cells along with the gene of interest. Preferred selectable markers include those which confer resistance to drugs, such as G418, hygromycin and methotrexate or in plants that confer resistance towards a herbicide such as glyphosate or glufosinate. Nucleic acid encoding a selectable marker can be introduced into a host cell on the same vector as that encoding the polypeptide of the present invention or can be introduced on a separate vector. Cells stably transfected with the introduced nucleic acid can be identified by, for example, drug selection (e.g., cells that have incorporated the selectable marker gene will survive, while the other cells die).

To create a homologous recombinant microorganism, a vector is prepared which contains at least a portion of the polynucleotide of the present invention into which a deletion, addition or substitution has been introduced to thereby alter, e.g., functionally disrupt, the PNO gene. Preferably, this PNO gene is a E. gracilis PNO gene, but it can be a homologue from a related or different source. In a preferred embodiment, the vector is designed such that, upon homologous recombination, the endogenous PNO gene is functionally disrupted (i.e., no longer encodes a functional protein; also referred to as a knock-out vector). Alternatively, the vector can be designed such that, upon homologous recombination, the endogenous PNO gene is mutated or otherwise altered but still encodes functional protein (e.g., the upstream regulatory region can be altered to thereby alter the expression of the endogenous PNO). To create a point mutation via homologous recombination also DNA-RNA hybrids can be used known as chimeraplasty known from Cole-Strauss et al. 1999, Nucleic Acids Research 27(5):1323-1330 and Kmiec Gene therapy. 19999, American Scientist. 87(3):240-247.

The vector is introduced into a cell and cells in which the introduced polynucleotide gene has homologously recombined with the endogenous PNO gene are selected, using art-known techniques.

Further host cells can be produced which contain selection systems which allow for regulated expression of the introduced gene. For example, inclusion of the polynucleotide of the invention on a vector placing it under control of the lac operon permits expression of the polynucleotide only in the presence of IPTG. Such regulatory systems are well known in the art.

Preferably, the introduced nucleic acid molecule is foreign to the host cell.

By "foreign" it is meant that the nucleic acid molecule is either heterologous with, respect to the host cell, this means derived from a cell or organism with a different genomic background, or is homologous with respect to the host cell but located in a different genomic environment than the naturally occurring counterpart of said nucleic acid molecule. This means that, if the nucleic acid molecule is homologous with respect to the host cell, it is not located in its natural location in the genome of said host cell, in particular it is surrounded by different genes. In this case the nucleic acid molecule may be either under the control of its own promoter or under the control of a heterologous promoter. The vector or nucleic acid molecule according to the invention which is present in the host cell may either be integrated into the genome of the host cell or it may be maintained in some form extrachromosomally. In this respect, it is also to be understood that the nucleic acid molecule of the invention can be used to restore or create a mutant gene via homologous recombination (Paszkowski (ed.), Homologous Recombination and Gene Silencing in Plants. Kluwer Academic Publishers (1994)).

Accordingly, in another embodiment the present invention relates to a host cell genetically engineered with the polynucleotide of the invention or the vector of the invention.

The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

For example, an polynucleotide of the present invention can be introduced in bacterial cells such as insect cells, fungal cells or mammalian cells (such as Chinese hamster ovary cells (CHO) or COS cells), algae, ciliates, plant cells, fungi or other microorganims like *C. glutamicum*. Other suitable host cells are known to those skilled in the art. Preferred are E. coli, baculovirus, Agrobacterium or fungal cells are, for example, those of the genus Saccharomyces, e.g. those of the species S. cerevisiae.

Further, the host cell can also be transformed such that further enzymes and proteins are (over)expressed which expression supports an increase of acetyl CoA or of metabolic products of acetyl CoA in a cell, for example transporters, which provide an increase of precursors in a cell or a compartment of a cell or which transport the product of a metabolic pathway based on acetyl CoA. Other enzymes are well know to a person skilled in the art and include enlongases, synthases, synthetases, dehydrogenases etc., plant fatty acid biosynthesis, desaturation, lipid metabolism and membrane transport of lipoic compounds, beta-oxidation, fatty acid modification, of educts and products of acetyl CoA based metablosims.

Further preferred are cells of one of herein mentioned plants, in particular, of one of the above-mentioned oil producing plants, and/or maise, rice, soya, rape of sunflower.

In another embodiment, the present invention relates to a process for the production of a polypeptide having PNO activity comprising culturing the host cell of the invention and recovering the polypeptide encoded by said polynucleotide and expressed by the host cell from the culture or the cells.

The term "expression" means the production of a protein or nucleotide sequence in the cell. However, said term also includes expression of the protein in a cell-free system. It includes transcription into an RNA product, post-transcriptional modification and/or translation to a protein product or polypeptide from a Dna encoding that product, as well as possible post-translational modifications.

Depending on the specific constructs and conditions used, the protein may be recovered from the cells, from the culture medium or from both. For the person skilled in the art it is well known that it is not only possible to express a native protein but also to express the protein as fusion polypeptides or to add signal sequences directing the protein to specific compartments of the host cell, e.g., ensuring secretion of the protein into the culture medium, etc. Furthermore, such a protein and fragments thereof can be chemically synthesized and/or modified according to standard methods described, for example hereinbelow.

A host cell of the invention, such as a prokaryotic or eukaryotic host cell in culture, can be used to produce (i.e., express) the polypeptide encoded by the polynucleotide of the invention, preferably having a PNO activity. An alternate method can be applied in addition in plants by the direct transfer of DNA into developing flowers via electroporation or Agrobacterium medium gene transfer. Accordingly, the invention further provides methods for producing PNO using the host cells of the invention. In one embodiment, the method comprises culturing the host cell of invention in a suitable medium such that PNO is produced. Further, the method comprises isolating recovering PNO from the medium or the host cell.

The polypeptide of the present invention is preferably produced by recombinant DNA techniques. For example, a nucleic acid molecule encoding the protein is cloned into an expression vector (as described above), the expression vector is introduced into a host cell (as described above) and said polypeptide is expressed in the host cell. Said polypeptide can then be isolated from the cells by an appropriate purification scheme using standard protein purification techniques. Alternative to recombinant expression, the PNO polypeptide or peptide can be synthesized chemically using standard peptide synthesis techniques. Moreover, native PNO can be isolated from cells (e.g., endothelial cells), for example using the antibody of the present invention as described below, in particular, an anti-PNO antibody, which can be produced by standard techniques utilizing PNO or fragment thereof, i.e., the polypeptide of this invention.

In one embodiment, the present invention relates to a polypeptide having the amino acid sequence encoded by a polynucleotide of the invention or obtainable by a process of the invention.

The terms "protein" and "polypeptide" used in this application are interchangeable. "Polypeptide" refers to a polymer of amino acids (amino acid sequence) and does not refer to a specific length of the molecule. Thus peptides and oligopeptides are included within the definition of polypeptide. This term does also refer to or include post-translational modifications of the polypeptide, for example, glycosylations, acetylations, phosphorylations and the like. Included within the definition are, for example, polypeptides containing one or more analogs of an amino acid (including, for example, unnatural amino acids, etc.), polypeptides with substituted linkages, as well as other modifications known in the art, both naturally occurring and non-naturally occurring.

Preferably, the polypeptide is isolated. An "isolated" or "purified" protein or biologically active portion thereof is substantially free of cellular material when produced by recombinant DNA techniques, or chemical precursors or other chemicals when chemically synthesized.

The language "substantially free of cellular material" includes preparations of the polypeptide of the invention in which the protein is separated from cellular components of the cells in which it is naturally or recombinantly produced. In one embodiment, the language "substantially free of cellular material" includes preparations having less than about 30% (by dry weight) of "contaminating protein", more preferably less than about 20% of "contaminating protein", still more preferably less than about 10% of "contaminating protein", and most preferably less than about 5% "contaminating protein". The term "Contaminating protein" relates to polypeptides which are not polypeptides of the present invention. When the polypeptide of the present invention or biologically active portion thereof is recombinantly produced, it is also preferably substantially free of culture medium, i.e., culture medium represents less than about 20%, more preferably less than about 10%, and most preferably less than about 5% of the volume of the protein preparation. The language "substantially free of chemical precursors or other chemicals" includes preparations in which the polypeptide or of the present invention is separated from chemical precursors or other chemicals which are involved in the synthesis of the protein. The language "substantially free of chemical precursors or other chemicals" includes preparations having less than about 30% (by dry weight) of chemical precursors or non-PNO chemicals, more preferably less than about 20% chemical precursors or non-PNO chemicals, still more preferably less than about 10% chemical precursors or non-PNO chemicals, and most preferably less than about 5% chemical precursors or non-PNO chemicals. In preferred embodiments, isolated proteins or biologically active portions thereof lack contaminating proteins from the same organism from which the polypeptide of the present invention is derived. Typically, such proteins are produced by recombinant expression of, for a example, a E. gracilis PNO in a plant or a microorganisms such as E. coli or *C. glutamicum* or ciliates, algae or fungi.

A polypeptide of the invention can participate in the polypeptide or portion thereof comprises preferably an amino acid sequence which is sufficiently homologous to an amino acid sequence of SEQ ID No:1 such that the protein or portion thereof maintains the ability to synthesis acetyl-CoA. The portion of the protein is preferably a biologically active portion as described herein. Preferably, the polypeptide of the invention has an amino acid sequence identical as shown in SEQ ID No:1. Further, the polypeptide can have an amino acid sequence which is encoded by a nucleotide sequence which hybridizes, preferably hybridizes under stringent conditions as described above, to a nucleotide sequence of the polynucleotide of the present invention. Accordingly, the PNO has an amino acid sequence which is encoded by a nucleotide sequence that is at least about 60-65%, preferably at least about 66-70%, more preferably at least about 70-80%, 80-90%, 90-95%, and even more preferably at least about 96%, 97%, 98%, 99% or more homologous to one of the amino acid sequences of SEQ ID No:1. The preferred polypeptide of the present invention also preferably possess at least one of the PNO activities described herein, e.g. its enzymatic or immunological acitivities. For example, a preferred polypeptide of the present invention includes an amino acid sequence encoded by a nucleotide sequence which hybridizes, e.g., hybridizes under stringent conditions, to a nucleotide sequence of SEQ ID No:2 or which is homologous thereto, as defined above.

Accordingly the polypeptide of the present invention can from SEQ ID No:1 in amino acid sequence due to natural variation or mutagenesis, as described in detail herein. Accordingly, the polypeptide comprise an amino acid sequence which is at least about 60-65%, preferably at least about 66-70%, and more preferably at least about 70-80, 80-90, 90-95%, and most preferably at least about 96%, 97%, 98%, 99% or more homologous to an entire amino acid sequence of SEQ ID No:1.

Biologically active portions of an polypeptide of the present invention include peptides comprising amino acid sequences derived from the amino acid sequence of an PNO, e.g., the amino acid sequence shown in SEQ ID No:1 or the amino acid sequence of a protein homologous to an PNO, which include fewer amino acids than a full length PNO or the full length protein which is homologous to an PNO, and exhibit at least one activity of an PNO. Typically, biologically (or immunologically) active portions (peptides, e.g., peptides which are, for example, 5, 10, 15, 20, 30, 35, 36, 37, 38, 39, 40, 50, 100 or more amino acids in length) comprise a domain or motif with at least one activity or epitope of an PNO. Moreover, other biologically (or immunologically) active portions, in which other regions of the polypeptide are deleted, can be prepared by recombinant techniques and evaluated for one or more of the activities described herein. Preferably, the biologically active portions of the PNO include one or more selected domains/motifs or portions thereof having biological activity.

The invention also provides chimeric or fusion proteins.

As used herein, an "chimeric protein" or "fusion protein" comprises an polypeptide operatively linked to a non-PNO polypeptide.

An "PNO polypeptide" refers to a polypeptide having an amino acid sequence corresponding to polypeptide having a PNO activity (e.g. biological or immunological), whereas a "non-PNO polypeptide" refers to a polypeptide having an amino acid sequence corresponding to a protein which is not substantially homologous to the PNO, e.g., a protein which is different from the PNO and which is derived from the same or a different organism.

Within the fusion protein, the term "operatively linked" is intended to indicate that the PNO polypeptide and the non-PNO polypeptide are fused to each other so that both sequences fulfil the proposed function addicted to the sequence used. The non-PNO polypeptide can be fused to the N-terminus or C-terminus of the PNO polypeptide. For example, in one embodiment the fusion protein is a GST-LMRP fusion protein in which the PNO sequences are fused to the C-terminus of the GST sequences. Such fusion proteins can facilitate the purification of recombinant PNO. In another embodiment, the fusion protein is an PNO containing a heterologous signal sequence at its N-terminus. In certain host cells (e.g., mammalian host cells), expression and/or secretion of an PNO can be increased through use of a heterologous signal sequence.

Preferably, an PNO chimeric or fusion protein of the invention is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. The fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, for example, *Current Protocols in Molecular Biology,* eds. Ausubel et al. John Wiley & Sons: 1992). Moreover, many expression vectors are commercially available that already encode a fusion moiety (e.g., a GST polypeptide). An PNO-encoding polynucleotide can be cloned into such an expression vector such that the fusion moiety is linked in-frame to the PNO.

Furthermore, folding simulations and computer redesign of structural motifs of the protein of the invention can be performed using appropriate computer programs (Olszewski, Proteins 25 (1996), 286-299; Hoffman, Comput. Appl. Biosci. 11 (1995), 675-679). Computer modeling of protein folding can be used for the conformational and energetic analysis of detailed peptide and protein models (Monge, J. Mol. Biol. 247 (1995), 995-1012; Renouf, Adv. Exp. Med. Biol. 376 (1995), 37-45). In particular, the appropriate programs can be used for the identification of interactive sites of mitogenic cyplin and its receptor, its ligand or other interacting proteins by computer assistant searches for complementary peptide sequences (Fassina, Immunomethods (1994), 114-120. Further appropriate computer systems for the design of protein and peptides are described in the prior art, for example in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used for, e.g., the preparation of peptidomimetics of the protein of the invention or fragments thereof. Such pseudopeptide analogues of the, natural amino acid sequence of the protein may very efficiently mimic the parent protein (Benkirane, J. Biol. Chem. 271 (1996), 33218-33224). For example, incorporation of easily available achiral Q-amino acid residues into a protein of the invention or a fragment thereof results in the substitution of amide bonds by polymethylene units of an aliphatic chain, thereby providing a convenient strategy for constructing a peptidomimetic (Banerjee, Biopolymers 39 (1996), 769-777).

Superactive peptidomimetic analogues of small peptide hormones in other systems are described in the prior art (Zhang, Biochem. Biophys. Res. Commun. 224 (1996), 327-331). Appropriate peptidomimetics of the protein of the present invention can also be identified by the synthesis of peptidomimetic combinatorial libraries through successive amide alkylation and testing the resulting compounds, e.g., for their binding and immunological properties. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715.

Furthermore, a three-dimensional and/or crystallographic structure of the protein of the invention can be used for the design of peptidomimetic inhibitors of the biological activity of the protein of the invention (Rose, Biochemistry 35 (1996), 12933-12944; Rutenber, Bioorg. Med. Chem. 4 (1996),1545-1558).

In a further embodiment, the present invention relates to an antibody that binds specifically to the polypeptide of the present invention or parts, i.e. specific fragments or epitopes of such a protein.

The antibodies of the invention can be used to identify and isolate other PNOs and genes in any organism, preferably algae. These antibodies can be monoclonal antibodies, polyclonal antibodies or synthetic antibodies as well as fragments of antibodies, such as Fab, Fv or scFv fragments etc. Monoclonal antibodies can be prepared, for example, by the techniques as originally described in K6hler and Milstein, Nature 256 (1975), 495, and Galfr6, Meth. Enzymol. 73 (1981), 3, which comprise the fusion of mouse myeloma cells to spleen cells derived from immunized mammals.

Furthermore, antibodies or fragments thereof to the aforementioned peptides can be obtained by using methods which are described, e.g., in Harlow and Lane "Antibodies, A Laboratory Manual", CSH Press, Cold Spring Harbor, 1988. These antibodies can be used, for example, for the immunoprecipitation and immunolocalization of proteins according to the invention as well as for the monitoring of the synthesis of such proteins, for example, in recombinant organisms, and for the identification of compounds interacting with the protein according to the invention. For example, surface plasmon resonance as employed in the BlAcore system can be used to increase the efficiency of phage antibodies selections, yielding a high increment of affinity from a single library of phage antibodies which bind to an epitope of the protein of the invention (Schier, Human Antibodies Hybridomas 7 (1996), 97-105; Malmborg, J. Immunol. Methods 183 (1995), 7-13). In many cases, the binding phenomena of antibodies to antigens is equivalent to other ligand/anti-ligand binding.

In one embodiment, the present invention relates to an antisense nucleic acid molecule comprising the complementary sequence of any one of (a) to (l).

Methods to modify the expression levels and/or the activity are known to persons skilled in the art and include for instance overexpression, co-suppression, the use of ribozymes, sense and anti-sense strategies, gene silencing approaches. "Sense strand" refers to the strand of a double-stranded DNA molecule that is homologous to a mRNA transcript thereof. The "anti-sense strand" contains an inverted sequence which is complementary to that of the "sense strand".

An "antisense" nucleic acid molecule comprises a nucleotide sequence which is complementary to a "sense" nucleic acid molecule encoding a protein, e.g., complementary to the coding strand of a double-stranded cDNA molecule or complementary to an mRNA sequence. Accordingly, an antisense nucleic acid molecule can hydrogen bond to a sense nucleic acid molecule. The antisense nucleic acid molecule can be complementary to an entire PNO coding strand, or to only a portion thereof. Accordingly, an antisense nucleic acid molecule can be antisense to a "coding region" of the coding strand of a nucleotide sequence encoding an PNO. The term "coding region" refers to the region of the nucleotide sequence comprising codons which are translated into amino acid residues. Further, the antisense nucleic acid molecule is antisense to a "noncoding region" of the coding strand of a nucleotide sequence encoding PNO. The term "noncoding region" refers to 5' and 3' sequences which flank the coding region that are not translated into a polypeptide (i.e., also referred to as 5' and 3' untranslated regions).

Given the coding strand sequences encoding PNO disclosed herein, antisense nucleic acid molecules of the invention can be designed according to the rules of Watson and Crick base pairing. The antisense nucleic acid molecule can be complementary to the entire coding region of PNO mRNA, but can also be an oligonucleotide which is antisense to only a portion of the coding or noncoding region of PNO mRNA. For example, the antisense oligonucleotide can be complementary to the region surrounding the translation start site of PNO mRNA. An antisense oligonucleotide can be, for example, about 5, 10, 15, 20, 25, 30, 35, 40, 45 or 50 nucleotides in length. An antisense nucleic acid molecule of the invention can be constructed using chemical synthesis and enzymatic ligation reactions using procedures known in the art. For example, an antisense nucleic acid molecule (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the anti-sense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a polynucleotide has been subcloned in an antisense orientation (i.e., RNA transcribed from the inserted polynucleotide will be of an antisense orientation to a target polynucleotide of interest, described further in the following subsection).

The antisense nucleic acid molecules of the invention are typically administered to a cell or generated *in situ* such that they hybridize with or bind to cellular mRNA and/or genomic DNA encoding an PNO to thereby inhibit expression of the protein, e.g., by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. The anti-sense molecule can be modified such that it specifically binds to a receptor or an antigen expressed on a selected cell surface, e.g., by linking the antisense nucleic acid molecule to a peptide or an antibody which binds to a cell surface receptor or antigen. The antisense nucleic acid molecule can also be delivered to cells using the vectors described herein. To achieve sufficient intracellular concentrations of the antisense molecules, vector constructs in which the antisense nucleic acid molecule is placed under the control of a strong prokaryotic, viral, or eukaryotic including plant promoters are preferred.

Further embodiment, the antisense nucleic acid molecule of the invention can be an α-anomeric nucleic acid molecule. An α-anomeric nucleic acid molecule forms specific double-stranded hybrids with complementary RNA in which, contrary to the usual β-units, the strands run parallel to each other (Gaultier et al. (1987) *Nucleic Acids. Res.* 15:6625-6641). The antisense nucleic acid molecule can also comprise a 2'-o-methylribonucleotide (Inoue et al. (1987) *Nucleic Acids Res.* 15:6131-6148) or a chimeric RNA-DNA analogue (Inoue et al. (1987) *FEBS Lett.* 215:327-330).

Further the antisense nucleic acid molecule of the invention can be a ribozyme. Ribozymes are catalytic RNA molecules with ribonuclease activity which are capable of cleaving a single-stranded nucleic acid, such as an mRNA, to which they have a complementary region. Thus, ribozymes (e.g., hammerhead ribozymes (described in Haselhoff and Gerlach (1988) *Nature* 334:585-591)) can be used to catalytically cleave PNO mRNA transcripts to thereby inhibit translation of mRNA. A ribozyme having specificity for an PNO-encoding nucleic acid molecule can be designed based upon the nucleotide sequence of an PNO cDNA disclosed herein or on the basis of a heterologous sequence to be isolated according to methods taught in this invention. For example, a derivative of a *Tetrahymena* L-19 IVS RNA can be constructed in which the nucleotide sequence of the active site is complementary to the nucleotide sequence to be cleaved in an encoding mRNA. See, e.g., Cech et al. U.S. Patent No. 4,987,071 and Cech et al. U.S. Patent No. 5,116,742. Alternatively, PNO mRNA can be used to select a catalytic RNA having a specific ribonuclease activity from a pool of RNA molecules. See, e.g., Bartel, D. and Szostak, J.W. (1993) *Science* 261:1411-1418.

Alternatively, PNO gene expression can be inhibited by targeting nucleotide sequences complementary to the regulatory region of an PNO nucleotide sequence (e.g., an PNO promoter and/or enhancers) to form triple helical structures that prevent transcription of an PNO gene in target cells. See generally, Helene, C. (1991) *Anticancer Drug Des.* 6(6):569-84; Helene, C. et al. (1992) *Ann. N.Y. Acad. Sci.* 660:27-36; and Maher, L.J. (1992) *Bioassays* 14 (12) :807-15.

In addition, in one embodiment, the present invention relates to a method for the production of transgenic plants, plant cells or plant tissue comprising the introduction of the polynucleotide or the vector of the present invention into the genome of said plant, plant tissue or plant cell.

For the expression of the nucleic acid molecules according to the invention in sense or antisense orientation in plant cells, the molecules are placed under the control of regulatory elements which ensure the expression in plant cells. These regulatory elements may be heterologous or homologous with respect to the nucleic acid molecule to be expressed as well with respect to the plant species to be transformed.

In general, such regulatory elements comprise a promoter active in plant cells. To obtain expression in all tissues of a transgenic plant, preferably constitutive promoters are used, such as the 35 S promoter of CaMV (Odell, Nature 313 (1985), 810-812) or promoters of the polyubiquitin genes of maize (Christensen, Plant Mol. Biol. 18 (1982), 675-689). In order to achieve expression in specific tissues of a transgenic plant it is possible to use tissue specific promoters (see, e.g., Stockhaus, EMBO J. 8 (1989), 2245-2251). Known are also promoters which are specifically active in tubers of potatoes or in seeds of different plants species, such as maize, Vicia, wheat, barley etc. Inducible promoters may be used in order to be able to exactly control expression.

An example for inducible promoters are the promoters of genes encoding heat shock proteins. Also microspore-specific regulatory elements and their uses have been described (W096/16182). Furthermore, the chemically inducible Tet-system may be employed (Gatz, Mol. Gen. Genet. 227 (1991); 229-237). Further suitable promoters are known to the person skilled in the art and are described, e.g., in Ward (Plant Mol. Biol. 22 (1993), 361-366). The regulatory elements may further comprise transcriptional and/or translational enhancers functional in plants cells. Furthermore, the regulatory elements may include transcription termination signals, such as a poly-A signal, which lead to the addition of a poly A tail to the transcript which may improve its stability.

Methods for the introduction of foreign DNA into plants are also well known in the art. These include, for example, the transformation of plant cells or tissues with T-DNA using Agrobacterium turnefaciens or Agrobacterium rhizogenes, the fusion of protoplasts, direct gene transfer (see, e.g., EP-A 164 575), injection, electroporation, biolistic methods like particle bombardment, pollen-mediated transformation, plant RNA virus-mediated transformation, liposome-mediated transformation, transformation using wounded or enzyme-degraded immature embryos, or wounded or enzyme-degraded embryogenic callus and other methods known in the art. The vectors used in the method of the invention may contain further functional elements, for example "left border"- and "right border"-sequences of the T-DNA of Agrobacterium which allow for stably integration into the plant genome. Furthermore, methods and vectors are known to the person skilled in the art which permit the generation of marker free transgenic plants, i.e. the selectable or scorable marker gene is lost at a certain stage of plant development or plant breeding. This can be achieved by, for example cotransformation (Lyznik, Plant Mol. Biol. 13 (1989), 151-161; Peng, Plant Mol. Biol. 27 (1995), 91-104) and/or by using systems which utilize enzymes capable of promoting homologous recombination in plants (see, e.g., W097/08331; Bayley, Plant Mol. Biol. 18 (1992), 353-361); Lloyd, Mol. Gen. Genet, 242 (1994), 653-657; Maeser, Mol. Gen. Genet. 230 (1991), 170-176; Onouchi, Nucl. Acids Res. 19 (1991), 6373-6378). Methods for the preparation of appropriate vectors are described by, e.g., Sambrook (Molecular Cloning; A Laboratory Manual, 2nd Edition (1989), Cold Spring Harbor Laboratory Press, Cold Spring Harbor, NY).

Suitable strains of Agrobacterium tumefaciens and vectors as well as transformation of Agrobacteria and appropriate growth and selection media are well known to those skilled in the art and are described in the prior art (GV31 01 (pMK90RK), Koncz, Mol. Gen. Genet. 204 (1986), 383-396; C58C1 (pGV 3850kan), Deblaere, Nucl. Acid Res. 13 (1985), 4777; Bevan, Nucleic. Acid Res. 12(1984), 8711; Koncz, Proc. NatI. Acad. Sci. USA 86 (1989), 8467-8471; Koncz, Plant Mol. Biol. 20 (1992), 963-976; Koncz, Specialized vectors for gene tagging and expression studies. In: Plant Molecular Biology Manual Vol 2, Gelvin and Schilperoort (Eds.), Dordrecht, The Netherlands: Kluwer Academic Publ. (1994), 1-22; EP-A-120 516; Hoekema: The Binary Plant Vector System, Offsetdrukkerij Kanters B.V., Alblasserdam (1985), Chapter V, Fraley, Crit. Rev. Plant. Sci., 4, 1-46; An, EMBO J. 4 (1985), 277-287).

Although the use of Agrobacteriurn tumefaciens is preferred in the method of the invention, other Agrobacterium strains, such as Agrobacterium rhizogenes, may be used, for example if a phenotype conferred by said strain is desired.

Methods for the transformation using biolistic methods are well known to the person skilled in the art; see, e.g., Wan, Plant Physiol. 104 (1994), 37-48; Vasil, Bio/Technology 11 (1993), 1553-1558 and Christou (1996) Trends in Plant Science 1, 423-431. Microinjection can be performed as described in Potrykus and Spangenberg (eds.), Gene Transfer To Plants. Springer Verlag, Berlin, NY (1995).

The transformation of most dicotyledonous plants is possible with the methods described above. But also for the transformation of monocotyledonous plants several successful transformation techniques have been developed. These include the transformation using biolistic methods as, e.g., described above as well as protoplast transformation, electroporation of partially permeabilized cells, introduction of DNA using glass fibers, etc.

The term "transformation" as used herein, refers to the transfer of an exogenous polynucleotide into a host cell, irrespective of the method used for the transfer. The polynucleotide may be transiently or stably introduced into the host cell and may be maintained non-integrated, for example, as a plasmid or as chimeric links, or alternatively, may be integrated into the host genome. The resulting transformed plant cell can then be used to regenerate a transformed plant in a manner known by a skilled person.

In general, the plants which can be modified according to the invention and which either show overexpression of a protein according to the invention or a reduction of the synthesis of such a protein can be derived from any desired plant species. They can be monocotyledonous plants or dicotyledonous plants, preferably they belong to plant species of interest in agriculture, wood culture or horticulture interest, such as crop plants (e.g. maize, rice, barley, wheat, rye, oats etc.), potatoes, oil producing plants (e.g. oilseed rape, sunflower, pea nut, soy bean, etc.), cotton, sugar beet, sugar cane, leguminous plants (e.g. beans, peas etc.), wood producing plants, preferably trees, etc.

Further, in one embodiment, the present invention relates to a plant cell comprising the polynucleotide the vector or obtainable by the method of the present invention.

Thus, the present invention relates also to transgenic plant cells which contain (preferably stably integrated into the genome) a polynucleotide according to the invention linked to regulatory elements which allow expression of the polynucleotide in plant cells and wherein the polynucleotide is foreign to the transgenic plant cell. For the meaning of foreign; see supra.

The presence and expression of the polynucleotide in the transgenic plant cells modulates, preferably increases the synthesis of acetyl CoA and leads to physiological and, preferably, to phenotypic changes in plants containing such cells.

Thus, the present invention also relates to transgenic plants and plant tissue comprising transgenic plant cells according to the invention. Due to the (over)expression of a polypeptide of the invention, e.g., at developmental stages and/or in plant tissue, e.g., which are involved in the fatty acids, carotenoids, isoprenoids, vitamins, lipids, wax ester, (poly)saccharides and/or polyhydroxyalkanoates, and/or its metabolism products, in particular, steroid hormones, prostaglandin, cholesterol, triacylglycerols, bile acids, ketone bodies etc biosynthesis, these transgenic plants may show various physiological, developmental and/or morphological modifications in comparison to wild-type plants.

For example, to obtain transgenic plants expressing the PNO gene, its coding region can be cloned, e.g., into the pBinAR vector (Höfgen und Willmitzer, Plant-Science, 66, 1990, 221-230). For example, following a polymerase chain reaction (PCR) technology the coding region of PNO can be amplified using Primers as shown in the examples and figures, e.g., SEQ ID NO: 3 and SEQ ID NO: 4.

The obtained PCR fragment can be purified and subsequently the fragment can be cloned into a vector.

The resulted vector can be transferred into Agrobacterium turnefaciens. This strain can be used to transform and transgenic plants can then be selected in another embodiment, the present invention relates to a transgenic plant or plant tissue comprising the plant cell of the present invention.

Further, the plant cell, plant tissue or plant can also be transformed such that further enzymes and proteins are (over)expressed which expression supports an increase of acetyl CoA or of metabolic products of acetyl CoA in a cell, for example transporters, which provide an increase of precursors in a cell or a compartment of a cell or which transport the product of a metabolic pathway based on acetyl CoA. Other enzymes are well know to a person skilled in the art and include enlongases, synthases, synthetases, dehydrogenases etc., plant fatty acid biosynthesis, desaturation, lipid metabolism and membrane transport of lipoic compounds, beta-oxidation, fatty acid modification, of educts and products of acetyl CoA based metabolisms.

In particular, due to the commercial value of plants exhibiting a modified fatty acid elongation system, DANN sequences involved in said system, e.g. beta-ketoacyl-CoA synthases could be also be overexpressed in the plant cell, plant tissue, or plant but also in above mentioned host cell. Further, enzymes of the de novo fatty acid synthesis, which are localized in the plastids and involve intermediates bound to acyl carrier proteins can be overexpressed together with the polynucleotide of the present invention.

The present invention also relates to cultured plant tissues comprising transgenic plant cells as described above which show expression of a protein according to the invention.

Any transformed plant obtained according to the invention can be used in a conventional breeding scheme or in in vitro plant propagation to produce more transformed plants with the same characteristics and/or can be used to introduce the same characteristic in other varieties of the same or related species. Such plants are also part of the invention. Seeds obtained from the transformed plants genetically also contain the same characteristic and are part of the invention. As mentioned before, the present invention is in principle applicable to any plant and crop that can be transformed with any of the transformation method known to those skilled in the art and includes for instance corn, wheat, barley, rice, oilseed crops, cotton, tree species, sugar beet, cassava, tomato, potato, numerous other vegetables, fruits.

In a preferred embodiment, the transgenic plant or plant tissue of the present invention has an altered acetyl-CoA synthesis upon the presence of the polynucleotide or the vector.

In a further embodiment, the present invention relates to a method for modulating the acetyl-CoA synthesis in a host cell comprising providing the host cell or the steps of the method of the present invention and further culturing the cell under conditions which permit the expression of the polypeptide of the present invention.

In another, preferred embodiment, in the method of the present invention the expressed polypeptide is localized in the plant cell's plastid. Methods to achive a plastid localization of a foreign polypeptide, i.e. of PNO polypeptide, are described above. For example, transit signal sequences are fused with said polypeptide.

Further, in one embodiment the invention relates to a method for modulating the acetyl-CoA synthesis in a plant, plant tissue, or plant cell comprising providing the plant, plant tissue or plant cell of the invention or comprising the steps of the method of the invention and further culturing the plant, plant tissue or plant cell under condition which permits the expression of the polypeptide of the present invention.

In another embodiment, the present invention relates to the transgenic plant, the host cell or the method of the invention, wherein the acetyl CoA synthesis is increased.

Further, in one preferred embodiment the present invention relates to the transgenic plant, the host cell or the method of the present invention, wherein the synthesis of fatty acids, carotenoids, isoprenoids, vitamins, wax esters, lipids, (poly)saccharides, and/or polyhydroxyalkanoates is increased. Further, the biosynthesis of other products mentioned herein might also be increased. Thus, the present invention also relates to plants, host cells or methods, wherein the biosynthesis of compounds is increased which biosynthesis starts with one of above mentioned compounds, in particular, steroid hormones, cholesteral, prostaglandin, triacylglycerols, bile acids and/or ketone bodies. Preferred is also the increased synthesis of vitamine E.

In yet another aspect, the invention also relates to harvestable parts and to propagation material of the transgenic plants according to the invention which either contain transgenic plant cells expressing a nucleic acid molecule according to the invention or which contain cells which show a reduced level of the described protein.

Harvestable parts can be in principle any useful parts of a plant, for example, flowers, pollen, seedlings, tubers, leaves, stems, fruit, seeds, roots etc. Propagation material includes, for example, seeds, fruits, cuttings, seedlings, tubers, root-stocks etc.

In one embodiment, the present invention relates to a method for the production of fatty acids, carotenoids, (poly)saccharides, vitamins, isoprenoids, lipids, wax esters, and/or polyhydroxyalkanoates and/or its metabolism products, in particular, steroid hormones, cholesterol, triacylglycerols, bile acids and/or ketone bodies comprising the steps of the method of the present invention and further isolating said compounds from the cell, culture, plant or tissue.

In another embodiment, the present invention relates to the use of the polynucleotide, the vector, or the polypeptide of the present invention for making fatty acids, carotenoids, isoprenoids, vitamins, lipids, wax esters, (poly)saccharides and/or polyhydroxyalkanoates, and/or its metabolism products, in particular, steroid hormones, cholesterol, prostaglandin, triacylglycerols, bile acids and/or ketone bodies producing cells, tissues and/or plants.

Manipulation of the PNO polynucleotide of the invention may result in the production of PNOs having functional differences from the wild-type PNOs. These proteins may be improved in efficiency or activity, may be present in greater numbers in the cell than is usual, or may be decreased in efficiency or activity.

There are a number of mechanisms by which the alteration of an PNO of the invention may directly affect the yield, production, and/or efficiency of production of fatty acids, carotenoids, isoprenoids, vitamins, wax esters, lipids, (poly)saccharides and/or polyhydroxyalkanoates, and/or its metabolism products, in particular, steroid hormones, cholesterol, triacylglycerols, prostaglandin, bile acids and/or ketone bodies or further of above defined fine chemicals incorporating such an altered protein. Recovery of said compounds from large-scale cultures of *C. glutamicum, ciliates, algae or fungi* is significantly improved if the cell secretes the desired compounds, since such compounds may be readily purified from the culture medium (as opposed to extracted from the mass of *cultured* cells). In the case of plants expressing PNOs increased transport can lead to improved partitioning within the plant tissue and organs. By either increasing the expression of acetyl-CoA which is the basis for many products, e.g., fatty acids, carotenoids, isoprenoids, vitamines, lipids, (poly)saccharides, wax esters, and/or polyhydroxyalkanoates, and/ or its metabolism products, in particular, prostaglandin, steroid hormones, cholesterol, triacylglycerols, bile acids and/or ketone bodies in a cell, it may be possible to increase the amount of the produced said compounds thus permitting greater ease of harvesting and purification or in case of plants more efficient partitioning. Conversely, in order to efficiently overproduce acetyl-CoA and further one or more of said acetyl CoA metabolism products, increased amounts of the cofactors, precursor molecules, and intermediate compounds for the appropriate biosynthetic pathways maybe required. Therefore, by increasing the number and/ or activity of transporter proteins involved in the import of nutrients, such as carbon sources (i.e., sugars), nitrogen sources (i.e., amino acids, ammonium salts), phosphate, and sulfur, it may be possible to improve the production of acetyl CoA and its metabolism products as mentioned above, due to the removal of any nutrient supply limitations on the biosynthetic process. In particular, it may be possible to increase the yield, production, and/or efficiency of production of said compounds, e.g. fatty acids, carotenoids, isoprenoids, vitamins, was esters, lipids, (poly)saccharides, and/or polyhydroxyalkanoates, and/or its metabolism products, in particular, steroid hormones, cholesterol, prostaglandin, triacylglycerols, bile acids and/or ketone bodies molecules etc. in algae, plants, fungi or other microorganims like *C. glutamicum*.

The aforementioned mutagenesis strategies for PNO to result in increased yields of said compound are not meant to be limiting; variations on these strategies will be readily apparent to one skilled in the art. Using such strategies, and incorporating the mechanisms disclosed herein, the polynucleotide and polypeptide of the invention may be utilized to generate algae, ciliates, plants, fungi or other microorganims like *C. glutamicum* expressing wildtyp PNO or mutated PNO polynucleotide and protein molecules such that the yield, production, and/or efficiency of production of a desired compound is improved. This desired compound may be any natural product of algae, ciliates, plants, fungi or *C. glutamicum,* which includes the final products of biosynthesis pathways and intermediates of naturally-occurring metabolic pathways, as well as molecules which do not naturally occur in the metabolism of said cells, but which are produced by a said cells of the invention.

Furthermore, in one embodiment, the present invention relates to a method for the identification of an agonist or antagonist of PNO activity comprising
(a) contacting cells which express the polypeptide of the present invention with a candidate compound;
(b) assaying the PNO activity;
(c) comparing the PNO activity to a standard response made in the absence of the candidate compound; whereby, an increased PNO activity over the standard indicates that the compound is an agonist and a decreased PNO activity indicates that the compound is an antagonist.

Said compound may be chemically synthesized or microbiologically produced and/or comprised in, for example, samples, e.g., cell extracts from, e.g., plants, animals or microorganisms. Furthermore, said compound(s) may be known in the art but hitherto not known to be capable of suppressing or activating PNO. The reaction mixture may be a cell free extract or may comprise a cell or tissue culture. Suitable set ups for the method of the invention are known to the person skilled in the art and are, for example, generally described in Alberts et al., Molecular Biology of the Cell, third edition (1994), in particular Chapter 17. The compounds may be, e.g., added to the reaction mixture, culture medium, injected into the cell or sprayed onto the plant.

If a sample containing a compound is identified in the method of the invention, then it is either possible to isolate the compound from the original sample identified as containing the compound capable of suppressing or activating PNO, or one can further subdivide the original sample, for example, if it consists of a plurality of different compounds, so as to reduce the number of different substances per sample and repeat the method with the subdivisions of the original sample. Depending on the complexity of the samples, the steps described above can be performed several times, preferably until the sample identified according to the method of the invention only comprises a limited number of or only one substance(s). Preferably said sample comprises substances of similar chemical and/or physical properties, and most preferably said substances are identical. Preferably, the compound identified according to the above described method or its derivative is further formulated in a form suitable for the application in plant breeding or plant cell and tissue culture.

The compounds which can be tested and identified according to a method of the invention may be expression libraries, e.g., cDNA expression libraries, peptides, proteins, nucleic acids, antibodies, small organic compounds, hormones, peptidomimetics, PNAs or the like (Milner, Nature Medicine 1 (1995), 879-880; Hupp, Cell 83 (1995), 237-245; Gibbs, Cell 79 (1994), 193-198 and references cited supra). Said compounds can also be functional derivatives or analogues of known inhibitors or activators. Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art. Furthermore, peptidomimetics and/or computer aided design of appropriate derivatives and analogues can be used, for example, according to the methods described above. The cell or tissue that may be employed in the method of the invention preferably is a host cell, plant cell or plant tissue of the invention described in the embodiments hereinbefore.

Determining whether a compound is capable of suppressing or activating PNO can be done, as described in the examples. The inhibitor or activator identified by the above-described method may prove useful as a chemotherapeutikum and/or as a plant growth regulator. Thus, in a further embodiment the invention relates to a compound obtained or identified according to the method of the invention said compound being an antagonist or agonist of PNO.

Accordingly, in one embodiment, the present invention further relates to a compound identified by the method of the present invention.

Said compound is, for example, a homologous of PNO. Homologues of the PNO can be generated by mutagenesis, e.g., discrete point mutation or truncation of the PNO. As used herein, the term "homologue" refers to a variant form of the PNO which acts as an agonist or antagonist of the activity of the PNO. An agonist of the PNO can retain substantially the same, or a subset, of the biological activities of the PNO. An antagonist of the PNO can inhibit one or more of the activities of the naturally occurring form of the PNO, by, for example, competitively binding to a downstream or upstream member of the acetyl CoA metabolic cascade which includes PNO, or by binding to an PNO, thereby preventing activity.

In one embodiment, the invention relates to an antibody specifically recognizing the compound of the present invention.

The invention also relates to a diagnostic composition comprising at least one of the aforementioned polynucleotides, nucleic acid molecules, vectors, proteins, antibodies or compounds and optionally suitable means for detection.

It comprises isolation of mRNA from a cell and contacting the mRNA so obtained with a probe comprising a nucleic acid probe as described above under hybridizing conditions, detecting the presence of mRNA hybridized to the probe, and thereby detecting the expression of the protein in the cell. Further methods of detecting the presence of a protein according to the present invention comprises immunotechniques well known in the art, for example enzyme linked immunosorbent assay. Furthermore, it is possible to use the nucleic acid molecules according to the invention as molecular markers in plant breeding.

In another embodiment, the present invention relates to a pharmaceutical composition comprising the antisense nucleic acid molecule, the antibody or the compound of the invention and optionally a pharmaceutically acceptable carrier.

The pharmaceutical composition of the present invention may further comprise a pharmaceutically acceptable carrier, excipient and/or diluent. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabrochchial administration. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Proteinaceous pharmaceutically active matter may be present in amounts between 1 ng and 10 mg per dose; however, doses below or above this exemplary range are envisioned, especially considering the aforementioned factors. Administration of the suitable compositions may be effected by different ways, e.g., by intravenous, intraperitoneal, subcutaneous, intramuscular, topical or intradermal administration. If the regimen is a continuous infusion, it should be in the range of 1 µg to 10 mg units per kilogram of body weight per minute, respectively. Progress can be monitored by periodic assessment. The compositions of the invention may be administered locally or systemically. Administration will generally be parenterally, e.g., intravenously. The compositions of the invention may also be administered directly to the target site, e.g., by biolistic delivery ot an interal or external target site or by catheter to a site in an artery. Preparations for parenteral administration include sterile aqueous or non-aqueous solutions, suspensions, and emulsions. Example of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition of the invention may comprise further agents such as interleukins, interferons and/or CpG-containing DANN stretches, depending on the intended use of the pharmaceutical composition.

For example the pharmaceutical composition as defined herein is a vaccine.

In one embodiment the present invention relates to the use of the antisense nucleic acid molecule, the antibody, or the compound which is an antogonist of the invention for the preparation of a pharmaceutical composition for the treatment of parasite infections.

The PNO polypeptide can also find use as drug target and for the development of novels drugs. For example, Pyruvate:ferredoxin oxidoreductase is known as drug target in amitochondriate parasites. Metronidazole (1-(2-hydroxyethyl)-2-methyl-5-nitroimidazole) is the drug of choice used in chemotherapy for the treatment of infections caused by anaerobic or microaerophilic microorganisms (Freeman et al. 1997). The antimicrobial effect of this drug depends on its metabolic reduction within the target cell resulting in the release of reactive free radicals (Edwards, 1993). A common property of organisms susceptible to 5-nitroimidazoles is the presence of electron-generating and electron-transport systems which are able to transfer electrons to the nitro group of the drug. The drug enters the cell through passive diffusion, where it acts as a preferential electron acceptor. The electron-transport proteins providing the source of electrons for the reductive activation of metronidazole are involved in oxidative fermentation of pyruvate. Key proteins in this pathway are PFO, and some other enzymes like hydrogenase found specifically in microaerophilic bacteria and protozoan parasites. These proteins are lakking in the aerobic cell of the eukaryotic host.

Metronidazole replaces the protons as the acceptor of electrons donated by ferredoxin. In the absence of the drug, protons would normally be reduced to molecular hydrogen by the action of hydrogenase (Johnson 1993, Marczak et al. 1983, Yarlett et al. 1985). The importance of PFO and ferredoxin in drug activaton has been substantiated by data showing that certain strains of protozoa and bacteria that have become resistant to the drug have altered activities for either PFO (Britz and Wilkinson, 1979; Sindar et al. 1982; Cerkasovova et al. 1984) or ferredoxin (Yarlett et al 1986; Lloyd et al 1986; Quon et al. 1992)

The antimicrobial activity of reduced metronidazole is proposed to result from the reactivity of intermediates formed as the nitro group of the drug is reduced in single electron steps to a hydroxylamine. By analogy with the action of other free radicals it has been suggested that the toxic intermediates interact with various cellular components such as DNA, proteins and membranes (Johnson, 1993). Reduction of the nitro group of metronidazole has been correlated with DNA damage both in vivo and in vitro (Ings et al. 1974, Edwards 1986).

Treatment with metronidazole is usually very effective, however, metronidazole resistence is well documented for various bacteria and protozoan species (Johnson 1993; Sindar et al. 1982). Although the precise mechanisms underlying metronidazole resistance in different anaerobic protozoa and bacteria are unknown, studies indicate that many resistent strains appear to be altered in their ability to activate the drug. The activity of one or more proteins involved in drug activation is frequently either diminished or abolished (Johnson, 1993). These proteins include PFO, ferredoxin, terminal oxidase and hydrogenase. PFO as a key enzyme in drug activation will therefore also play an important role in the understanding and overcome of drug resistence in parasites.

Accordingly, parasites, e.g., plasmodium, in particular plasmodium falciparum, depend in some stages on an acetyl CoA synthesis via an PFO polypeptide, PNO polypeptide or related enzymes, which are homologous to the PNO polypeptide of the present invention. The parasites may have anaerobic or microaerophilic stages. Preferably, they can be treated with drugs, which specifically inhibit the activity of PFO or PNO or which are activated by the PFO or PNO pathway. Preferably, those drugs are not toxic to the host organisms/cells since they do not interact with PDH or related pathways.

Due to the conserved structures in PNO and PFO, the polypeptides of the present invention can be used to identify antagonists or agonists of PFO. Accordingly, the method of the present invention can comprise one or more further steps, relating to the identification of PFO antagonists, e.g., testing an PNO antagonist for its activity to inhibit PFO. Preferred are antagonists of parasites PFO, e.g. of plasmodium,

In another embodiment, the present invention relates to a kit comprising the polynucleotide of any one of claims 1 to 4, the vector of claim 6 or 7, the host cell of claim 9, the polypeptide of claim 12, the antisense nucleic acid of claim 14, the antibody of claim 13 or 31, plant cell of claim 16, the plant or plant tissue of claim 17, the harvestable part of claim 24, the propagation material of claim 25 or the compound of claim 30 or 31.

The compounds of the kit of the present invention may be packaged in containers such as vials, optionally with/in buffers and/or solution. If appropriate, one or more of said components may be packaged in one and the same container. Additionally or alternatively, one or more of said components may be adsorbed to a solid support as, e.g. a nitrocellulose filter, a glas plate, a chip, or a nylon membrane or to the well of a micro titerplate. The kit can be used for any of the herein described methods and embodiments, e.g. for the production of the host cells, transgenic plants, pharmaceutical compositions, detection of homologous sequences, identification of antagonists or agonists, etc.

Further, the kit can comprise instructions for the use of the kit for any of said embodiments, in particular for its use for modulating acetyl CoA biosynthesis in a host cell, plant cell, plant tissue or plant.

In another embodiment, the present invention relates to a method for the production of a pharmaceutical composition comprising the steps of the method of the present invention; and
(a) formulating the compound identified in step (c) in a pharmaceutically acceptable form.

The present invention also pertains to several embodiments relating to further uses and methods.

The polynucleotide, polypeptide, protein homologues, fusion proteins, primers, vectors, host cells, described herein can be used in one or more of the following methods: identification of E. gracilis and related organisms; mapping of genomes of organisms related to E. gracilis; identification and localization of E. gracilis sequences of interest; evolutionary studies; determination of PNO regions required for function; modulation of an PNO activity; modulation of the metabolism of acetyl-CoA and modulation of cellular production of the desired compound, such as fatty acids, carotenoids, isoprenoids, wax esters, vitamins, lipids, (poly)saccharides and/or polyhydroxyalkanoates, and/or its metabolism products, in particular, steroid hormones, prostaglandin, cholesterol, triacylglycerols, bile acids and/or ketone bodies.

Accordingly, the polynucleotides of the present invention have a variety of uses. First, they may be used to identify an organism as being E. gracilis or a close relative thereof. Also, they may be used to identify the presence of E. gracilis or a relative thereof in a mixed population of microorganisms. By probing the extracted genomic DNA of a culture of a unique or mixed population of microorganisms under stringent conditions with a probe spanning a region of a E. gracilis gene which is unique to this organism, one can ascertain whether this organism is present.

Further, the polynucleotide of the invention may be sufficiently homologous to the sequences of related species such that these nucleic acid molecules may serve as markers for the construction of a genomic map in related organism.

The polynucleotides of the invention are also useful for evolutionary and protein structural studies. By comparing the sequences of the PNO of the present invention to those encoding similar enzymes from other organisms, the evolutionary relatedness of the organisms can be assessed. Similarly, such a comparison permits an assessment of which regions of the sequence are conserved and which are not, which may aid in determining those regions of the protein which are essential for the functioning of the enzyme. This type of determination is of value for protein engineering studies and may give an indication of what the protein can tolerate in terms of mutagenesis without losing function.

These and other embodiments are disclosed and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on the Internet, for example under hftp://www.ncbi.nim.nih.gov/PubMed/medline.html. Further databases and addresses, such as hftp://www.ncbi.nlm.nih.gov/, hftp://www.infobiogen. fr/, hftp://www.fmi.ch/biology/research-tools.html, hftp://www.tigr.org/, are known to the person skilled in the art and can also be obtained using, e.g., hftp://www.lycos.com. An overview of patent information in biotechnology and a survey of relevant sources of patent information useful for retrospective searching and for current awareness is given in Berks, TIBTECH 12 (1994), 352-364.

The figures show:
Figure 1:
   (a) Processed amino-terminal leader sequences of *Trichomonas vaginalis* hydrogenosomal PFO and comparison of transit peptide regions from *Euglena gracilis* mitochondrial complex III and PNO. Solid lines denote the amino-termini of mature proteins isolated from the organelle or the organism. The determined NH₂-terminal amino acid sequences of both proteolytic fragments of *Euglena* PNO are underlined. EgPNOmt, *Euglena* mitochondrial PNO; CIII, mitochondrial complex III; SU, subunit. (Hrdy and Müller 1995, Cui et al. 1994, Inui et al. 1991).
   (b) Southern-blot analysis of the PNO gene in *E. gracilis* genomic DNA; 20 µg of nuclear DNA was digested with HindIII *(lane 1)*, KpnI *(lane 2),* EcoRI *(lane 3)* and SalI *(lane 4)*. The probe was the 700 bp amplification product obtained with degenerated PCR-primers against PNO from *E. gracilis.* Numbers on the left indicate the size (kb) of DNA markers.
   (c) Northern-blot analysis of RNA from *E. gracilis* extracted from cells grown under aerobic and anaerobic conditions (light and dark). The blot was loaded with 5 µg per lane and probed with pEgPNO3.
Figure 2:
   Structural model of the *E. gracilis* PFO/CPR fusion protein. The flow of electrons can be predicted to be from pyruvate to TPP, to the conserved [4Fe-4S] clusters of the PFO domain, to FMN, to FAD and finally to NADP⁺ bound to the corresponding domains of the C-terminal CPR fusion. [Fe-S], iron sulfur cluster; FAD, ferredoxin adenine dinucleotide; FMN, ferredoxin mononucleotide; TPP, thiamine pyrophosphate
Figure 3:
   Sequence similarity among the PFO and CPR domains of PNO. (a) Modular domain structure of the *Desulvovibrios* PFO (Charon et al 1999) and NADPH:cytochromeP450 reductase from rat liver microsomes (Wang et al 1997) inferred from their crystal structure. Solid cycles denote each one conserved cysteinyl residue implicated in binding the iron-sulfur centers; small square indicates the conserved Gly-Asp-Gly of the beginning of the putative TPP binding motif. (b) Deduced domain organization of homodimeric eukaryotic PFO, eubacterial PFO and nifJ and *Chlorobium* PFO/PS(pyruvate synthase). (c) Domain structure of heterotetrameric achaebacterial PFO/PS(pyruvate synthase) and heterotetrameric eubacterial PFO from *Thermotoga* and *Helicobacter.* (d) *Euglena* PNO fusion protein consisting of a complete PFO and NADPH:cytochrome P450 reductase with an N-terminal ∼40 amino acid mitochondrial transit peptide (T). Large asteriks denote the determined amino-termini of the PNO- and CPR domain (Inui et al. 1991); arrows indicate the primers used for RT-PCR. (e) Patterns of similarity revealed by BLAST and DOTPLOT (GCG) between PNO and hypothetical proteins from the *S. cerevisiae genome* annotated as putative sulfite and the *S. pombe* genome. (f) Patterns of similarity revealed by BLAST and DOTPLOT (GCG) between PNO and a protein termed MET10 (sufite reductase, ??subunit) both in yeast and its homologue in the *S. pombe* genome (T41439). (g) NADPH sulfite reductase (?-subunit) from *Salmonella* and *Thiocapsa.* (h) NADPH:cytochromeP450 reductase from eubacteria, fungi, plants and animals and NADPH:ferrihemoprotein reductase from fungi, plants and animals. (i) Fatty acid hydroxylase P450BM-3 from *Bacillus megaterium* (Govindaraj and Poulos, 1997) and *Fusarium oxysporum* (GenBank Ac. AB030037). (j) Metazoan nitric-oxide synthetase. (k,l) Ferredoxin:NADP reductase from cyanobacteria and plants and eubacterial and plant flavodoxin.
   Small asteriks denote regions which revealed no similarity to anything with BLAST, regions underlayed with grey indicate domains with no similarity to the above and beneath protein domains.
Figure 4:
   Scheme of metronidazole activation in an anaerobic parasite. In the presence of metronidazole, electrons generated by pyruvate:ferredoxin oxidoreductase (PFO) are transported by ferredoxin [2Fe-2S] to the drug and not to their natural acceptor hydrogenase (HY). Consequently, metronidazole reduction occurs while production of H₂ is ceased. The cytotoxic radicals (R-NO₂-) are formed as intermediate products of the drug reduction. (Kulda, 1999).
Figure 5:
   (a) polypeptide sequence of E. gracilis PNO (SEQ ID NO: 1),
   (b) polynucleotide sequence of E. gracilis PNO (SEQ ID NO: 2).
Figure 6:
   Results of blast search of E. gracilis PNO polypeptide sequence.
   This invention is further illustrated by the following examples which should not be construed as limiting. The contents of all references, patent applications, patents, and published patent applications cited throughout this application are hereby incorporated by reference.

### Examples:

### Example 1: General processes

### Growth conditions.

Euglena gracilis strain SAG 1224-5/25 was grown as 5 1 cultures under continuous light in Euglena medium with minerals (Botanica Acta(1997) 107: 111-186) in 10 l fermenters with aeration (2 l/ min). For aerobic growth, 2% CO₂ in air, for anaerobic growth, 2% CO₂ in N₂ was used. Cultures were harvested after four days. For dark treatment, Euglena cultures were grown two days in the light, subjected to darkness and harvested after two additional days.

### Molecular methods.

Messenger RNA isolation, cDNA synthesis and cloning in ?ZapII for *Euglena gracilis* were performed as described (Henze et al., 1996). A cDNA library was prepared from mRNA isolated from aerobically light-grown cells. A hybridization probe for PNO from *Euglena* was isolated by PCR against genomic DNA using combinations of oligonucleotides designed against the conserved amino acid motifs LFEDNEFG(F/W/Y)G (SEQ ID NO.: 8) and GGDGWAY-DIG(F/Y) (SEQ ID NO.: 9) identified through alignment of prokaryotic and eukaryotic PFO extracted from the databases. PCR was performed with a Perkin-Elmer thermocycler for one cycle of 95°C for 10 min and 29 cyclesof 95°C for 30 sec, 67°C for 30 sec, 72°C for 1 min in 10 mM Tris pH 8.3, 50 mM KCl, 2 mMMg²⁺, 50 µM of each dNTP, 40 pmol of each primer,10 ng of template DNA and 0.5 units of Taq polymerase (Qiagen) in a final volume of 25 µl. The primers pno1F953 5'-TITTYGARGAYAAYGCIGARTTYGGITTYGG-3' (SEQ ID NO: 3) and pno2R1095 5'-AAICCDATRTCRTAIGCCCAICCRTCICC-3' (SEQ ID NO: 4) yielded a ∼700 bp amplification product that was cloned, verified by sequencing and used as a hybridization probe for cDNA screening. Sequencing of clones so identified was determined using nested deletions and synthetic primers. Northerns (Hannaert et al, 2000) and standard molecular methods were performed as described (Sambrook et al. 1989).

Phylogenetic methods. Databases searching, sequence handling, sequence similarity searching and multiple alignment were performed with BLAST (Altschul et al. 1989) and with programs of the GCG Pakkage version 9.1 (Genetics Computer Group, Madison, WI, USA). Alignments were reinspected and adjusted manually.

Agrobacterium mediated plant transformation was performed using the GV3101(pMP90) (Koncz and Schell, Mol. Gen.Genet. 204 (1986), 383-396) Agrobacterium tumefaciens strain. Transformation cause performed by standard transformation techniques (Deblaere et al., Nucl. Acids. Tes. 13 (1984), 4777-4788).

### Plant transformation

Agrobacterium mediated plant transformation cause performed using standard transformation and regeneration techniques (Gelvin, Stanton B.; Schilperoort, Robert A, "Plant Molecular Biology Manual",2nd Ed. - Dordrecht : Kluwer Academic Publ., 1995. - in Sect., Ringbuc Zentrale Signatur: BT11-P ISBN 0-7923-2731-4; Glick, Bernard R.; Thompson, John E., "Methods in Plant Molecular Biology and Biotechnology", Boca Raton : CRC Press, 1993. - 360 S.,ISBN 0-8493-5164-2).

Rapeseed cause transformed via cotyledon transformation (Moloney et al., Plant cell Report 8 (1989), 238-242; De Block et al., Plant Physiol. 91 (1989, 694-701). Kanamycin was used for Agrobacterium and plant selection.

Transformation of soybean can be performed using for example a technique described in EP 0424 047, US 322 783 (Pioneer Hi-Bred International) or in EP 0397 687, US 5 376 543, US 5 169 770 (University Toledo).

Instead of Agrobacteria mediated plant transformation particle bombardment or Polyethylene Glycol mediated DNA uptake or via the Silicon Carbide Fiber technique can be used alternatively (Freeling and Walbot "The maize handbook" (1993)ISBN 3-540-97826-7, Springer Verlag New York).

### Example 2:

### Identity and Expression of Euglena mitochondrial PNO.

Previous biochemical work by Inui and colleagues on PNO from *Euglena* (Inui et al. 1987, 1990, 1991) suggested that the "pyruvate dehydrogenase active fragment" (Inui et al. 1991) released by tryptic digestion of the isolated enzyme corresponded to a PFO domain. The N-terminus of the E. gracilis PNO active enzyme (TSGPKPASXI, SEQ ID No.: 5;TSGPXPASXIEVSXAK, SEQ ID NO: 6) and the E. gracilis N-terminus of the PNO CPR domain (AAAPSGNXVTILYGSEEGNS, SEQ ID NO 7) have been determined by Inui et al, 1991. Said sequences are shown in Figure 1. However, PCR reactions with primes constructed on the polynucleotide sequences encoding said sequences did not result in any useful product. Accordingly, a new isolation strategy had to be developed. Using PCR with primers from conserved regions of PFO alignments with *Euglena* total DNA as a substrate, a 695 bp fragment was isolated that contained 300 bp of coding region, the deduced amino acid sequence of which shared roughly 50% amino acid identity to known PFO sequences from eukaryotes and eubacteria, and two introns of 221 and 174 bp. Using this probe, 12 independent positives were found among 300,000 cDNA clones that showed sequence similarity to PFO and were identical in overlapping regions. Two full-size cDNAs encoding PNO, pEgPNO3 and pEgPNO12, from mitochondria of the photosynthetic protist *Euglena gracilis* were isolated. pEgPNO3 was 15 bp shorter at the 5-'end and 12 bp shorter at the 3-'end of the cDNA in comparison to pEgPNO12. Both were proofed to be identical over a∼200 bp region at the 5-' and 3'-ends. The insert of pEgPNO3 is 5812 bp, encoding an ORF of 1803 amino acids (aa) corresponding to a protein with a calculated Mr of 199819 Da and extensive similarity to PFO in the N-terminal portion (∼1250 aa residues), and to NADPH:cytochromeP₄₅₀ reductases (CPR) and related proteins over the remaining C-terminal ∼550 aa (see below). Additionally, pEgPNO3 bears a 37 aa long N-terminal transit peptide for import into the mitochondrion. PNO from *Euglena* mitochondria thus consists of a translational fusion of a complete PFO and NADPH-cytochrome P₄₅₀ reductase.

The deduced proteins are identical with peptides previously determined from the active enzyme purified from *Euglena* mitochondria (Inui et al. 1991).

With the exception of one uncertain amino acid "X" at position 9, the first 12 residues of the N-terminal sequence of purified PNO from *Euglena* mitochondria (Inui et al. 1991) are identical to the sequence starting from amino acid position 38 of the protein encoded by pEgPNO3 (Fig. 1A). Furthermore, the fifteen unambiguous residues determined by amino acid sequencing from the N-terminus of the smaller tryptic fragment obtained from purified PNO, the "NADPH diaphorase active fragment" (Inui et al. 1991), are identical to the sequence starting from amino acid position 1249 of the protein encoded by pEgPNO3 (Fig. 1A). The identity of 27 amino acids stemming from two different regions of the purified protein to the deduced amino sequence of pEgPNO3 provides strong and direct evidence that the protein encoded by pEgPNO3 is the precursor of *Euglena* mitochondrial PNO (designated EgPNOmt) and that the cleavage site of the transit peptide is as indicated in Fig. 1A.

The frequency and identity of positives observed in cDNA screening suggested that *Euglena* expresses only one PNO gene under aerobic conditions in the light. A Southern blot of total *Euglena* DNA probed with pEgPNO3 and washed at low stringency (55°C in 2x SSPE, 0.1% SDS) revealed a very simple pattern, indicating the presence of one to at most three genes in the genome (Fig. 1B). A Northern blot loaded with 5 µg per lane of polyA+ Euglena RNA extracted from cells grown under aerobic and anaerobic conditions and probed with the ∼700 bp amplification product obtained by PCR with degenerate primers against PFO revealed that in the light the gene is strongly expressed under aerobic conditions but strongly reduced in anaerobically grown cells. Higher expression levels were found in anaerobically grown cultures transferred to the dark (Fig 1C). This PNO mRNA levels are in agreement with the finding that the PNO activity in *E. gracilis* is very high under both aerobic and anaerobic conditions, but the transition to anaerobiosis does not coincide with a dramatic change in PNO activity levels (Kitaoka et al. 1989).

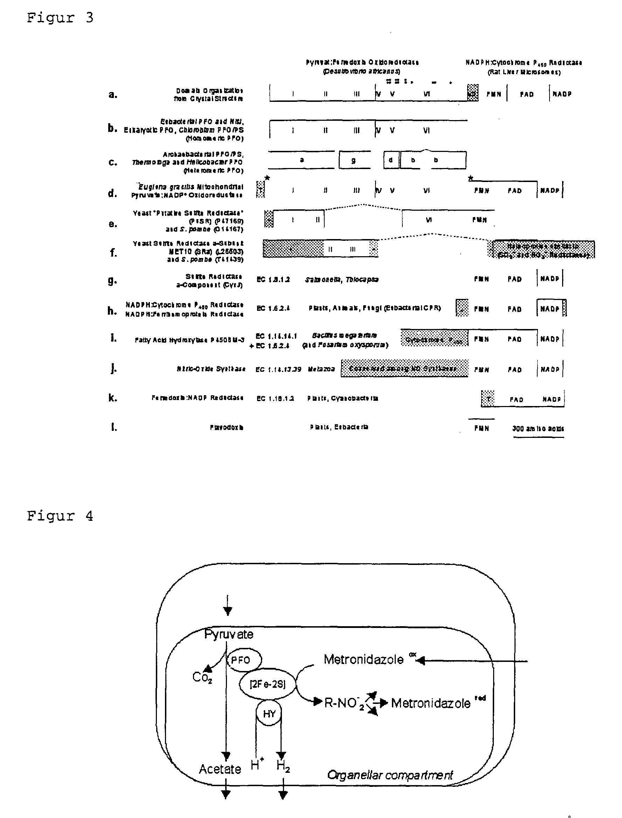

A structural model of the *E. gracilis* PFO/CPR fusion protein is shown in Fig. 2. In contrast to PDH, being an aggregate multi-enzyme complex of E1?, E1?, E2 and E3 proteins, PNO is a dimer of identical subunits. The flow of electrons within PNO can be predicted to be from pyruvate to TPP, to the conserved [4Fe-4S] clusters of the PFO-domain, and finally to NADP⁺ bound to the corresponding domains of the C-terminal CPR fusion (Inui et al. 1991).

### Example 3:

### Sequence similarity among the PFO and CPR domains of PNO.

Database searching of *Euglena* PNO and their constituent PFO and CPR domains revealed extremely complex patterns of sequence similarity, shared domains among proteins, gene fusions and apparent recombination events, as summarized in Fig. 3. An important guide to understanding these patterns are the functional domains of PFO from the *Desulfovibrio africanus* (Chabriere et al., 1999; Charon et al., 1999) and of rat microsomal NADPH-cytochrome P₄₅₀ reductase (Wang et al., 1997) inferred from their crystal structures (Fig. 3a). Although the fusion of a complete PFO and an NADPH-cytochrome P₄₅₀ reductase in EgPNOmt is unique among sequences reported to date (Fig. 3d), partial fusions of the two domains are found among eukaryotes. Fig. 3e shows the pattern of similarity revealed by BLAST and DOTPLOT between PNO and a hypothetical protein from the *Saccharomyces cerevisiae* genome annotated as a putative sulfite reductase and to a homologue of this hypothetical protein in the *Schizosaccharomyces pombe* genome. These proteins constitute a translational fusion of PFO domains I, II (partial) and VI with the FMN domain of CPR, as in EgPNOmt and CpPNO, that in turn is fused to a hemoprotein domain. The PFO domains of PuSR share ∼30% identity in conserved regions to eubacterial PFO. The FMN domain shares ∼30% identity to FMN domains from eubacterial and eukaryotic CPR (yet only ∼20-25% identity to the FMN domain of EgPNOmt and CpPNO), whereas the C-terminal hemoprotein domain shares ∼40% identity to the hemoprotein components of eubacterial sulfite reductase and ∼25% identity to nitrite reductases. Domain III and a portion of domain II of PFO are located on a separate protein, MET10, in both the yeast and *S. pombe* genomes (Fig. 3f).

The CPR domain of EgPNOmt and CpPNO also shares similarity to a number of other proteins and protein components. Among these are the ?-subunit of NADPH sulfite reductase (CysJ, Fig. 3h) from *Salmonella* (Ostrowski et al., 1989), which requires the hemoprotein ?-component CysI (similar to the C-terminaldomain of yeast PuSR in Fig. 3e) for activity. Further similarity is found in NADPH:cytochrome P450reductases (CPR) (Fig. 3h), enzymes involved in the oxidative metabolism of numerous compounds (Wang et al., 1997), e.g. fatty acid oxidation. The cognate substrate of CPR is typically cytochrome P₄₅₀ (Wang et al., 1997), which is found fused to the CPR domain both in the fatty acid hydroxylase P450BM-3 (Fig. 3i) from *Bacillus megaterium* (Govindaraj and Poulos, 1997) and in an identically organized protein in the genome of the fungus *Fusarium oxysporum.* The CPR domain also occurs in the C-terminus of metazoan nitric oxide synthases (Fig. 3j). Finally, constituent components of the CPR domain are found as individual proteins, including ferredoxin:NADP reductase of cyanobacteria and chloroplasts, which transfers electrons from the photosynthetic membrane to NADP⁺, yielding NADPH (Fig. 3k), and the soluble protein flavodoxin itself (Fig. 31).

### Example 4:

### Fatty acid synthesis in Euglena gracilis.

Under anaerobic conditions, acetyl-CoA from the PNO reaction serves as the end acceptor of electrons stemming from oxidative glucose breakdown (Inui et al. 1984b) in that it is used for malonyl-CoA dependent fatty acid synthesis, regenerating NAD(P): fatty acids are synthesized by reversal of ?-oxidation with the exception that the last step is catalyzed by trans-2-enoyl-CoA reductase (EC 1.3.1.-) instead of acyl-CoA dehydrogenase (EC 1.3.99.3, Inui et al. 1984a). This mitochondrial-localized system has the ability to synthesize fatty acids directly from acetyl-CoA which serves both as primer and C2-donor using NADH as electron donor and does not require any ATP (Inui et al. 1984a). The main products of this mitochondrial fatty acid synthetic system are fatty acids and alcohols ranging from C10 to C17, the main ones being myristic acid and myristyl alcohol (Inui et al. 1982, 1984a). The fatty acids appear to be transferred to the cytosol by the action of acyl carnitine transferase, where they are partly reduced to fatty alcohols and finally esterified to wax esters in microsomes (Inui et al. 1983). The composition of wax esters in anaerobically grown cells is also important: mainly saturated C28 esters with considerable amounts of saturated C26 and C27 esters but none of unsaturated ones are formed (Inui et al. 1983).

### Example 5:

### Functional analysis of EgPNOmt by overexpression in anaerobically growing E. coli cells.

The overexpression of the cloned cDNA will give further proof for the functional identity of the pEgPNO3 cDNA with the Pyruvate:NADP⁺ oxidoreductase. As the only known PFO so far, the *por* gene encoding pyruvate:ferredoxin oxidoreductase in *Desulvovibrio africanus* has been expressed in anaerobically grown *E. coli* cells behind the isopropyl-?-D-thiogalactopyranoside -inducible *tac*-promotor, resulting in the production of POR in its active form (Pieulle et al. 1997). The properties of the recombinant protein indicated that the recombinant PFO behaved like the native *D. africanus* enzyme (Pieulle et al. 1997). The enzyme, so obtained was active and crystallized, the tertiary structure of the enzyme is known (Pieulle et al. 1999, Charon et al. 1999). In analogy, overexpression of the *Euglena* pEgPNO3 will be performed in anaerobically grown *E. coli* cells. The recombinant protein will be further isolated and used for assay of PNO activity.

The activities of pyruvate:NADP⁺ oxidoreductase with NADP⁺ as electron acceptor can be determined photometrically by assay of the absorbance change at 340 nm due to the formation of NADPH. The reaction mixture for PNO contains 5 mM pyruvate, 0.2 mM CoA, 1 mM NADP⁺, 100 mM potassium phosphate buffer, pH 6.8, and the enzyme solution in a total volume of 2 ml (Inui et al. 1984b). The enzymatic reaction is initiated by the addition of enzyme and conducted at 30°C under anaerobic conditions. Anaerobiosis can be achieved by bubbling argon into the reaction mixture for 1 min in a rubber-capped quartz cuvette or test tube without the enzyme. The enzyme solution is freed of oxygen and added anaerobically by using a microsyringe.

Alternatively pyruvate:NADP⁺ oxidoreductase can be measured by the hydroxylamine method (Reed et al. 1966) with some modifications according to Inui et al. 1984b. The assay mixture contains 5 mM pyruvate, 0.2 mM CoA, 5 mM NADP⁺, 10 U of phosphotransacetylase, 100 mM potassium phosphate buffer, pH 6.8, and the enzyme solution in a total volume of 0.5 ml. Initiation and conduction is performed as described above.

### Literatur:

Altschul SF, (1997) Gapped BLAST and PSI-BLAST: a new generation of protein database search programs. *Nucleic Acids Res.* 25:3389-3402
Britz ML, (1979) Antimicrob Agents Chemother 16: 19-27
Buetow DE (1989) The mitochondrion. In: The Biology of Euglena, Vol. IV (Buetow D.E., ed.) pp. 247-314, Academic Press, San Diego.
Cerkasovova A, (1984) Mol Biochem Parasitol 11: 105-118
Chabriere, E., 1999. Nature Struct. Biol. 6: 182-90.
Charon M-H, (1999) Curr Opin Struct Biol 9: 663-669
Edwards DI (1986) Biochem Pharmacol 35: 53-58
Edwards DI (1993) J Antimicrob Chemother 31: 9-20
Freeman CD, (1997) Drugs 54: 679-708)
Genetics Computer Group. 1994. Program manual for Version 8, 575 Science Drive, Madison, Wisconsin, 53711,USA.
Govindaraj S, (1997) J Biol Chem 272:7915-7921
Hannaert V, Mol. Biol. Evol. in press.(2000)
Henze K, (1995) Proc Natl Acad Sci USA 92: 9122-9126.
Ings RM, (1974) Biochem Pharmacol 23: 1421-1429
Inui H, (1982) FEBS Lett 150: 89-93
Inui H, (1983) Agric Biol Chem 47: 2669-2671
Inui H, (1984a) Eur J Biochem 142: 121-126
Inui H, (1984b) J.Biochem. 96: 931-934
Inui H, (1987) J Biol Chem 262: 9130-9135
Inui H, (1990) Arch Biochem Biophys 280: 292-298
Inui H, (1991) Arch Biochem Biophys 286: 270-276
Johnson PJ (1993) Metronidazole and drug resistence. Parasitol Today 9: 183-186
Kitaoka S, (1989) Enzymes and their functional location. In Buetow DE (ed) The Biology of *Euglena,* Vol 6, Subcellular Biochemistry and Molecular Biology, pp 2-135. Academic Press, San Diego.
Kulda J (1999) Int J Parasitol 29: 199-212
Lloyd D, (1986) Biochem Pharmacol 35: 61-64
Marczak T, (1983) J Biol Chem 258: 12427-12433
Ostrowski J, (1989) J Biol Chem 264: 15796-15808.
Pieulle L, (1997) J Bacteriol 179: 5684-5692.
Quon DVK, (1992) Proc Natl Acad Sci 89: 4402-4406
Reed LJ, (1966) in Methods in Enzymology (Colowick, SP and Kaplan NO, eds.) Vol. IX, pp. 247-265, Academic Press, Inc., New York. Sambrook J, Molecular Cloning: A Laboratory Manual. Cold Spring Harbor, New York(1989).
Sindar P, (1982) J Med Microbiol 15: 503-509
Wang M, 1997. Proc Natl Acad Sci 4: 8411-8416.
Yarlett N, (1985) Mol Biochem Parasitol 14: 29-40
Yarlett N, (1986) Biochem Pharmacol 35: 1703-1708

## Claims

1. A polynucleotide comprising a nucleic acid molecule selected from the group consisting of:
(a) nucleic acid molecules encoding at least the mature form of the polypeptide depicted in SEQ ID NO: 1 (Figure 5);
(b) nucleic acid molecules comprising the coding sequence as depicted in SEQ ID NO: 2 (Figure 5) encoding at least the mature form of the polypeptide;
(c) nucleic acid molecules the nucleotide sequence of which is degenerate as a result of the genetic code to a nucleotide sequence of (a) or (b);
(d) nucleic acid molecules encoding a polypeptide derived from the polypeptide encoded by a polynucleotide of (a) to (c) by way of substitution, deletion and/or addition of one or several amino acids of the amino acid sequence of the polypeptide encoded by a polynucleotide of (a) to (c);
(e) nucleic acid molecules encoding a polypeptide the sequence of which has an identity of 60% or more to the amino acid sequence of the polypeptide encoded by a nucleic acid molecule of (a) or (b);
(f) nucleic acid molecules comprising a fragment or a epitope-bearing portion of a polypeptide encoded by a nucleic acid molecule of any one of (a) to (e) and having acetyl-CoA synthesis regulating activity;
(g) nucleic acid molecules comprising a polynucleotide having a sequence of a nucleic acid molecule amplified from an Euglena nucleic acid library using the primers depicted in SEQ ID NO: 3 and 4 (Figure 5); (h) nucleic acid molecules encoding a pyruvate dehydrogenase active fragment, a pyruvate:ferredoxin oxidoreductase active fragment, and/or a NADPH-cytochrome P450 reductase active fragment of a polypeptide encoded by any one of (a) to (g) ;
(i) nucleic acid molecules comprising at least 15 nucleotides of a polynucleotide of any one of (a) or (d);
(j) nucleic acid molecules encoding a polypeptide having pyruvate:NADP+ oxidoreductase (PNO) activity being recognized by antibodies that have been raised against a polypeptide encoded by a nucleic acid molecule of any one of (a) to (h) ;
(k) nucleic acid molecules obtainable by screening an appropriate library under stringent conditions with a probe having the sequence of the nucleic acid molecule of any one of (a) to (j) and having a PNO activity;
(l) nucleic acid molecules the complementary strand of which hybridizes under stringent conditions with a nucleic acid molecule of any one of (a) or (k) and having PNO activity;
or the complementary strand of any one of (a) to (l);
wherein the polynucleotide is not a polynucleotide encoding a polypeptide having the sequence TSGPKPASXI (SEQ ID No.: 5), TSGPKPASXIEVSXAK (SEQ ID No.: 6) or AAAPSGNXVTILYGSEEGNS (SEQ ID No.: 7).

2. The polynucleotide of claim 1 which is DNA or RNA.

3. The polynucleotide of claim 1 or 2, wherein the polynucleotide is operatively linked to a nucleic acid sequence encoding a signal sequence.

4. The polynucleotide of claim 3, wherein the signal sequence is a plastidal transit signal sequence.

5. A method for making a recombinant vector comprising inserting a polynucleotide of any one of claims 1 to 4 into a vector.

6. A recombinant vector containing the polynucleotide of any one of claims 1 to 4 or produced by the method of claim 5.

7. The vector of claim 6 in which the polynucleotide is operatively linked to expression control sequences allowing expression in prokaryotic or eukaryotic host cells.

8. A method of making a recombinant host cell comprising introducing the vector of claim 6 or 7 into a host cell.

9. A host cell genetically engineered with the polynucleotide of any one of claims 1 to 4 or the vector of claim 6 or 7.

10. The host cell of claim 9, which is E. coli, baculovirus, Agrobacterium, or a plant cell.

11. A process for the production of a polypeptide having PNO activity comprising culturing the host cell of claim 9 or 10 and recovering the polypeptide encoded by said polynucleotide and expressed by the host cell from the culture or the host cells.

12. A polypeptide having the amino acid sequence encoded by a polynucleotide of any one of claims 1 to 4 or obtainable by the process of claim 11.

13. An antibody that binds specifically to the polypeptide of claim 12.

14. An antisense nucleic acid molecule comprising the complementary sequence of any one of (a) to (1).

15. A method for the production of transgenic plants, plant cells or plant tissue comprising the introduction of the polynucleotide of any one of claims 1 to 4, or the vector of claim 6 or 7 into the genome of said plant, plant tissue or plant cell.

16. A plant cell comprising the polynucleotide of any one of claims 1 to 4, the vector of claim 6 or 7 or obtainable by the method of claim 15.

17. A transgenic plant or plant tissue comprising the plant cell of claim 16.

18. The transgenic plant or plant tissue of claim 17, which upon the presence of the polynucleotide or the vector has an altered acetyl-CoA synthesis.

19. A method for modulating the acetyl CoA synthesis in a host cell comprising providing the host cell of claim 9 or the steps of the method of claim 8 and further culturing the cell under conditions which permit the expression of the polypeptide of claim 12.

20. The method of claim 19 wherein the expressed polypeptide is localized in the cell's plastid.

21. A method for modulating the acetyl-CoA synthesis in a plant, plant tissue, or plant cell comprising providing the plant, plant tissue or plant cell of claim 16 or 17 or comprising the steps of the method of claim 15 and further culturing the plant, plant tissue or plant cell under condition which permits the expression of the polypeptide of claim 12.

22. The transgenic plant of claim 17 or 18, the host cell of claim 9 or the method of any one of claims 19 to 21, wherein the acetyl-CoA-synthesis is increased.

23. The transgenic plant of claim 16, 17 or 22, the host cell of claim 9 or 22 or the method of any one of claims 19 to 22, wherein the synthesis of the fatty acids, carotenoids, isoprenoids, vitamins, lipids, wax esters, (poly)saccharides) and/or polyhydroxyalkanoates is increased.

24. Harvestable parts of the transgenic plant of claim 17, 18, 22 or 23 comprising the plant cell of claim 16.

25. Propagation material of the transgenic plant of claim 17, 18, 22 or 23 comprising the cell of claim 16.

26. A method for the production of fatty acids, carotenoids, (poly)saccharides), vitamins, isoprenoids, lipids, wax esters, and/or polyhydroxyalkanoates comprising the steps of the method of any one of claims 19 to 22 and further isolating said compound from the cell, culture, plant or tissue.

27. Use of the polynucleotide of any one of claims 1 to 4, the vector of claim 6 or 7, or the polypeptide of claim 12 for making fatty acid, carotenoids, hexose, vitamin, isoprenoid, lipid, wax ester, and/or polyhydroxyalkanoate producing cells, tissues and/or plants.

28. A method for the identification of an agonist or antagonist of PNO activity comprising
(a) contacting cells which express the polypeptide of claim 12 with a candidate compound;
(b) assaying the PNO activity;
(c) comparing the PNO activity to a standard response made in the absence of the candidate compound; whereby, an increased PNO activity over the standard indicates that the compound is an agonist and a decreased PNO activity indicates that the compound is an antagonist.

29. A compound identified by the method of claim 28.

30. A compound identified by the method of claim 28 which is an antagonist or an agonist of the polypeptide of claim 12.

31. An antibody specifically recognizing the compound of claim 29 or 30.

32. Use of the polynucleotide of any one of claims 1 to 4, the vector of claim 6 or 7, the polypeptide of claim 12, the antibody of claim 13 or 31, or the compound of claim 29 or 30 for identifying and/or producing compounds activating or inhibiting PNO activity.

33. A pharmaceutical composition comprising the antisense nucleic acid molecule of claims 14, the antibody of claim 13 or 31 or the compound of claim 30, which is an antagonist and optionally a pharmaceutically acceptable carrier.

34. Use of the antisense nucleic acid molecule of claim 14, the antibody of claim 13 or 31, or the compound of claim 30, which is an antagonist for the preparation of a pharmaceutical composition for the treatment of parasite infections.

35. A kit comprising the polynucleotide of any one of claims 1 to 4, the vector of claim 6 or 7, the host cell of claim 9, 22 or 23, the polypeptide of claim 12, the antisense nucleic acid of claim 14, the antibody of claim 13 or 31, plant cell of claim 16, the plant or plant tissue of claim 17, 18, 22 or 23, the harvestable part of claim 24, the propagation material of claim 25 or the compound of claim 29 or 30.

36. A method for the production of a pharmaceutical composition comprising the steps of the method of claim 28; and
(a) formulating the compound identified in step (c) in a pharmaceutically acceptable form.


### Annex to the application documents - subsequently filed sequences listing

SEQUENZPROTOKOLL

<110> BASF Aktiengesellschaft

<120> Pyruvate:NADP+ oxidoreductase and uses thereof

<130> 0050/51654

<140> 00117730
<141> 2000-08-17

<160> 10

<170> PatentIn Ver. 2.1

<210> 1
<211> 1805
<212> PRT
<213> Euglena gracilis

<400> 1
Tyr Asn Met Lys Gln Ser Val Arg Pro Ile Ile Ser Asn Val Leu Arg
  1               5                  10                  15

Lys Glu Val Ala Leu Tyr Ser Thr Ile Ile Gly Gln Asp Lys Gly Lys
             20                  25                  30

Glu Pro Thr Gly Arg Thr Tyr Thr Ser Gly Pro Lys Pro Ala Ser His
         35                  40                  45

Ile Glu Val Pro His His Val Thr Val Pro Ala Thr Asp Arg Thr Pro
     50                  55                  60

Asn Pro Asp Ala Gln Phe Phe Gln Ser Val Asp Gly Ser Gln Ala Thr
 65                  70                  75                  80

Ser His Val Ala Tyr Ala Leu Ser Asp Thr Ala Phe Ile Tyr Pro Ile
                 85                  90                  95

Thr Pro Ser Ser Val Met Gly Glu Leu Ala Asp Val Trp Met Ala Gln
            100                 105                 110

Gly Arg Lys Asn Ala Phe Gly Gln Val Val Asp Val Arg Glu Met Gln
        115                 120                 125

Ser Glu Ala Gly Ala Ala Gly Ala Leu His Gly Ala Leu Ala Ala Gly
    130                 135                 140

Ala Ile Ala Thr Thr Phe Thr Ala Ser Gln Gly Leu Leu Leu Met Ile
145                 150                 155                 160

Pro Asn Met Tyr Lys Ile Ala Gly Glu Leu Met Pro Ser Val Ile His
                165                 170                 175

Val Ala Ala Arg Glu Leu Ala Gly His Ala Leu Ser Ile Phe Gly Gly
            180                 185                 190

His Ala Asp Val Met Ala Val Arg Gln Thr Gly Trp Ala Met Leu Cys
        195                 200                 205

Ser His Thr Val Gln Gln Ser His Asp Met Ala Leu Ile Ser His Val
    210                 215                 220

Ala Thr Leu Lys Ser Ser Ile Pro Phe Val His Phe Phe Asp Gly Phe
225                 230                 235                 240

Arg Thr Ser His Glu Val Asn Lys Ile Lys Met Leu Pro Tyr Ala Glu
                245                 250                 255

Leu Lys Lys Leu Val Pro Pro Gly Thr Met Glu Gln His Trp Ala Arg
            260                 265                 270

Ser Leu Asn Pro Met His Pro Thr Ile Arg Gly Thr Asn Gln Ser Ala
        275                 280                 285

Asp Ile Tyr Phe Gln Asn Met Glu Ser Ala Asn Gln Tyr Tyr Thr Asp
    290                 295                 300

Leu Ala Glu Val Val Gln Glu Thr Met Asp Glu Val Ala Pro Tyr Ile
305                 310                 315                 320

Gly Arg His Tyr Lys Ile Phe Glu Tyr Val Gly Ala Pro Asp Ala Glu
                325                 330                 335

Glu Val Thr Val Leu Met Gly Ser Gly Ala Thr Thr Val Asn Glu Ala
            340                 345                 350

Val Asp Leu Leu Val Lys Arg Gly Lys Lys Val Gly Ala Val Leu Val
        355                 360                 365

His Leu Tyr Arg Pro Trp Ser Thr Lys Ala Phe Glu Lys Val Leu Pro
    370                 375                 380

Lys Thr Val Lys Arg Ile Ala Ala Leu Asp Arg Cys Lys Glu Val Thr
385                 390                 395                 400

Ala Leu Gly Glu Pro Leu Tyr Leu Asp Val Ser Ala Thr Leu Asn Leu
                405                 410                 415

Phe Pro Glu Arg Gln Asn Val Lys Val Ile Gly Gly Arg Tyr Gly Leu
            420                 425                 430

Gly Ser Lys Asp Phe Ile Pro Glu His Ala Leu Ala Ile Tyr Ala Asn
        435                 440                 445

Leu Ala Ser Glu Asn Pro Ile Gln Arg Phe Thr Val Gly Ile Thr Asp
    450                 455                 460

Asp Val Thr Gly Thr Ser Val Pro Phe Val Asn Glu Arg Val Asp Thr
465                 470                 475                 480

Leu Pro Glu Gly Thr Arg Gln Cys Val Phe Trp Gly Ile Gly Ser Asp
                485                 490                 495

Gly Thr Val Gly Ala Asn Arg Ser Ala Val Arg Ile Ile Gly Asp Asn
            500                 505                 510

Ser Asp Leu Met Val Gln Ala Tyr Phe Gln Phe Asp Ala Phe Lys Ser
        515                 520                 525

Gly Gly Val Thr Ser Ser His Leu Arg Phe Gly Pro Lys Pro Ile Thr
    530                 535                 540

Ala Gln Tyr Leu Val Thr Asn Ala Asp Tyr Ile Ala Cys His Phe Gln
545                 550                 555                 560

Glu Tyr Val Lys Arg Phe Asp Met Leu Asp Ala Ile Arg Glu Gly Gly
                565                 570                 575

Thr Phe Val Leu Asn Ser Arg Trp Thr Thr Glu Asp Met Glu Lys Glu
            580                 585                 590

Ile Pro Ala Asp Phe Arg Arg Asn Val Ala Gln Lys Lys Val Arg Phe
        595                 600                 605

Tyr Asn Val Asp Ala Arg Lys Ile Cys Asp Ser Phe Gly Leu Gly Lys
    610                 615                 620

Arg Ile Asn Met Leu Met Gln Ala Cys Phe Phe Lys Leu Ser Gly Val
625                 630                 635                 640

Leu Pro Leu Ala Glu Ala Gln Arg Leu Leu Asn Glu Ser Ile Val His
                645                 650                 655

Glu Tyr Gly Lys Lys Gly Gly Lys Val Val Glu Met Asn Gln Ala Val
            660                 665                 670

Val Asn Ala Val Phe Ala Gly Asp Leu Pro Gln Glu Val Gln Val Pro
        675                 680                 685

Ala Ala Trp Ala Asn Ala Val Asp Thr Ser Thr Arg Thr Pro Thr Gly
    690                 695                 700

Ile Glu Phe Val Asp Lys Ile Met Arg Pro Leu Met Asp Phe Lys Gly
705                 710                 715                 720

Asp Gln Leu Pro Val Ser Val Met Thr Pro Gly Gly Thr Phe Pro Val
                725                 730                 735

Gly Thr Thr Gln Tyr Ala Lys Arg Ala Ile Ala Ala Phe Ile Pro Gln
            740                 745                 750

Trp Ile Pro Ala Asn Cys Thr Gln Cys Asn Tyr Cys Ser Tyr Val Cys
        755                 760                 765

Pro His Ala Thr Ile Arg Pro Phe Val Leu Thr Asp Gln Glu Val Gln
    770                 775                 780

Leu Ala Pro Glu Ser Phe Val Thr Arg Lys Ala Lys Gly Asp Tyr Gln
785                 790                 795                 800

Gly Met Asn Phe Arg Ile Gln Val Ala Pro Glu Asp Cys Thr Gly Cys
                805                 810                 815

Gln Val Cys Val Glu Thr Cys Pro Asp Asp Ala Leu Glu Met Thr Asp
            820                 825                 830

Ala Phe Thr Ala Thr Pro Val Gln Arg Thr Asn Trp Glu Phe Ala Ile
        835                 840                 845

Lys Val Pro Asn Arg Gly Thr Met Thr Asp Arg Tyr Ser Leu Lys Gly
    850                 855                 860

Ser Gln Phe Gln Gln Pro Leu Leu Glu Phe Ser Gly Ala Cys Glu Gly
865                 870                 875                 880

Cys Gly Glu Thr Pro Tyr Val Lys Leu Leu Thr Gln Leu Phe Gly Glu
                885                 890                 895

Arg Thr Val Ile Ala Asn Ala Thr Gly Cys Ser Ser Ile Trp Gly Gly
            900                 905                 910

Thr Ala Gly Leu Ala Pro Tyr Thr Thr Asn Ala Lys Gly Gln Gly Pro
        915                 920                 925

Ala Trp Gly Asn Ser Leu Phe Glu Asp Asn Ala Glu Phe Gly Phe Gly
    930                 935                 940

Ile Ala Val Ala Asn Ala Gln Lys Arg Ser Arg Val Arg Asp Cys Ile
945                 950                 955                 960

Leu Gln Ala Val Glu Lys Lys Val Ala Asp Glu Gly Leu Thr Thr Leu
                965                 970                 975

Leu Ala Gln Trp Leu Gln Asp Trp Asn Thr Gly Asp Lys Thr Leu Lys
            980                 985                 990

Tyr Gln Asp Gln Ile Ile Ala Gly Leu Ala Gln Gln Arg Ser Lys Asp
        995                 1000                1005

Pro Leu Leu Glu Gln Ile Tyr Gly Met Lys Asp Met Leu Pro Asn Ile
   1010                1015                1020

Ser Gln Trp Ile Ile Gly Gly Asp Gly Trp Ala Asn Asp Ile Gly Phe
1025                1030                1035                1040

Gly Gly Leu Asp His Val Leu Ala Ser Gly Gln Asn Leu Asn Val Leu
                1045                1050                1055

Val Leu Asp Thr Glu Met Tyr Ser Asn Thr Gly Gly Gln Ala Ser Lys
            1060                1065                1070

Ser Thr His Met Ala Ser Val Ala Lys Phe Ala Leu Gly Gly Lys Arg
        1075                1080                1085

Thr Asn Lys Lys Asn Leu Thr Glu Met Ala Met Ser Tyr Gly Asn Val
   1090                1095                1100

Tyr Val Ala Thr Val Ser His Gly Asn Met Ala Gln Cys Val Lys Ala
1105                1110                1115                1120

Phe Val Glu Ala Glu Ser Tyr Asp Gly Pro Ser Leu Ile Val Gly Tyr
                1125                1130                1135

Ala Pro Cys Ile Glu His Gly Leu Arg Ala Gly Met Ala Arg Met Val
            1140                1145                1150

Gln Glu Ser Glu Ala Ala Ile Ala Thr Gly Tyr Trp Pro Leu Tyr Arg
        1155                1160                1165

Phe Asp Pro Arg Leu Ala Thr Glu Gly Lys Asn Pro Phe Gln Leu Asp
    1170                1175                1180

Ser Lys Arg Ile Lys Gly Asn Leu Gln Glu Tyr Leu Asp Arg Gln Asn
1185                1190                1195                1200

Arg Tyr Val Asn Leu Lys Lys Asn Asn Pro Lys Gly Ala Asp Leu Leu
                1205                1210                1215

Lys Ser Gln Met Ala Asp Asn Ile Thr Ala Arg Phe Asn Arg Tyr Arg
            1220                1225                1230

Arg Met Leu Glu Gly Pro Asn Thr Lys Ala Ala Ala Pro Ser Gly Asn
        1235                1240                1245

His Val Thr Ile Leu Tyr Gly Ser Glu Thr Gly Asn Ser Glu Gly Leu
    1250                1255                1260

Ala Lys Glu Leu Ala Thr Asp Phe Glu Arg Arg Glu Tyr Ser Val Ala
1265                1270                1275                1280

Val Gln Ala Leu Asp Asp Ile Asp Val Ala Asp Leu Glu Asn Met Gly
                1285                1290                1295

Phe Val Val Ile Ala Val Ser Thr Cys Gly Gln Gly Gln Phe Pro Arg
            1300                1305                1310

Asn Ser Gln Leu Phe Trp Arg Glu Leu Gln Arg Asp Lys Pro Glu Gly
        1315                1320                1325

Trp Leu Lys Asn Leu Lys Tyr Thr Val Phe Gly Leu Gly Asp Ser Thr
    1330                1335                1340

Tyr Tyr Phe Tyr Cys His Thr Ala Lys Gln Ile Asp Ala Arg Leu Ala
1345                1350                1355                1360

Ala Leu Gly Ala Gln Arg Val Val Pro Ile Gly Phe Gly Asp Asp Gly
                1365                1370                1375

Asp Glu Asp Met Phe His Thr Gly Phe Asn Asn Trp Ile Pro Ser Val
            1380                1385                1390

Trp Asn Glu Leu Lys Thr Lys Thr Pro Glu Glu Ala Leu Phe Thr Pro
        1395                1400                1405

Ser Ile Ala Val Gln Leu Thr Pro Asn Ala Thr Pro Gln Asp Phe His
    1410                1415                1420

Phe Ala Lys Ser Thr Pro Val Leu Ser Ile Thr Gly Ala Glu Arg Ile
1425                1430                1435                1440

Thr Pro Ala Asp His Thr Arg Asn Phe Val Thr Ile Arg Trp Lys Thr
                1445                1450                1455

Asp Leu Ser Tyr Gln Val Gly Asp Ser Leu Gly Val Phe Pro Glu Asn
            1460                1465                1470

Thr Arg Ser Val Val Glu Glu Phe Leu Gln Tyr Tyr Gly Leu Asn Pro
        1475                1480                1485

Lys Asp Val Ile Thr Ile Glu Asn Lys Gly Ser Arg Glu Leu Pro His
    1490                1495                1500

Cys Met Ala Val Gly Asp Leu Phe Thr Lys Val Leu Asp Ile Leu Gly
1505                1510                1515                1520

Lys Pro Asn Asn Arg Phe Tyr Lys Thr Leu Ser Tyr Phe Ala Val Asp
                1525                1530                1535

Lys Ala Glu Lys Glu Arg Leu Leu Lys Ile Ala Glu Met Gly Pro Glu
            1540                1545                1550

Tyr Ser Asn Ile Leu Ser Glu Met Tyr His Tyr Ala Asp Ile Phe His
        1555                1560                1565

Met Phe Pro Ser Ala Arg Pro Thr Leu Gln Tyr Leu Ile Glu Met Ile
    1570                1575                1580

Pro Asn Ile Lys Pro Arg Tyr Tyr Ser Ile Ser Ser Ala Pro Ile His
1585                1590                1595                1600

Thr Pro Gly Glu Val His Ser Leu Val Leu Ile Asp Thr Trp Ile Thr
                1605                1610                1615

Leu Ser Gly Lys His Arg Thr Gly Leu Thr Cys Thr Met Leu Glu His
            1620                1625                1630

Leu Gln Ala Gly Gln Val Val Asp Gly Cys Ile His Pro Thr Ala Met
        1635                1640                1645

Glu Phe Pro Asp His Glu Lys Pro Val Val Met Cys Ala Met Gly Ser
    1650                1655                1660

Gly Leu Ala Pro Phe Val Ala Phe Leu Arg Glu Arg Ser Thr Leu Arg
1665                1670                1675                1680

Lys Gln Gly Lys Lys Thr Gly Asn Met Ala Leu Tyr Phe Gly Asn Arg
            1685                1690                1695

Tyr Glu Lys Thr Glu Phe Leu Met Lys Glu Glu Leu Lys Gly His Ile
        1700                1705                1710

Asn Asp Gly Leu Leu Thr Leu Arg Cys Ala Phe Ser Arg Asp Asp Pro
    1715                1720                1725

Lys Lys Lys Val Tyr Val Gln Asp Leu Ile Lys Met Asp Glu Lys Met
1730                1735                1740

Met Tyr Asp Tyr Leu Val Val Gln Lys Gly Ser Met Tyr Cys Cys Gly
1745                1750                1755                1760

Ser Arg Ser Phe Ile Lys Pro Val Gln Glu Ser Leu Lys His Cys Phe
            1765                1770                1775

Met Lys Ala Gly Gly Leu Thr Ala Glu Gln Ala Glu Asn Glu Val Ile
        1780                1785                1790

Asp Met Phe Thr Thr Gly Arg Tyr Asn Ile Glu Ala Trp
    1795                1800                1805

<210> 2
<211> 5812
<212> DNA
<213> Euglena gracilis

<220>
<221> CDS
<222> (7)..(5418)
<223> Begin of the CPR-domain 3724

<400> 2
tacaac atg aag cag tct gtc cgc cca att att tcc aat gta ctg cgc     48
       Met Lys Gln Ser Val Arg Pro Ile Ile Ser Asn Val Leu Arg
       1               5                   10

aag gag gtt gct ctg tac tca aca atc att gga caa gac aag ggg aag     96
Lys Glu Val Ala Leu Tyr Ser Thr Ile Ile Gly Gln Asp Lys Gly Lys
15                  20                  25                  30

gaa cca act ggt cga aca tac acc agt ggc cca aaa ccg gca tct cac     144
Glu Pro Thr Gly Arg Thr Tyr Thr Ser Gly Pro Lys Pro Ala Ser His
                35                  40                  45

att gaa gtt ccc cat cat gtg act gtg cct gcc act gac cgc acc ccg    192
Ile Glu Val Pro His His Val Thr Val Pro Ala Thr Asp Arg Thr Pro
                50                  55                  60

aat ccc gat gct caa ttc ttt cag tct gta gat ggg tca caa gcc acc    240
Asn Pro Asp Ala Gln Phe Phe Gln Ser Val Asp Gly Ser Gln Ala Thr
            65                  70                  75

agt cac gtt gcg tac gct ctg tct gac aca gcg ttc att tac cca att    288
Ser His Val Ala Tyr Ala Leu Ser Asp Thr Ala Phe Ile Tyr Pro Ile
    80                  85                  90

aca ccc agt tct gtg atg ggc gag ctg gct gat gtt tgg atg gct caa    336
Thr Pro Ser Ser Val Met Gly Glu Leu Ala Asp Val Trp Met Ala Gln
95                  100                 105                 110

ggg agg aag aac gcc ttt ggt cag gtt gtg gat gtc cgt gag atg caa    384
Gly Arg Lys Asn Ala Phe Gly Gln Val Val Asp Val Arg Glu Met Gln
                115                 120                 125

tct gag gct gga gcc gca ggc gcc ctg cat ggg gca ctg gct gct gga    432
Ser Glu Ala Gly Ala Ala Gly Ala Leu His Gly Ala Leu Ala Ala Gly
            130                 135                 140

gcc att gct aca acc ttc act gcc tct caa ggg ttg ttg ttg atg att    480
Ala Ile Ala Thr Thr Phe Thr Ala Ser Gln Gly Leu Leu Leu Met Ile
        145                 150                 155

ccc aac atg tat aag att gca ggt gag ctg atg ccc tct gtc atc cac    528
Pro Asn Met Tyr Lys Ile Ala Gly Glu Leu Met Pro Ser Val Ile His
    160                 165                 170

gtt gca gcc cga gag ctt gca ggc cac gct ctg tcc att ttt gga gga    576
Val Ala Ala Arg Glu Leu Ala Gly His Ala Leu Ser Ile Phe Gly Gly
175                 180                 185                 190

cac gct gat gtc atg gct gtc cgc caa aca gga tgg gct atg ctg tgc    624
His Ala Asp Val Met Ala Val Arg Gln Thr Gly Trp Ala Met Leu Cys
                195                 200                 205

tcc cac aca gtg cag cag tct cac gac atg gct ctc atc tcc cac gtg    672
Ser His Thr Val Gln Gln Ser His Asp Met Ala Leu Ile Ser His Val
            210                 215                 220

gcc acc ctc aag tcc agc atc ccc ttc gtt cac ttc ttt gat ggt ttc    720
Ala Thr Leu Lys Ser Ser Ile Pro Phe Val His Phe Phe Asp Gly Phe
        225                 230                 235

cgc aca agc cac gaa gtg aac aaa atc aaa atg ctg cct tat gca gaa    768
Arg Thr Ser His Glu Val Asn Lys Ile Lys Met Leu Pro Tyr Ala Glu
    240                 245                 250

ctg aag aaa ctg gtg cct cct ggc acc atg gaa cag cac tgg gct cgt    816
Leu Lys Lys Leu Val Pro Pro Gly Thr Met Glu Gln His Trp Ala Arg
255                 260                 265                 270

tcg ctg aac ccc atg cac ccc acc atc cga gga aca aac cag tct gca    864
Ser Leu Asn Pro Met His Pro Thr Ile Arg Gly Thr Asn Gln Ser Ala
                275                 280                 285

gac atc tac ttc cag aat atg gaa agt gca aac cag tac tac act gat    912
Asp Ile Tyr Phe Gln Asn Met Glu Ser Ala Asn Gln Tyr Tyr Thr Asp
            290                 295                 300

ctg gcc gag gtc gtt cag gag aca atg gac gaa gtt gca cca tac atc    960
Leu Ala Glu Val Val Gln Glu Thr Met Asp Glu Val Ala Pro Tyr Ile
        305                 310                 315

ggt cgc cac tac aag atc ttt gag tat gtt ggt gca cca gat gca gaa    1008
Gly Arg His Tyr Lys Ile Phe Glu Tyr Val Gly Ala Pro Asp Ala Glu
    320                 325                 330

gaa gtg aca gtg ctc atg ggt tct ggt gca acc aca gtc aac gag gca    1056
Glu Val Thr Val Leu Met Gly Ser Gly Ala Thr Thr Val Asn Glu Ala
335                 340                 345                 350

gtg gac ctt ctt gtg aag cgt gga aag aag gtt ggt gca gtc ttg gtg    1104
Val Asp Leu Leu Val Lys Arg Gly Lys Lys Val Gly Ala Val Leu Val
                355                 360                 365

cac ctc tac cga cca tgg tca aca aag gca ttt gaa aag gtc ctg ccc    1152
His Leu Tyr Arg Pro Trp Ser Thr Lys Ala Phe Glu Lys Val Leu Pro
            370                 375                 380

aag aca gtg aag cgc att gct gct ctg gat cgc tgc aag gag gtg act    1200
Lys Thr Val Lys Arg Ile Ala Ala Leu Asp Arg Cys Lys Glu Val Thr
        385                 390                 395

gca ctg ggt gag cct ctg tat ctg gat gtg tcg gca act ctg aat ttg    1248
Ala Leu Gly Glu Pro Leu Tyr Leu Asp Val Ser Ala Thr Leu Asn Leu
    400                 405                 410

ttc ccg gaa cgc cag aat gtg aaa gtc att gga gga cgt tac gga ttg    1296
Phe Pro Glu Arg Gln Asn Val Lys Val Ile Gly Gly Arg Tyr Gly Leu
415                 420                 425                 430

ggc tca aag gat ttc atc ccg gag cat gcc ctg gca att tac gcc aac     1344
Gly Ser Lys Asp Phe Ile Pro Glu His Ala Leu Ala Ile Tyr Ala Asn
                435                 440                 445

ttg gcc agc gag aac ccc att caa aga ttc act gtg ggt atc aca gat     1392
Leu Ala Ser Glu Asn Pro Ile Gln Arg Phe Thr Val Gly Ile Thr Asp
            450                 455                 460

gat gtc act ggc aca tcc gtt cct ttc gtc aac gag cgt gtt gac acg     1440
Asp Val Thr Gly Thr Ser Val Pro Phe Val Asn Glu Arg Val Asp Thr
        465                 470                 475

ttg ccc gag ggc acc cgc cag tgt gtc ttc tgg gga att ggt tca gat     1488
Leu Pro Glu Gly Thr Arg Gln Cys Val Phe Trp Gly Ile Gly Ser Asp
    480                 485                 490

gga aca gtg gga gcc aat cgc tct gcc gtg aga atc att gga gac aac     1536
Gly Thr Val Gly Ala Asn Arg Ser Ala Val Arg Ile Ile Gly Asp Asn
495                 500                 505                 510

agc gat ttg atg gtt cag gcc tac ttc caa ttt gat gct ttc aag tca     1584
Ser Asp Leu Met Val Gln Ala Tyr Phe Gln Phe Asp Ala Phe Lys Ser
                515                 520                 525

ggt ggt gtc act tcc tcg cat ctc cgt ttt gga cca aag ccc atc aca     1632
Gly Gly Val Thr Ser Ser His Leu Arg Phe Gly Pro Lys Pro Ile Thr
            530                 535                 540

gcg caa tac ctt gtt acc aat gct gac tac atc gcg tgc cac ttc cag     1680
Ala Gln Tyr Leu Val Thr Asn Ala Asp Tyr Ile Ala Cys His Phe Gln
        545                 550                 555

gag tat gtc aag cgc ttt gac atg ctt gat gcc atc cgt gag ggg ggc     1728
Glu Tyr Val Lys Arg Phe Asp Met Leu Asp Ala Ile Arg Glu Gly Gly
    560                 565                 570

acc ttt gtt ctc aat tct cgg tgg acc acg gag gac atg gag aag gag     1776
Thr Phe Val Leu Asn Ser Arg Trp Thr Thr Glu Asp Met Glu Lys Glu
575                 580                 585                 590

att ccg gct gac ttc cgg cgc aac gtg gca cag aag aag gtc cgc ttc     1824
Ile Pro Ala Asp Phe Arg Arg Asn Val Ala Gln Lys Lys Val Arg Phe
                595                 600                 605

tac aat gtg gat gct cga aag atc tgt gac agt ttt ggt ctt ggg aag     1872
Tyr Asn Val Asp Ala Arg Lys Ile Cys Asp Ser Phe Gly Leu Gly Lys
            610                 615                 620

cgc atc aat atg ctg atg cag gct tgt ttc ttc aag ctg tct ggg gtg 1920
Arg Ile Asn Met Leu Met Gln Ala Cys Phe Phe Lys Leu Ser Gly Val
        625                 630                 635

ctc cca ctg gcc gaa gct cag cgg ctg ctg aac gag tcc att gtg cat 1968
Leu Pro Leu Ala Glu Ala Gln Arg Leu Leu Asn Glu Ser Ile Val His
    640                 645                 650

gag tat gga aag aag ggt ggc aag gtg gtg gag atg aac caa gca gtg 2016
Glu Tyr Gly Lys Lys Gly Gly Lys Val Val Glu Met Asn Gln Ala Val
655                 660                 665                 670

gtg aat gct gtc ttt gct ggt gac ctg ccc cag gaa gtt caa gtc cct 2064
Val Asn Ala Val Phe Ala Gly Asp Leu Pro Gln Glu Val Gln Val Pro
                675                 680                 685

gcc gcc tgg gca aac gca gtt gat aca tcc acc cgt acc ccc acc ggg 2112
Ala Ala Trp Ala Asn Ala Val Asp Thr Ser Thr Arg Thr Pro Thr Gly
            690                 695                 700

att gag ttt gtt gac aag atc atg cgc ccg ctg atg gat ttc aag ggt 2160
Ile Glu Phe Val Asp Lys Ile Met Arg Pro Leu Met Asp Phe Lys Gly
        705                 710                 715

gac cag ctc cca gtc agt gtg atg act cct ggt gga acc ttc cct gtc 2208
Asp Gln Leu Pro Val Ser Val Met Thr Pro Gly Gly Thr Phe Pro Val
    720                 725                 730

ggg aca aca cag tat gcc aag cgt gca att gct gct ttc att ccc cag 2256
Gly Thr Thr Gln Tyr Ala Lys Arg Ala Ile Ala Ala Phe Ile Pro Gln
735                 740                 745                 750

tgg att cct gcc aac tgc aca cag tgc aac tat tgt tcg tat gtt tgc 2304
Trp Ile Pro Ala Asn Cys Thr Gln Cys Asn Tyr Cys Ser Tyr Val Cys
                755                 760                 765

ccc cac gcc acc atc cga cct ttc gtg ctg aca gac cag gag gtg cag 2352
Pro His Ala Thr Ile Arg Pro Phe Val Leu Thr Asp Gln Glu Val Gln
            770                 775                 780

ctg gcc ccg gag agc ttt gtg aca cgc aag gcg aag ggt gat tac cag 2400
Leu Ala Pro Glu Ser Phe Val Thr Arg Lys Ala Lys Gly Asp Tyr Gln
        785                 790                 795

ggg atg aat ttc cgc atc caa gtt gct cct gag gat tgc act ggc tgc 2448
Gly Met Asn Phe Arg Ile Gln Val Ala Pro Glu Asp Cys Thr Gly Cys
    800                 805                 810

cag gtg tgc gtg gag acg tgc ccc gat gat gcc ctg gag atg acc gac 2496
Gln Val Cys Val Glu Thr Cys Pro Asp Asp Ala Leu Glu Met Thr Asp
815                 820                 825                 830

gct ttc acc gcc acc cct gtg caa cgc acc aac tgg gag ttc gcc atc 2544
Ala Phe Thr Ala Thr Pro Val Gln Arg Thr Asn Trp Glu Phe Ala Ile
                835                 840                 845

aag gtg ccc aac cgc ggc acc atg acg gac cgc tac tcc ctg aag ggc 2592
Lys Val Pro Asn Arg Gly Thr Met Thr Asp Arg Tyr Ser Leu Lys Gly
            850                 855                 860

agc cag ttc cag cag ccc ctc ctg gag ttc tcc ggg gcc tgc gag ggc 2640
Ser Gln Phe Gln Gln Pro Leu Leu Glu Phe Ser Gly Ala Cys Glu Gly
        865                 870                 875

tgc ggc gag acc cca tat gtc aag ctg ctc acc cag ctc ttc ggc gag 2688
Cys Gly Glu Thr Pro Tyr Val Lys Leu Leu Thr Gln Leu Phe Gly Glu
    880                 885                 890

cgg acg gtc atc gcc aac gcc acc ggc tgc agt tcc atc tgg ggt ggc 2736
Arg Thr Val Ile Ala Asn Ala Thr Gly Cys Ser Ser Ile Trp Gly Gly
895                 900                 905                 910

act gcc ggc ctg gcg ccg tac acc acc aac gcc aag ggc cag ggc ccg 2784
Thr Ala Gly Leu Ala Pro Tyr Thr Thr Asn Ala Lys Gly Gln Gly Pro
                915                 920                 925

gcc tgg ggc aac agc ctg ttc gag gac aac gcc gag ttc ggc ttt ggc 2832
Ala Trp Gly Asn Ser Leu Phe Glu Asp Asn Ala Glu Phe Gly Phe Gly
            930                 935                 940

att gca gtg gcc aac gcc cag aag agg tcc cgc gtg agg gac tgc atc 2880
Ile Ala Val Ala Asn Ala Gln Lys Arg Ser Arg Val Arg Asp Cys Ile
        945                 950                 955

ctg cag gca gtg gag aag aag gtc gcc gat gag ggt ttg acc aca ttg 2928
Leu Gln Ala Val Glu Lys Lys Val Ala Asp Glu Gly Leu Thr Thr Leu
    960                 965                 970

ttg gcg caa tgg ctg cag gat tgg aac aca gga gac aag acc ttg aag 2976
Leu Ala Gln Trp Leu Gln Asp Trp Asn Thr Gly Asp Lys Thr Leu Lys
975                 980                 985                 990

tac caa gac cag atc att gca ggg ctg gca cag cag cgc agc aag gat 3024
Tyr Gln Asp Gln Ile Ile Ala Gly Leu Ala Gln Gln Arg Ser Lys Asp
                995                 1000                1005

ccc ctt ctg gag cag atc tat ggc atg aag gac atg ctg cct aac atc 3072
Pro Leu Leu Glu Gln Ile Tyr Gly Met Lys Asp Met Leu Pro Asn Ile
            1010                1015                1020

agc cag tgg atc att ggt ggt gat ggc tgg gcc aac gac att ggt ttc 3120
Ser Gln Trp Ile Ile Gly Gly Asp Gly Trp Ala Asn Asp Ile Gly Phe
        1025                1030                1035

ggt ggg ctg gac cac gtg ctg gcc tct ggg cag aac ctc aac gtc ctg 3168
Gly Gly Leu Asp His Val Leu Ala Ser Gly Gln Asn Leu Asn Val Leu
    1040                1045                1050

gtg ctg gac acc gag atg tac agc aac acc ggt ggg cag gcc tcc aag 3216
Val Leu Asp Thr Glu Met Tyr Ser Asn Thr Gly Gly Gln Ala Ser Lys
1055                1060                1065                1070

tcc acc cac atg gcc tct gtg gcc aag ttt gcc ctg gga ggg aag cgc 3264
Ser Thr His Met Ala Ser Val Ala Lys Phe Ala Leu Gly Gly Lys Arg
                1075                1080                1085

acc aac aag aag aac ttg acg gag atg gca atg agc tat ggc aac gtc 3312
Thr Asn Lys Lys Asn Leu Thr Glu Met Ala Met Ser Tyr Gly Asn Val
            1090                1095                1100

tat gtg gcc acc gtc tcc cat ggc aac atg gcc cag tgc gtc aag gcg 3360
Tyr Val Ala Thr Val Ser His Gly Asn Met Ala Gln Cys Val Lys Ala
        1105                1110                1115

ttt gtg gag gct gag tct tat gat gga cct tcg ctc att gtt ggc tat 3408
Phe Val Glu Ala Glu Ser Tyr Asp Gly Pro Ser Leu Ile Val Gly Tyr
    1120                1125                1130

gcg cca tgc atc gag cat ggt ctg cgt gct ggt atg gca agg atg gtt 3456
Ala Pro Cys Ile Glu His Gly Leu Arg Ala Gly Met Ala Arg Met Val
1135                1140                1145                1150

caa gag tct gag gct gcc atc gcc acg gga tac tgg ccc ctg tac cgc 3504
Gln Glu Ser Glu Ala Ala Ile Ala Thr Gly Tyr Trp Pro Leu Tyr Arg
                1155                1160                1165

ttt gac ccc cgc ctg gcg acc gag ggc aag aac ccc ttc cag ctg gac 3552
Phe Asp Pro Arg Leu Ala Thr Glu Gly Lys Asn Pro Phe Gln Leu Asp
            1170                1175                1180

tcc aag cgc atc aag ggc aac ctg cag gag tac ctg gac cgc cag aac 3600
Ser Lys Arg Ile Lys Gly Asn Leu Gln Glu Tyr Leu Asp Arg Gln Asn
        1185                1190                1195

cgg tat gtc aac ctg aag aag aac aac ccg aag ggt gcg gat ctg ctg    3648
Arg Tyr Val Asn Leu Lys Lys Asn Asn Pro Lys Gly Ala Asp Leu Leu
    1200                1205                1210

aag tct cag atg gcc gac aac atc acc gcc cgg ttc aac cgc tac cga    3696
Lys Ser Gln Met Ala Asp Asn Ile Thr Ala Arg Phe Asn Arg Tyr Arg
1215                1220                1225                1230

cgc atg ttg gag ggc ccc aat aca aaa gcc gcc gcc ccc agc ggc aac    3744
Arg Met Leu Glu Gly Pro Asn Thr Lys Ala Ala Ala Pro Ser Gly Asn
                1235                1240                1245

cat gtg acc atc ctg tac ggc tcc gaa act ggc aac agt gag ggt ctg    3792
His Val Thr Ile Leu Tyr Gly Ser Glu Thr Gly Asn Ser Glu Gly Leu
            1250                1255                1260

gca aag gag ctg gcc acc gac ttc gag cgc cgg gag tac tcc gtc gca    3840
Ala Lys Glu Leu Ala Thr Asp Phe Glu Arg Arg Glu Tyr Ser Val Ala
        1265                1270                1275

gtg cag gct ttg gat gac atc gac gtt gct gac ttg gag aac atg ggc    3888
Val Gln Ala Leu Asp Asp Ile Asp Val Ala Asp Leu Glu Asn Met Gly
    1280                1285                1290

ttc gtg gtc att gcg gtg tcc acc tgt ggg cag gga cag ttc ccc cgc    3936
Phe Val Val Ile Ala Val Ser Thr Cys Gly Gln Gly Gln Phe Pro Arg
1295                1300                1305                1310

aac agc cag ctg ttc tgg cgg gag ctg cag cgg gac aag cct gag ggc    3984
Asn Ser Gln Leu Phe Trp Arg Glu Leu Gln Arg Asp Lys Pro Glu Gly
                1315                1320                1325

tgg ctg aag aac ttg aag tac act gtc ttc ggg ctg ggc gac agc aca    4032
Trp Leu Lys Asn Leu Lys Tyr Thr Val Phe Gly Leu Gly Asp Ser Thr
            1330                1335                1340

tac tac ttc tac tgc cac acc gcc aag cag atc gac gct cgc ctg gcc    4080
Tyr Tyr Phe Tyr Cys His Thr Ala Lys Gln Ile Asp Ala Arg Leu Ala
        1345                1350                1355

gcc ttg ggc gct cag cgg gtg gtg ccc att ggc ttc ggc gac gat ggg    4128
Ala Leu Gly Ala Gln Arg Val Val Pro Ile Gly Phe Gly Asp Asp Gly
    1360                1365                1370

gat gag gac atg ttc cac acc ggc ttc aac aac tgg atc ccc agt gtg    4176
Asp Glu Asp Met Phe His Thr Gly Phe Asn Asn Trp Ile Pro Ser Val
1375                1380                1385                1390

tgg aat gag ctc aag acc aag act ccg gag gaa gcg ctg ttc acc ccg 4224
Trp Asn Glu Leu Lys Thr Lys Thr Pro Glu Glu Ala Leu Phe Thr Pro
                1395                1400                1405

agc atc gcc gtg cag ctc acc ccc aac gcc acc ccg cag gat ttc cat 4272
Ser Ile Ala Val Gln Leu Thr Pro Asn Ala Thr Pro Gln Asp Phe His
            1410                1415                1420

ttc gcc aag tcc acc cca gtg ctg tcc atc acc ggt gcc gaa cgc atc 4320
Phe Ala Lys Ser Thr Pro Val Leu Ser Ile Thr Gly Ala Glu Arg Ile
        1425                1430                1435

acg ccg gca gac cac acc cgc aac ttc gtc act atc cga tgg aag acc 4368
Thr Pro Ala Asp His Thr Arg Asn Phe Val Thr Ile Arg Trp Lys Thr
    1440                1445                1450

gat ttg tcg tac cag gtg ggt gac tct ctt ggt gtc ttc cct gag aac 4416
Asp Leu Ser Tyr Gln Val Gly Asp Ser Leu Gly Val Phe Pro Glu Asn
1455                1460                1465                1470

acc cgg tca gtg gtg gag gag ttc ctg cag tat tac ggc ttg aac ccc 4464
Thr Arg Ser Val Val Glu Glu Phe Leu Gln Tyr Tyr Gly Leu Asn Pro
                1475                1480                1485

aag gac gtc atc acc atc gaa aac aag ggc agc cgg gag ttg ccc cac 4512
Lys Asp Val Ile Thr Ile Glu Asn Lys Gly Ser Arg Glu Leu Pro His
            1490                1495                1500

tgc atg gct gtt ggg gat ctc ttc acg aag gtg ttg gac atc ttg ggc 4560
Cys Met Ala Val Gly Asp Leu Phe Thr Lys Val Leu Asp Ile Leu Gly
        1505                1510                1515

aaa ccc aac aac cgg ttc tac aag acc ctt tct tac ttt gca gtg gac 4608
Lys Pro Asn Asn Arg Phe Tyr Lys Thr Leu Ser Tyr Phe Ala Val Asp
    1520                1525                1530

aag gcc gag aag gag cgc ttg ttg aag atc gcc gag atg ggg ccg gag 4656
Lys Ala Glu Lys Glu Arg Leu Leu Lys Ile Ala Glu Met Gly Pro Glu
1535                1540                1545                1550

tac agc aac atc ctg tct gag atg tac cac tac gcg gac atc ttc cac 4704
Tyr Ser Asn Ile Leu Ser Glu Met Tyr His Tyr Ala Asp Ile Phe His
                1555                1560                1565

atg ttc ccg tcc gcc cgg ccc acg ctg cag tac ctc atc gag atg atc 4752
Met Phe Pro Ser Ala Arg Pro Thr Leu Gln Tyr Leu Ile Glu Met Ile
            1570                1575                1580

ccc aac atc aag ccc cgg tac tac tcc atc tcc tcc gcc ccc atc cac 4800
Pro Asn Ile Lys Pro Arg Tyr Tyr Ser Ile Ser Ser Ala Pro Ile His
        1585                1590                1595

acc cct ggc gag gtc cac agc ctg gtg ctc atc gac acc tgg atc acg 4848
Thr Pro Gly Glu Val His Ser Leu Val Leu Ile Asp Thr Trp Ile Thr
    1600                1605                1610

ctg tcc ggc aag cac cgc acg ggg ctg acc tgc acc atg ctg gag cac 4896
Leu Ser Gly Lys His Arg Thr Gly Leu Thr Cys Thr Met Leu Glu His
1615                1620                1625                1630

ctg cag gcg ggc cag gtg gtg gat ggc tgc atc cac ccc acg gcg atg 4944
Leu Gln Ala Gly Gln Val Val Asp Gly Cys Ile His Pro Thr Ala Met
                1635                1640                1645

gag ttc ccc gac cac gag aag ccg gtg gtg atg tgc gcc atg ggc agt 4992
Glu Phe Pro Asp His Glu Lys Pro Val Val Met Cys Ala Met Gly Ser
            1650                1655                1660

ggc ctg gca ccg ttc gtt gct ttc ctg cgc gag cgc tcc acg ctg cgg 5040
Gly Leu Ala Pro Phe Val Ala Phe Leu Arg Glu Arg Ser Thr Leu Arg
        1665                1670                1675

aag cag ggc aag aag acc ggg aac atg gca ttg tac ttc ggc aac agg 5088
Lys Gln Gly Lys Lys Thr Gly Asn Met Ala Leu Tyr Phe Gly Asn Arg
    1680                1685                1690

tat gag aag acg gag ttc ctg atg aag gag gag ctg aag ggt cac atc 5136
Tyr Glu Lys Thr Glu Phe Leu Met Lys Glu Glu Leu Lys Gly His Ile
1695                1700                1705                1710

aac gat ggt ttg ctg aca ctt cga tgc gct ttc agc cga gat gac ccc 5184
Asn Asp Gly Leu Leu Thr Leu Arg Cys Ala Phe Ser Arg Asp Asp Pro
                1715                1720                1725

aag aag aag gtg tat gtg cag gac ctt atc aag atg gac gaa aag atg 5232
Lys Lys Lys Val Tyr Val Gln Asp Leu Ile Lys Met Asp Glu Lys Met
            1730                1735                1740

atg tac gat tac ctc gtg gtg cag aag ggt tct atg tat tgc tgt gga 5280
Met Tyr Asp Tyr Leu Val Val Gln Lys Gly Ser Met Tyr Cys Cys Gly
        1745                1750                1755

tcc cgc agt ttc atc aag cct gtc cag gag tca ttg aaa cat tgc ttc 5328
Ser Arg Ser Phe Ile Lys Pro Val Gln Glu Ser Leu Lys His Cys Phe
    1760                1765                1770

atg aaa gct ggt ggg ctg act gca gag caa gct gag aac gag gtc atc     5376
Met Lys Ala Gly Gly Leu Thr Ala Glu Gln Ala Glu Asn Glu Val Ile
1775                1780                1785                1790

gat atg ttc acg acc ggg cgg tac aat atc gag gca tgg taa             5418
Asp Met Phe Thr Thr Gly Arg Tyr Asn Ile Glu Ala Trp
                1795                1800

gctgtgccac tggtgtggac catttttaac cctctaacca ccactttttt tttggaatcg 5478

atgcgtcaaa gcgagtatat actgtattgt ttctttttgc ctgggtgtga tggtcaccat 5538

tctcattggg cgatccataa cacagtgtgt cacccgggaa caggagcgga ctttctgacc 5598

tggctgacat ttcagaactc tccctccagc cccaccacct ctgactgagg atgcatgttg 5658

actgactgcg ctgcccactt ccttagcgga tcatttgaat ggtgggatat gcattttgca 5718

ctctgctgtc atgtgcactt acggctcgac caaccgtctc cgagctggcc ccgaagcgac 5778

aaccatatga tcggatttga gcggccgcga attc                              5812

<210> 3
<211> 1803
<212> PRT
<213> Euglena gracilis

<400> 3
Met Lys Gln Ser Val Arg Pro Ile Ile Ser Asn Val Leu Arg Lys Glu
1               5                   10                  15
Val Ala Leu Tyr Ser Thr Ile Ile Gly Gln Asp Lys Gly Lys Glu Pro
            20                  25                  30
Thr Gly Arg Thr Tyr Thr Ser Gly Pro Lys Pro Ala Ser His Ile Glu
        35                  40                  45
Val Pro His His Val Thr Val Pro Ala Thr Asp Arg Thr Pro Asn Pro
    50                  55                  60
Asp Ala Gln Phe Phe Gln Ser Val Asp Gly Ser Gln Ala Thr Ser His
65                  70                  75                  80
Val Ala Tyr Ala Leu Ser Asp Thr Ala Phe Ile Tyr Pro Ile Thr Pro
                85                  90                  95
Ser Ser Val Met Gly Glu Leu Ala Asp Val Trp Met Ala Gln Gly Arg
            100                 105                 110
Lys Asn Ala Phe Gly Gln Val Val Asp Val Arg Glu Met Gln Ser Glu
        115                 120                 125
Ala Gly Ala Ala Gly Ala Leu His Gly Ala Leu Ala Ala Gly Ala Ile
    130                 135                 140
Ala Thr Thr Phe Thr Ala Ser Gln Gly Leu Leu Leu Met Ile Pro Asn

145                 150                 155                 160
Met Tyr Lys Ile Ala Gly Glu Leu Met Pro Ser Val Ile His Val Ala
                165                 170                 175
Ala Arg Glu Leu Ala Gly His Ala Leu Ser Ile Phe Gly Gly His Ala
            180                 185                 190
Asp Val Met Ala Val Arg Gln Thr Gly Trp Ala Met Leu Cys Ser His
        195                 200                 205
Thr Val Gln Gln Ser His Asp Met Ala Leu Ile Ser His Val Ala Thr
    210                 215                 220
Leu Lys Ser Ser Ile Pro Phe Val His Phe Phe Asp Gly Phe Arg Thr
225                 230                 235                 240
Ser His Glu Val Asn Lys Ile Lys Met Leu Pro Tyr Ala Glu Leu Lys
                245                 250                 255
Lys Leu Val Pro Pro Gly Thr Met Glu Gln His Trp Ala Arg Ser Leu
            260                 265                 270
Asn Pro Met His Pro Thr Ile Arg Gly Thr Asn Gln Ser Ala Asp Ile
        275                 280                 285
Tyr Phe Gln Asn Met Glu Ser Ala Asn Gln Tyr Tyr Thr Asp Leu Ala
    290                 295                 300
Glu Val Val Gln Glu Thr Met Asp Glu Val Ala Pro Tyr Ile Gly Arg
305                 310                 315                 320
His Tyr Lys Ile Phe Glu Tyr Val Gly Ala Pro Asp Ala Glu Glu Val
                325                 330                 335
Thr Val Leu Met Gly Ser Gly Ala Thr Thr Val Asn Glu Ala Val Asp
            340                 345                 350
Leu Leu Val Lys Arg Gly Lys Lys Val Gly Ala Val Leu Val His Leu
        355                 360                 365
Tyr Arg Pro Trp Ser Thr Lys Ala Phe Glu Lys Val Leu Pro Lys Thr
    370                 375                 380
Val Lys Arg Ile Ala Ala Leu Asp Arg Cys Lys Glu Val Thr Ala Leu
385                 390                 395                 400
Gly Glu Pro Leu Tyr Leu Asp Val Ser Ala Thr Leu Asn Leu Phe Pro
                405                 410                 415
Glu Arg Gln Asn Val Lys Val Ile Gly Gly Arg Tyr Gly Leu Gly Ser
            420                 425                 430
Lys Asp Phe Ile Pro Glu His Ala Leu Ala Ile Tyr Ala Asn Leu Ala
        435                 440                 445
Ser Glu Asn Pro Ile Gln Arg Phe Thr Val Gly Ile Thr Asp Asp Val
    450                 455                 460
Thr Gly Thr Ser Val Pro Phe Val Asn Glu Arg Val Asp Thr Leu Pro
465                 470                 475                 480
Glu Gly Thr Arg Gln Cys Val Phe Trp Gly Ile Gly Ser Asp Gly Thr
                485                 490                 495
Val Gly Ala Asn Arg Ser Ala Val Arg Ile Ile Gly Asp Asn Ser Asp
            500                 505                 510
Leu Met Val Gln Ala Tyr Phe Gln Phe Asp Ala Phe Lys Ser Gly Gly
        515                 520                 525
Val Thr Ser Ser His Leu Arg Phe Gly Pro Lys Pro Ile Thr Ala Gln

530                 535                 540
Tyr Leu Val Thr Asn Ala Asp Tyr Ile Ala Cys His Phe Gln Glu Tyr
545                 550                 555                 560
Val Lys Arg Phe Asp Met Leu Asp Ala Ile Arg Glu Gly Gly Thr Phe
                565                 570                 575
Val Leu Asn Ser Arg Trp Thr Thr Glu Asp Met Glu Lys Glu Ile Pro
            580                 585                 590
Ala Asp Phe Arg Arg Asn Val Ala Gln Lys Lys Val Arg Phe Tyr Asn
        595                 600                 605
Val Asp Ala Arg Lys Ile Cys Asp Ser Phe Gly Leu Gly Lys Arg Ile
    610                 615                 620
Asn Met Leu Met Gln Ala Cys Phe Phe Lys Leu Ser Gly Val Leu Pro
625                 630                 635                 640
Leu Ala Glu Ala Gln Arg Leu Leu Asn Glu Ser Ile Val His Glu Tyr
                645                 650                 655
Gly Lys Lys Gly Gly Lys Val Val Glu Met Asn Gln Ala Val Val Asn
            660                 665                 670
Ala Val Phe Ala Gly Asp Leu Pro Gln Glu Val Gln Val Pro Ala Ala
        675                 680                 685
Trp Ala Asn Ala Val Asp Thr Ser Thr Arg Thr Pro Thr Gly Ile Glu
    690                 695                 700
Phe Val Asp Lys Ile Met Arg Pro Leu Met Asp Phe Lys Gly Asp Gln
705                 710                 715                 720
Leu Pro Val Ser Val Met Thr Pro Gly Gly Thr Phe Pro Val Gly Thr
                725                 730                 735
Thr Gln Tyr Ala Lys Arg Ala Ile Ala Ala Phe Ile Pro Gln Trp Ile
            740                 745                 750
Pro Ala Asn Cys Thr Gln Cys Asn Tyr Cys Ser Tyr Val Cys Pro His
        755                 760                 765
Ala Thr Ile Arg Pro Phe Val Leu Thr Asp Gln Glu Val Gln Leu Ala
    770                 775                 780
Pro Glu Ser Phe Val Thr Arg Lys Ala Lys Gly Asp Tyr Gln Gly Met
785                 790                 795                 800
Asn Phe Arg Ile Gln Val Ala Pro Glu Asp Cys Thr Gly Cys Gln Val
                805                 810                 815
Cys Val Glu Thr Cys Pro Asp Asp Ala Leu Glu Met Thr Asp Ala Phe
            820                 825                 830
Thr Ala Thr Pro Val Gln Arg Thr Asn Trp Glu Phe Ala Ile Lys Val
        835                 840                 845
Pro Asn Arg Gly Thr Met Thr Asp Arg Tyr Ser Leu Lys Gly Ser Gln
    850                 855                 860
Phe Gln Gln Pro Leu Leu Glu Phe Ser Gly Ala Cys Glu Gly Cys Gly
865                 870                 875                 880
Glu Thr Pro Tyr Val Lys Leu Leu Thr Gln Leu Phe Gly Glu Arg Thr
                885                 890                 895
Val Ile Ala Asn Ala Thr Gly Cys Ser Ser Ile Trp Gly Gly Thr Ala
            900                 905                 910
Gly Leu Ala Pro Tyr Thr Thr Asn Ala Lys Gly Gln Gly Pro Ala Trp

915                 920                 925
Gly Asn Ser Leu Phe Glu Asp Asn Ala Glu Phe Gly Phe Gly Ile Ala
    930                 935                 940
Val Ala Asn Ala Gln Lys Arg Ser Arg Val Arg Asp Cys Ile Leu Gln
945                 950                 955                 960
Ala Val Glu Lys Lys Val Ala Asp Glu Gly Leu Thr Thr Leu Leu Ala
                965                 970                 975
Gln Trp Leu Gln Asp Trp Asn Thr Gly Asp Lys Thr Leu Lys Tyr Gln
            980                 985                 990
Asp Gln Ile Ile Ala Gly Leu Ala Gln Gln Arg Ser Lys Asp Pro Leu
        995                 1000                1005
Leu Glu Gln Ile Tyr Gly Met Lys Asp Met Leu Pro Asn Ile Ser Gln
    1010                1015                1020
Trp Ile Ile Gly Gly Asp Gly Trp Ala Asn Asp Ile Gly Phe Gly Gly
1025                1030                1035                1040
Leu Asp His Val Leu Ala Ser Gly Gln Asn Leu Asn Val Leu Val Leu
                1045                1050                1055
Asp Thr Glu Met Tyr Ser Asn Thr Gly Gly Gln Ala Ser Lys Ser Thr
            1060                1065                1070
His Met Ala Ser Val Ala Lys Phe Ala Leu Gly Gly Lys Arg Thr Asn
        1075                1080                1085
Lys Lys Asn Leu Thr Glu Met Ala Met Ser Tyr Gly Asn Val Tyr Val
    1090                1095                1100
Ala Thr Val Ser His Gly Asn Met Ala Gln Cys Val Lys Ala Phe Val
1105                1110                1115                1120
Glu Ala Glu Ser Tyr Asp Gly Pro Ser Leu Ile Val Gly Tyr Ala Pro
                1125                1130                1135
Cys Ile Glu His Gly Leu Arg Ala Gly Met Ala Arg Met Val Gln Glu
            1140                1145                1150
Ser Glu Ala Ala Ile Ala Thr Gly Tyr Trp Pro Leu Tyr Arg Phe Asp
        1155                1160                1165
Pro Arg Leu Ala Thr Glu Gly Lys Asn Pro Phe Gln Leu Asp Ser Lys
    1170                1175                1180
Arg Ile Lys Gly Asn Leu Gln Glu Tyr Leu Asp Arg Gln Asn Arg Tyr
1185                1190                1195                1200
Val Asn Leu Lys Lys Asn Asn Pro Lys Gly Ala Asp Leu Leu Lys Ser
                1205                1210                1215
Gln Met Ala Asp Asn Ile Thr Ala Arg Phe Asn Arg Tyr Arg Arg Met
            1220                1225                1230
Leu Glu Gly Pro Asn Thr Lys Ala Ala Ala Pro Ser Gly Asn His Val
        1235                1240                1245
Thr Ile Leu Tyr Gly Ser Glu Thr Gly Asn Ser Glu Gly Leu Ala Lys
    1250                1255                1260
Glu Leu Ala Thr Asp Phe Glu Arg Arg Glu Tyr Ser Val Ala Val Gln
1265                1270                1275                1280
Ala Leu Asp Asp Ile Asp Val Ala Asp Leu Glu Asn Met Gly Phe Val
                1285                1290                1295
Val Ile Ala Val Ser Thr Cys Gly Gln Gly Gln Phe Pro Arg Asn Ser

            1300                1305                1310
Gln Leu Phe Trp Arg Glu Leu Gln Arg Asp Lys Pro Glu Gly Trp Leu
        1315                1320                1325
Lys Asn Leu Lys Tyr Thr Val Phe Gly Leu Gly Asp Ser Thr Tyr Tyr
    1330                1335                1340
Phe Tyr Cys His Thr Ala Lys Gln Ile Asp Ala Arg Leu Ala Ala Leu
1345                1350                1355                1360
Gly Ala Gln Arg Val Val Pro Ile Gly Phe Gly Asp Asp Gly Asp Glu
                1365                1370                1375
Asp Met Phe His Thr Gly Phe Asn Asn Trp Ile Pro Ser Val Trp Asn
            1380                1385                1390
Glu Leu Lys Thr Lys Thr Pro Glu Glu Ala Leu Phe Thr Pro Ser Ile
        1395                1400                1405
Ala Val Gln Leu Thr Pro Asn Ala Thr Pro Gln Asp Phe His Phe Ala
    1410                1415                1420
Lys Ser Thr Pro Val Leu Ser Ile Thr Gly Ala Glu Arg Ile Thr Pro
1425                1430                1435                1440
Ala Asp His Thr Arg Asn Phe Val Thr Ile Arg Trp Lys Thr Asp Leu
. 1445 1450 1455
Ser Tyr Gln Val Gly Asp Ser Leu Gly Val Phe Pro Glu Asn Thr Arg
1460 1465 1470
Ser Val Val Glu Glu Phe Leu Gln Tyr Tyr Gly Leu Asn Pro Lys Asp
1475 1480 1485
Val Ile Thr Ile Glu Asn Lys Gly Ser Arg Glu Leu Pro His Cys Met
1490 1495 1500
Ala Val Gly Asp Leu Phe Thr Lys Val Leu Asp Ile Leu Gly Lys Pro
1505 1510 1515 1520
Asn Asn Arg Phe Tyr Lys Thr Leu Ser Tyr Phe Ala Val Asp Lys Ala
1525 1530 1535
Glu Lys Glu Arg Leu Leu Lys Ile Ala Glu Met Gly Pro Glu Tyr Ser
1540 1545 1550
Asn Ile Leu Ser Glu Met Tyr His Tyr Ala Asp Ile Phe His Met Phe
1555 1560 1565
Pro Ser Ala Arg Pro Thr Leu Gln Tyr Leu Ile Glu Met Ile Pro Asn
1570 1575 1580
Ile Lys Pro Arg Tyr Tyr Ser Ile Ser Ser Ala Pro Ile His Thr Pro
1585 1590 1595 1600
Gly Glu Val His Ser Leu Val Leu Ile Asp Thr Trp Ile Thr Leu Ser
1605 1610 1615
Gly Lys His Arg Thr Gly Leu Thr Cys Thr Met Leu Glu His Leu Gln
1620 1625 1630
Ala Gly Gln Val Val Asp Gly Cys Ile His Pro Thr Ala Met Glu Phe
1635 1640 1645
Pro Asp His Glu Lys Pro Val Val Met Cys Ala Met Gly Ser Gly Leu
1650 1655 1660
Ala Pro Phe Val Ala Phe Leu Arg Glu Arg Ser Thr Leu Arg Lys Gln
1665 1670 1675 1680
Gly Lys Lys Thr Gly Asn Met Ala Leu Tyr Phe Gly Asn Arg Tyr Glu

1685                1690                1695
Lys Thr Glu Phe Leu Met Lys Glu Glu Leu Lys Gly His Ile Asn Asp
        1700                1705                1710
Gly Leu Leu Thr Leu Arg Cys Ala Phe Ser Arg Asp Asp Pro Lys Lys
    1715                1720                1725
Lys Val Tyr Val Gln Asp Leu Ile Lys Met Asp Glu Lys Met Met Tyr
1730                1735                1740
Asp Tyr Leu Val Val Gln Lys Gly Ser Met Tyr Cys Cys Gly Ser Arg
1745                1750                1755                1760
Ser Phe Ile Lys Pro Val Gln Glu Ser Leu Lys His Cys Phe Met Lys
                1765                1770                1775
Ala Gly Gly Leu Thr Ala Glu Gln Ala Glu Asn Glu Val Ile Asp Met
            1780                1785                1790
Phe Thr Thr Gly Arg Tyr Asn Ile Glu Ala Trp
        1795                1800

<210> 4
<211> 31
<212> DNA
<213> Künstliche Sequenz

<220>
<223> n encodes for inosins

<220>
<221> modified_base
<222> (2)
<223> i

<220>
<221> modified_base
<222> (17)
<223> i

<220>
<221> modified_base
<222> (26)
<223> i

<220>
<223> Beschreibung der künstlichen Sequenz: primer

<400> 4
tnttygarga yaaygcngar ttyggnttyg g                                31

<210> 5
<211> 29
<212> DNA
<213> Künstliche Sequenz

<220>
<223> Beschreibung der künstlichen Sequenz: Primer

<220>
<221> modified_base
<222> (3)
<223> i

<220>
<221> modified_base
<222> (15)
<223> i

<220>
<221> modified_base
<222> (15)
<223> i

<220>
<221> modified_base
<222> (27)
<223> i

<220>
<223> n encodes for  inosins

<400> 5
aanccdatrt crtangccca nccrtcncc                                    29

<210> 6
<211> 10
<212> PRT
<213> Euglena gracilis

<400> 6
Thr Ser Gly Pro Lys Pro Ala Ser Xaa Ile
  1               5                  10

<210> 7
<211> 16
<212> PRT

<213> Euglena gracilis

<400> 7
Thr Ser Gly Pro Xaa Pro Ala Ser Xaa Ile Glu Val Ser Xaa Ala Lys
1               5                   10                  15

<210> 8
<211> 20
<212> PRT
<213> Euglena gracilis

<400> 8
Ala Ala Ala Pro Ser Gly Asn Xaa Val Thr Ile Leu Tyr Gly Ser Glu
1               5                   10                  15

Glu Gly Asn Ser
            20

<210> 9
<211> 10
<212> PRT
<213> Euglena gracilis

<220>
<223> Xaa = (Phe/Trp/Tyr)

<400> 9
Leu Phe Glu Asp Asn Glu Phe Gly Xaa Gly
1               5                   10

<210> 10
<211> 11
<212> PRT
<213> Euglena gracilis

<220>
<223> Xaa = (Phe/Tyr)

<400> 10
Gly Gly Asp Gly Trp Ala Tyr Asp Ile Gly Xaa
1               5                   10